(19) Europäisches Patentamt
European Patent Office
Office européen des brevets

(11) **EP 4 382 540 A1**

(12) **EUROPEAN PATENT APPLICATION**
published in accordance with Art. 153(4) EPC

(43) Date of publication:
**12.06.2024 Bulletin 2024/24**

(21) Application number: **22852355.1**

(22) Date of filing: **05.08.2022**

(51) International Patent Classification (IPC):
**C07K 16/46** (2006.01)   **C12N 15/13** (2006.01)
**A61K 39/395** (2006.01)   **A61P 29/00** (2006.01)
**A61P 35/00** (2006.01)   **A61P 35/02** (2006.01)

(52) Cooperative Patent Classification (CPC):
**A61K 39/395; A61P 29/00; A61P 31/00;**
**A61P 35/00; A61P 35/02; A61P 35/04;**
**A61P 37/02; C07K 16/00; C07K 16/46**

(86) International application number:
**PCT/CN2022/110627**

(87) International publication number:
**WO 2023/011644 (09.02.2023 Gazette 2023/06)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **06.08.2021   CN 202110903033**

(71) Applicant: **Bio-Thera Solutions, Ltd.**
**Guangzhou, Guangdong 510530 (CN)**

(72) Inventors:
• **CHEN, Yifan**
**Guangzhou, Guangdong 510530 (CN)**
• **FU, Ziyi**
**Guangzhou, Guangdong 510530 (CN)**

• **LIANG, Qiulian**
**Guangzhou, Guangdong 510530 (CN)**
• **YU, Jin-chen**
**Guangzhou, Guangdong 510530 (CN)**
• **LI, Shengfeng**
**Guangzhou, Guangdong 510530 (CN)**

(74) Representative: **Engelhard, Markus**
**Boehmert & Boehmert**
**Anwaltspartnerschaft mbB**
**Pettenkoferstrasse 22**
**80336 München (DE)**

Remarks:
The complete document including Reference
Table(s) and the Sequence Listing(s) can be
downloaded from the EPO website

(54) **USE OF ANTI-PD-L1/CD47 BISPECIFIC ANTIBODY IN TREATMENT OF DISEASES**

(57)   The present invention discloses use of an anti-PD-L1/CD47 bispecific antibody in the treatment of a disease. A treatment method comprises administering to a patient in need thereof an effective amount of an anti-PD-L1/CD47 bispecific antibody or an antigen-binding fragment.

FIG. 1

**Description**

<u>TECHNICAL FIELD</u>

The present invention belongs to the field of bio-pharmaceuticals, and particularly relates to use of an anti-PD-L1/CD47 bispecific antibody in the treatment of an autoimmune disease, an inflammatory disease, an infectious disease, or a tumor.

<u>BACKGROUND</u>

Cancer immunotherapy, a revolutionary breakthrough in cancer therapy in recent years, is a research fever in the field of biological science. As more new antibody drugs are approved for marketing, the prospect of cancer therapy has shifted from complementary elements of traditional therapies to central and standard cancer treatment regimens. On the road to conquering cancer by activating the function of T cells with an immune checkpoint as a target and thus enhancing enhance the ability of the acquired immune system, the development of antibody therapy around the PD-L1/PD-1 pathway has become a hotspot in the field. When an organism develops inflammation or is infected, the programmed death-ligand 1 (PD-L1) can be induced to be expressed on hematopoietic, endothelial or epithelial cells, suppressing the immune response by binding to programmed death receptor 1 (PD-1) expressed on T cells, B cells and monocytes. The PD-1 cytoplasmic region contains two tyrosine-based signaling domains, an immunoreceptor tyrosine-based inhibitory motif (ITIM) and an immunoreceptor tyrosine-based switch motif (ITSM). PD-L1 is overexpressed in many cancers, including a wide variety of solid tumors and hematological tumors, such as bladder cancer, breast cancer, colon cancer, lung cancer, melanoma, ovarian cancer, gastric cancer, thyroid cancer, primary mediastinal large B-cell lymphoma and classical Hodgkin's lymphoma. PD-L1 overexpressed on tumor cells binds to PD-1 on T cells and activates ITIM domain of PD-1, which results in T cell dysfunction and failure, prevents cytotoxic T cells from effectively targeting tumor cells, and sends out a "don't find me" signal to the immune system. Thus, tumor cells obtain adaptive immune tolerance, thereby promoting tumor invasion and causing poor prognosis.

The innate immune system is the first line of nonspecific defense against infection and malignant cell transformation. In the innate immune system, monocytes, macrophages and dendritic cells act as antigen presenting cells (APCs) by phagocytosis. The ability of APCs to phagocytose tumor cells by phagocytosis is an indispensable bridge linking the innate immunity and the adaptive immunity. Targeting checkpoints that regulate phagocytosis (phagocytosis checkpoints, such as CD47-SIRPα) provides a new approach to the development of cancer immunotherapy. CD47 (integrin associated protein, IAP, CD47) is widely expressed on the surface of normal cells and, by binding to signal regulatory protein α (SIRPα) on the surface of macrophages, releases a "don't eat me" signal, thereby protecting healthy cells from being "eaten" by macrophages. Cancer cells take advantage of this mechanism and overexpress CD47 on their surface, such that macrophages treat them as "normal cells" and thus they escape from the monitoring of the innate immunity and the macrophage-mediated phagocytic attack. Studies have confirmed that almost all tumor cells highly express CD47, such as thyroid cancer, ovarian cancer, prostate cancer, cervical cancer, bladder cancer, head and neck cancer, gastric cancer, acute myelogenous leukemia, B- and T-cell acute leukemia and non-Hodgkin's lymphoma, and that CD47 overexpression is associated with poor clinical prognosis.

<u>SUMMARY</u>

The present invention discloses a method or use of an anti-PD-L1/CD47 bispecific antibody or an antigen-binding fragment in the treatment of an autoimmune disease, an inflammatory disease, an infectious disease, or a tumor. In some embodiments, provided is use of an anti-PD-L1/CD47 bispecific antibody or an antigen-binding fragment in the preparation of a medicament for treating an autoimmune disease, an inflammatory disease, an infectious disease, or a tumor.

In some embodiments, the invention provides the anti-PD-L1/CD47 bispecific antibody or the antigen-binding fragment, wherein the anti-PD-L1/CD47 bispecific antibody or the antigen-binding fragment comprises a variable region a specifically binding to PD-L1; wherein the variable region a comprises one or more amino acid sequences of (a)-(f):

  (a) a VHa CDR1 comprising an amino acid sequence set forth in SEQ ID NO: 1 or an amino acid sequence having one or more conservative amino acid substitutions as compared with the amino acid sequence set forth in SEQ ID NO: 1;

  (b) a VHa CDR2 comprising an amino acid sequence set forth in SEQ ID NO: 2 or an amino acid sequence having one or more conservative amino acid substitutions as compared with the amino acid sequence set forth in SEQ ID NO: 2;

  (c) a VHa CDR3 comprising an amino acid sequence set forth in SEQ ID NO: 3 or an amino acid sequence having one or more conservative amino acid substitutions as compared with the amino acid sequence set forth in SEQ ID NO: 3;

(d) a VLa CDR1 comprising an amino acid sequence set forth in SEQ ID NO: 4 or an amino acid sequence having one or more conservative amino acid substitutions as compared with the amino acid sequence set forth in SEQ ID NO: 4;

(e) a VLa CDR2 comprising an amino acid sequence set forth in SEQ ID NO: 5 or an amino acid sequence having one or more conservative amino acid substitutions as compared with the amino acid sequence set forth in SEQ ID NO: 5;

(f) a VLa CDR3 comprising an amino acid sequence set forth in SEQ ID NO: 6 or an amino acid sequence having one or more conservative amino acid substitutions as compared with the amino acid sequence set forth in SEQ ID NO: 6.

In some embodiments, the variable region a comprises a heavy chain variable region a (VHa), wherein the heavy chain variable region a comprises a VHa CDR1, a VHa CDR2, and a VHa CDR3; wherein the VHa CDR1 comprises an amino acid sequence set forth in SEQ ID NO: 1; the VHa CDR2 comprises an amino acid sequence set forth in SEQ ID NO: 2; the VHa CDR3 comprises an amino acid sequence set forth in SEQ ID NO: 3.

In some embodiments, the variable region a comprises a light chain variable region a (VLa) wherein the light chain variable region a comprises a VLa CDR1, a VLa CDR2, and a VLa CDR3; wherein the VLa CDR1 comprises an amino acid sequence set forth in SEQ ID NO: 4; the VLa CDR2 comprises an amino acid sequence set forth in SEQ ID NO: 5; the VLa CDR3 comprises an amino acid sequence set forth in SEQ ID NO: 6.

In some embodiments, the variable region a comprises a VHa and a VLa; wherein the VHa comprises a VHa CDR1, a VHa CDR2, and a VHa CDR3; the VLa comprises a VLa CDR1, a VLa CDR2, and a VLa CDR3, wherein the VHa CDR1 comprises an amino acid sequence set forth in SEQ ID NO: 1; the VHa CDR2 comprises an amino acid sequence set forth in SEQ ID NO: 2; the VHa CDR3 comprises an amino acid sequence set forth in SEQ ID NO: 3; the VLa CDR1 comprises an amino acid sequence set forth in SEQ ID NO: 4; the VLa CDR2 comprises an amino acid sequence set forth in SEQ ID NO: 5; the VLa CDR3 comprises an amino acid sequence set forth in SEQ ID NO: 6.

In some embodiments, the VHa CDR1 comprises an amino acid sequence set forth in SEQ ID NO: 1 or an amino acid sequence having one or more conservative amino acid substitutions as compared with the amino acid sequence set forth in SEQ ID NO: 1; the VHa CDR2 comprises an amino acid sequence set forth in SEQ ID NO: 2 or an amino acid sequence having one or more conservative amino acid substitutions as compared with the amino acid sequence set forth in SEQ ID NO: 2; the VHa CDR3 comprises an amino acid sequence set forth in SEQ ID NO: 3 or an amino acid sequence having one or more conservative amino acid substitutions as compared with the amino acid sequence set forth in SEQ ID NO: 3.

In some embodiments, the VLa CDR1 comprises an amino acid sequence set forth in SEQ ID NO: 4 or an amino acid sequence having one or more conservative amino acid substitutions as compared with the amino acid sequence set forth in SEQ ID NO: 4; the VLa CDR2 comprises an amino acid sequence set forth in SEQ ID NO: 5 or an amino acid sequence having one or more conservative amino acid substitutions as compared with the amino acid sequence set forth in SEQ ID NO: 5; the VLa CDR3 comprises an amino acid sequence set forth in SEQ ID NO: 6 or an amino acid sequence having one or more conservative amino acid substitutions as compared with the amino acid sequence set forth in SEQ ID NO: 6.

In some embodiments, the VHa CDR1 comprises an amino acid sequence set forth in SEQ ID NO: 1 or an amino acid sequence having one or more conservative amino acid substitutions as compared with the amino acid sequence set forth in SEQ ID NO: 1; the VHa CDR2 comprises an amino acid sequence set forth in SEQ ID NO: 2 or an amino acid sequence having one or more conservative amino acid substitutions as compared with the amino acid sequence set forth in SEQ ID NO: 2; the VHa CDR3 comprises an amino acid sequence set forth in SEQ ID NO: 3 or an amino acid sequence having one or more conservative amino acid substitutions as compared with the amino acid sequence set forth in SEQ ID NO: 3; the VLa CDR1 comprises an amino acid sequence set forth in SEQ ID NO: 4 or an amino acid sequence having one or more conservative amino acid substitutions as compared with the amino acid sequence set forth in SEQ ID NO: 4; the VLa CDR2 comprises an amino acid sequence set forth in SEQ ID NO: 5 or an amino acid sequence having one or more conservative amino acid substitutions as compared with the amino acid sequence set forth in SEQ ID NO: 5; the VLa CDR3 comprises an amino acid sequence set forth in SEQ ID NO: 6 or an amino acid sequence having one or more conservative amino acid substitutions as compared with the amino acid sequence set forth in SEQ ID NO: 6.

In some embodiments, the VHa comprises an amino acid sequence set forth in SEQ ID NO: 10, or an amino acid sequence having at least 80% or 90% identity to the amino acid sequence set forth in SEQ ID NO: 10, or an amino acid sequence having one or more conservative amino acid substitutions as compared with the amino acid sequence set forth in SEQ ID NO: 10. In some embodiments, the VLa comprises an amino acid sequence set forth in SEQ ID NO: 12, or an amino acid sequence having at least 80% or 90% identity to the amino acid sequence set forth in SEQ ID NO:

12, or an amino acid sequence having one or more conservative amino acid substitutions as compared with the amino acid sequence set forth in SEQ ID NO: 12.

In some embodiments, the variable region a comprises a VHa and a VLa. The VHa comprises an amino acid sequence set forth in SEQ ID NO: 10, or an amino acid sequence having at least 80% or 90% identity to the amino acid sequence set forth in SEQ ID NO: 10, or an amino acid sequence having one or more conservative amino acid substitutions as compared with the amino acid sequence set forth in SEQ ID NO: 10; the VLa comprises an amino acid sequence set forth in SEQ ID NO: 12, or an amino acid sequence having at least 80% or 90% identity to the amino acid sequence set forth in SEQ ID NO: 12, or an amino acid sequence having one or more conservative amino acid substitutions as compared with the amino acid sequence set forth in SEQ ID NO: 12.

In some embodiments, the anti-PD-L1/CD47 bispecific antibody or the antigen-binding fragment further comprises a light chain constant region a (CLa) and a heavy chain constant region a (CHa).

In some embodiments, the CHa comprises an amino acid sequence set forth in SEQ ID NO: 13 or 19, or an amino acid sequence having at least 80% or 90% identity to the amino acid sequence set forth in SEQ ID NO: 13 or 19, or an amino acid sequence having one or more conservative amino acid substitutions as compared with the amino acid sequence set forth in SEQ ID NO: 13 or 19.

In some embodiments, the CLa comprises an amino acid sequence set forth in SEQ ID NO: 14, or an amino acid sequence having at least 80% or 90% identity to the amino acid sequence set forth in SEQ ID NO: 14, or an amino acid sequence having one or more conservative amino acid substitutions as compared with the amino acid sequence set forth in SEQ ID NO: 14.

In some embodiments, the anti-PD-L1/CD47 bispecific antibody provided herein comprises one heavy chain a and one light chain a; wherein the heavy chain a and the light chain a are paired to form a PD-L1 antigen-combining site.

In some embodiments, the heavy chain a comprises an amino acid sequence set forth in SEQ ID NO: 16 or 21, or an amino acid sequence having at least 80% or 90% identity to the amino acid sequence set forth in SEQ ID NO: 16 or 21, or an amino acid sequence having one or more conservative amino acid substitutions as compared with the amino acid sequence set forth in SEQ ID NO: 16 or 21.

In some embodiments, the anti-PD-L1/CD47 bispecific antibody or the antigen-binding fragment provided herein has an affinity index KD of $\leq$ 10 nM for PD-L1. In some embodiments, the anti-PD-L1/CD47 bispecific antibody or the antigen-binding fragment provided herein has an affinity index KD of $\leq$ 1 nM for PD-L1. In some embodiments, the anti-PD-L1/CD47 bispecific antibody or the antigen-binding fragment provided herein has an affinity index KD of $\leq$ 0.5 nM for PD-L1. In some embodiments, the anti-PD-L1/CD47 bispecific antibody or the antigen-binding fragment provided herein has an affinity index KD of $\leq$ 0.2 nM for PD-L1.

In another aspect, the present invention provides the anti-PD-L1/CD47 bispecific antibody or the antigen-binding fragment, wherein the anti-PD-L1/CD47 bispecific antibody or the antigen-binding fragment comprises a variable region b specifically binding to CD47; wherein the variable region b comprises one or more amino acid sequences of (g)-(l):

> (g) a VHb CDR1 comprising an amino acid sequence set forth in SEQ ID NO: 7 or an amino acid sequence having one or more conservative amino acid substitutions as compared with the amino acid sequence set forth in SEQ ID NO: 7;
> (h) a VHb CDR2 comprising an amino acid sequence set forth in SEQ ID NO: 8 or an amino acid sequence having one or more conservative amino acid substitutions as compared with the amino acid sequence set forth in SEQ ID NO: 8;
> (i) a VHb CDR3 comprising an amino acid sequence set forth in SEQ ID NO: 9 or an amino acid sequence having one or more conservative amino acid substitutions as compared with the amino acid sequence set forth in SEQ ID NO: 9;
> (j) a VLb CDR1 comprising an amino acid sequence set forth in SEQ ID NO: 4 or an amino acid sequence having one or more conservative amino acid substitutions as compared with the amino acid sequence set forth in SEQ ID NO: 4;
> (k) a VLb CDR2 comprising an amino acid sequence set forth in SEQ ID NO: 5 or an amino acid sequence having one or more conservative amino acid substitutions as compared with the amino acid sequence set forth in SEQ ID NO: 5;
> (l) a VLb CDR3 comprising an amino acid sequence set forth in SEQ ID NO: 6 or an amino acid sequence having one or more conservative amino acid substitutions as compared with the amino acid sequence set forth in SEQ ID NO: 6.

In some embodiments, the variable region b comprises a heavy chain variable region b (VHb), wherein the heavy chain variable region b comprises a VHb CDR1, a VHb CDR2, and a VHb CDR3; wherein the VHb CDR1 comprises an amino acid sequence set forth in SEQ ID NO: 7; the VHb CDR2 comprises an amino acid sequence set forth in SEQ ID NO: 8; the VHb CDR3 comprises an amino acid sequence set forth in SEQ ID NO: 9.

In some embodiments, the variable region b comprises a light chain variable region b (VLb), wherein the light chain variable region b comprises a VLb CDR1, a VLb CDR2, and a VLb CDR3; wherein the VLb CDR1 comprises an amino acid sequence set forth in SEQ ID NO: 4; the VLb CDR2 comprises an amino acid sequence set forth in SEQ ID NO: 5; the VLb CDR3 comprises an amino acid sequence set forth in SEQ ID NO: 6.

In some embodiments, the variable region b comprises a VHb and a VLb; wherein the VHb comprises a VHb CDR1, a VHb CDR2, and a VHb CDR3; the VLb comprises a VLb CDR1, a VLb CDR2, and a VLb CDR3, wherein the VHb CDR1 comprises an amino acid sequence set forth in SEQ ID NO: 7; the VHb CDR2 comprises an amino acid sequence set forth in SEQ ID NO: 8; the VHb CDR3 comprises an amino acid sequence set forth in SEQ ID NO: 9; the VLb CDR1 comprises an amino acid sequence set forth in SEQ ID NO: 4; the VLb CDR2 comprises an amino acid sequence set forth in SEQ ID NO: 5; the VLb CDR3 comprises an amino acid sequence set forth in SEQ ID NO: 6.

In some embodiments, the VHb CDR1 comprises an amino acid sequence set forth in SEQ ID NO: 7 or an amino acid sequence having one or more conservative amino acid substitutions as compared with the amino acid sequence set forth in SEQ ID NO: 7; the VHb CDR2 comprises an amino acid sequence set forth in SEQ ID NO: 8 or an amino acid sequence having one or more conservative amino acid substitutions as compared with the amino acid sequence set forth in SEQ ID NO: 8; the VHb CDR3 comprises an amino acid sequence set forth in SEQ ID NO: 9 or an amino acid sequence having one or more conservative amino acid substitutions as compared with the amino acid sequence set forth in SEQ ID NO: 9.

In some embodiments, the VLb CDR1 comprises an amino acid sequence set forth in SEQ ID NO: 4 or an amino acid sequence having one or more conservative amino acid substitutions as compared with the amino acid sequence set forth in SEQ ID NO: 4; the VLb CDR2 comprises an amino acid sequence set forth in SEQ ID NO: 5 or an amino acid sequence having one or more conservative amino acid substitutions as compared with the amino acid sequence set forth in SEQ ID NO: 5; the VLb CDR3 comprises an amino acid sequence set forth in SEQ ID NO: 6 or an amino acid sequence having one or more conservative amino acid substitutions as compared with the amino acid sequence set forth in SEQ ID NO: 6.

In some embodiments, the VHb CDR1 comprises an amino acid sequence set forth in SEQ ID NO: 7 or an amino acid sequence having one or more conservative amino acid substitutions as compared with the amino acid sequence set forth in SEQ ID NO: 7; the VHb CDR2 comprises an amino acid sequence set forth in SEQ ID NO: 8 or an amino acid sequence having one or more conservative amino acid substitutions as compared with the amino acid sequence set forth in SEQ ID NO: 8; the VHb CDR3 comprises an amino acid sequence set forth in SEQ ID NO: 9 or an amino acid sequence having one or more conservative amino acid substitutions as compared with the amino acid sequence set forth in SEQ ID NO: 9; the VLb CDR1 comprises an amino acid sequence set forth in SEQ ID NO: 4 or an amino acid sequence having one or more conservative amino acid substitutions as compared with the amino acid sequence set forth in SEQ ID NO: 4; the VLb CDR2 comprises an amino acid sequence set forth in SEQ ID NO: 5 or an amino acid sequence having one or more conservative amino acid substitutions as compared with the amino acid sequence set forth in SEQ ID NO: 5; the VLb CDR3 comprises an amino acid sequence set forth in SEQ ID NO: 6 or an amino acid sequence having one or more conservative amino acid substitutions as compared with the amino acid sequence set forth in SEQ ID NO: 6.

In some embodiments, the VHb comprises an amino acid sequence set forth in SEQ ID NO: 11, or an amino acid sequence having at least 80% or 90% identity to the amino acid sequence set forth in SEQ ID NO: 11, or an amino acid sequence having one or more conservative amino acid substitutions as compared with the amino acid sequence set forth in SEQ ID NO: 11.

In some embodiments, the VLb comprises an amino acid sequence set forth in SEQ ID NO: 12, or an amino acid sequence having at least 80% or 90% identity to the amino acid sequence set forth in SEQ ID NO: 12, or an amino acid sequence having one or more conservative amino acid substitutions as compared with the amino acid sequence set forth in SEQ ID NO: 12. In some embodiments, the variable region b comprises a VHb and a VLb. The VHb comprises an amino acid sequence set forth in SEQ ID NO: 11, or an amino acid sequence having at least 80% or 90% identity to the amino acid sequence set forth in SEQ ID NO: 11, or an amino acid sequence having one or more conservative amino acid substitutions as compared with the amino acid sequence set forth in SEQ ID NO: 11. The VLb comprises an amino acid sequence set forth in SEQ ID NO: 12, or an amino acid sequence having at least 80% or 90% identity to the amino acid sequence set forth in SEQ ID NO: 12, or an amino acid sequence having one or more conservative amino acid substitutions as compared with the amino acid sequence set forth in SEQ ID NO: 12.

In some embodiments, the anti-PD-L1/CD47 bispecific antibody or the antigen-binding fragment further comprises a light chain constant region b (CLb) and a heavy chain constant region b (CHb).

In some embodiments, the CHb comprises an amino acid sequence set forth in SEQ ID NO: 15 or 20, or an amino acid sequence having at least 80% or 90% identity to the amino acid sequence set forth in SEQ ID NO: 15 or 20, or an amino acid sequence having one or more conservative amino acid substitutions as compared with the amino acid sequence set forth in SEQ ID NO: 15 or 20.

In some embodiments, the CLb comprises an amino acid sequence set forth in SEQ ID NO: 14, or an amino acid sequence having at least 80% or 90% identity to the amino acid sequence set forth in SEQ ID NO: 14, or an amino acid sequence having one or more conservative amino acid substitutions as compared with the amino acid sequence set forth in SEQ ID NO: 14. In some embodiments, the anti-PD-L1/CD47 bispecific antibody provided herein comprises one heavy chain b and one light chain b; wherein the heavy chain b and the light chain b are paired to form a CD47 antigen-combining site.

In some embodiments, the heavy chain b comprises an amino acid sequence set forth in SEQ ID NO: 17 or 22, or an amino acid sequence having at least 80% or 90% identity to the amino acid sequence set forth in SEQ ID NO: 17 or 22, or an amino acid sequence having one or more conservative amino acid substitutions as compared with the amino acid sequence set forth in SEQ ID NO: 17 or 22.

In some embodiments, the anti-PD-L1/CD47 bispecific antibody or the antigen-binding fragment provided herein has an affinity index KD of ≤ 10 nM for CD47. In some embodiments, the anti-PD-L1/CD47 bispecific antibody or the antigen-binding fragment provided herein has an affinity index KD of ≤ 5 nM for CD47. In some embodiments, the anti-PD-L1/CD47 bispecific antibody or the antigen-binding fragment provided herein has an affinity index KD of ≤ 4 nM for CD47.

In some embodiments, the anti-PD-L1/CD47 bispecific antibody or the antigen-binding fragment provided herein does not cause agglutination of red blood cells.

In another aspect, the present invention provides the anti-PD-L1/CD47 bispecific antibody or the antigen-binding fragment, wherein the anti-PD-L1/CD47 bispecific antibody or the antigen-binding fragment comprises a variable region a specifically binding to PD-L1 and a variable region b specifically binding to CD47.

The variable region a comprises one or more amino acid sequences of (a)-(f):

(a) a VHa CDR1 comprising an amino acid sequence set forth in SEQ ID NO: 1 or an amino acid sequence having one or more conservative amino acid substitutions as compared with the amino acid sequence set forth in SEQ ID NO: 1;

(b) a VHa CDR2 comprising an amino acid sequence set forth in SEQ ID NO: 2 or an amino acid sequence having one or more conservative amino acid substitutions as compared with the amino acid sequence set forth in SEQ ID NO: 2;

(c) a VHa CDR3 comprising an amino acid sequence set forth in SEQ ID NO: 3 or an amino acid sequence having one or more conservative amino acid substitutions as compared with the amino acid sequence set forth in SEQ ID NO: 3;

(d) a VLa CDR1 comprising an amino acid sequence set forth in SEQ ID NO: 4 or an amino acid sequence having one or more conservative amino acid substitutions as compared with the amino acid sequence set forth in SEQ ID NO: 4;

(e) a VLa CDR2 comprising an amino acid sequence set forth in SEQ ID NO: 5 or an amino acid sequence having one or more conservative amino acid substitutions as compared with the amino acid sequence set forth in SEQ ID NO: 5;

(f) a VLa CDR3 comprising an amino acid sequence set forth in SEQ ID NO: 6 or an amino acid sequence having one or more conservative amino acid substitutions as compared with the amino acid sequence set forth in SEQ ID NO: 6.

The variable region b comprises one or more amino acid sequences of (g)-(l):

(g) a VHb CDR1 comprising an amino acid sequence set forth in SEQ ID NO: 7 or an amino acid sequence having one or more conservative amino acid substitutions as compared with the amino acid sequence set forth in SEQ ID NO: 7;

(h) a VHb CDR2 comprising an amino acid sequence set forth in SEQ ID NO: 8 or an amino acid sequence having one or more conservative amino acid substitutions as compared with the amino acid sequence set forth in SEQ ID NO: 8;

(i) a VHb CDR3 comprising an amino acid sequence set forth in SEQ ID NO: 9 or an amino acid sequence having one or more conservative amino acid substitutions as compared with the amino acid sequence set forth in SEQ ID NO: 9;

(j) a VLb CDR1 comprising an amino acid sequence set forth in SEQ ID NO: 4 or an amino acid sequence having one or more conservative amino acid substitutions as compared with the amino acid sequence set forth in SEQ ID NO: 4;

(k) a VLb CDR2 comprising an amino acid sequence set forth in SEQ ID NO: 5 or an amino acid sequence having one or more conservative amino acid substitutions as compared with the amino acid sequence set forth in SEQ ID NO: 5;

(l) a VLb CDR3 comprising an amino acid sequence set forth in SEQ ID NO: 6 or an amino acid sequence having one or more conservative amino acid substitutions as compared with the amino acid sequence set forth in SEQ ID NO: 6.

In some embodiments, the variable region a comprises a heavy chain variable region a (VHa), wherein the heavy chain variable region a comprises a VHa CDR1, a VHa CDR2, and a VHa CDR3; wherein the VHa CDR1 comprises an amino acid sequence set forth in SEQ ID NO: 1; the VHa CDR2 comprises an amino acid sequence set forth in SEQ ID NO: 2; the VHa CDR3 comprises an amino acid sequence set forth in SEQ ID NO: 3. The variable region b comprises a heavy chain variable region b (VHb), wherein the heavy chain variable region b comprises a VHb CDR1, a VHb CDR2, and a VHb CDR3; wherein the VHb CDR1 comprises an amino acid sequence set forth in SEQ ID NO: 7; the VHb CDR2 comprises an amino acid sequence set forth in SEQ ID NO: 8; the VHb CDR3 comprises an amino acid sequence set forth in SEQ ID NO: 9.

In some embodiments, the variable region a comprises a light chain variable region a (VLa) wherein the light chain variable region a comprises a VLa CDR1, a VLa CDR2, and a VLa CDR3; wherein the VLa CDR1 comprises an amino acid sequence set forth in SEQ ID NO: 4; the VLa CDR2 comprises an amino acid sequence set forth in SEQ ID NO: 5; the VLa CDR3 comprises an amino acid sequence set forth in SEQ ID NO: 6. The variable region b comprises a light chain variable region b (VLb), wherein the light chain variable region b comprises a VLb CDR1, a VLb CDR2, and a VLb CDR3; wherein the VLb CDR1 comprises an amino acid sequence set forth in SEQ ID NO: 4; the VLb CDR2 comprises an amino acid sequence set forth in SEQ ID NO: 5; the VLb CDR3 comprises an amino acid sequence set forth in SEQ ID NO: 6.

In some embodiments, the variable region a comprises a VHa and a VLa; the variable region b comprises a VHb and a VLb. The VHa comprises a VHa CDR1, a VHa CDR2, and a VHa CDR3; the VLa comprises a VLa CDR1, a VLa CDR2, and a VLa CDR3, wherein the VHa CDR1 comprises an amino acid sequence set forth in SEQ ID NO: 1; the VHa CDR2 comprises an amino acid sequence set forth in SEQ ID NO: 2; the VHa CDR3 comprises an amino acid sequence set forth in SEQ ID NO: 3. The VHb comprises a VHb CDR1, a VHb CDR2, and a VHb CDR3; the VLb comprises a VLb CDR1, a VLb CDR2, and a VLb CDR3, wherein the VHb CDR1 comprises an amino acid sequence set forth in SEQ ID NO: 7; the VHb CDR2 comprises an amino acid sequence set forth in SEQ ID NO: 8; the VHb CDR3 comprises an amino acid sequence set forth in SEQ ID NO: 9. The VLa CDR1 or the VLb CDR1 comprises an amino acid sequence set forth in SEQ ID NO: 4; the VLa CDR2 or the VLb CDR2 comprises an amino acid sequence set forth in SEQ ID NO: 5; the VLa CDR3 or the VLb CDR3 comprises an amino acid sequence set forth in SEQ ID NO: 6.

In some embodiments, the VHa CDR1 comprises an amino acid sequence set forth in SEQ ID NO: 1 or an amino acid sequence having one or more conservative amino acid substitutions as compared with the amino acid sequence set forth in SEQ ID NO: 1; the VHa CDR2 comprises an amino acid sequence set forth in SEQ ID NO: 2 or an amino acid sequence having one or more conservative amino acid substitutions as compared with the amino acid sequence set forth in SEQ ID NO: 2; the VHa CDR3 comprises an amino acid sequence set forth in SEQ ID NO: 3 or an amino acid sequence having one or more conservative amino acid substitutions as compared with the amino acid sequence set forth in SEQ ID NO: 3; the VHb CDR1 comprises an amino acid sequence set forth in SEQ ID NO: 7 or an amino acid sequence having one or more conservative amino acid substitutions as compared with the amino acid sequence set forth in SEQ ID NO: 7; the VHb CDR2 comprises an amino acid sequence set forth in SEQ ID NO: 8 or an amino acid sequence having one or more conservative amino acid substitutions as compared with the amino acid sequence set forth in SEQ ID NO: 8; the VHb CDR3 comprises an amino acid sequence set forth in SEQ ID NO: 9 or an amino acid sequence having one or more conservative amino acid substitutions as compared with the amino acid sequence set forth in SEQ ID NO: 9.

In some embodiments, the VLa CDR1 or the VLb CDR1 comprises an amino acid sequence set forth in SEQ ID NO: 4 or an amino acid sequence having one or more conservative amino acid substitutions as compared with the amino acid sequence set forth in SEQ ID NO: 4; the VLa CDR2 or the VLb CDR2 comprises an amino acid sequence set forth in SEQ ID NO: 5 or an amino acid sequence having one or more conservative amino acid substitutions as compared with the amino acid sequence set forth in SEQ ID NO: 5; the VLa CDR3 or the VLb CDR3 comprises an amino acid sequence set forth in SEQ ID NO: 6 or an amino acid sequence having one or more conservative amino acid substitutions as compared with the amino acid sequence set forth in SEQ ID NO: 6.

In some embodiments, the VHa CDR1 comprises an amino acid sequence set forth in SEQ ID NO: 1 or an amino acid sequence having one or more conservative amino acid substitutions as compared with the amino acid sequence set forth in SEQ ID NO: 1; the VHa CDR2 comprises an amino acid sequence set forth in SEQ ID NO: 2 or an amino acid sequence having one or more conservative amino acid substitutions as compared with the amino acid sequence set forth in SEQ ID NO: 2; the VHa CDR3 comprises an amino acid sequence set forth in SEQ ID NO: 3 or an amino acid sequence having one or more conservative amino acid substitutions as compared with the amino acid sequence set forth in SEQ ID NO: 3; the VHb CDR1 comprises an amino acid sequence set forth in SEQ ID NO: 7 or an amino acid sequence having one or more conservative amino acid substitutions as compared with the amino acid sequence set forth in SEQ ID NO: 7; the VHb CDR2 comprises an amino acid sequence set forth in SEQ ID NO: 8 or an amino acid sequence having one or more conservative amino acid substitutions as compared with the amino acid sequence set forth in SEQ ID NO: 8; the VHb CDR3 comprises an amino acid sequence set forth in SEQ ID NO: 9 or an amino acid sequence having one or more conservative amino acid substitutions as compared with the amino acid sequence set forth in SEQ ID NO: 9; the VLa CDR1 or the VLb CDR1 comprises an amino acid sequence set forth in SEQ ID NO: 4 or an amino acid sequence having one or more conservative amino acid substitutions as compared with the amino acid sequence set forth in SEQ ID NO: 4; the VLa CDR2 or the VLb CDR2 comprises an amino acid sequence set forth in SEQ ID NO: 5 or an amino acid sequence having one or more conservative amino acid substitutions as compared with the amino acid sequence set forth in SEQ ID NO: 5; the VLa CDR3 or the VLb CDR3 comprises an amino acid sequence set forth in SEQ ID NO: 6 or an amino acid sequence having one or more conservative amino acid substitutions as compared with the amino acid sequence set forth in SEQ ID NO: 6.

In some embodiments, the VLa CDR1 and the VLb CDR1 comprise identical amino acid sequences.

In some embodiments, the VLa CDR2 and the VLb CDR2 comprise identical amino acid sequences.

In some embodiments, the VLa CDR3 and the VLb CDR3 comprise identical amino acid sequences.

In some embodiments, the VLa CDR1 and the VLb CDR1 comprise identical amino acid sequences, the VLa CDR2 and the VLb CDR2 comprise identical amino acid sequences, and the VLa CDR3 and the VLb CDR3 comprise identical amino acid sequences.

In some embodiments, the variable region a comprises a heavy chain variable region VHa and a light chain variable region VLa; wherein the VHa comprises an amino acid sequence set forth in SEQ ID NO: 10, or an amino acid sequence having at least 80% or 90% identity to the amino acid sequence set forth in SEQ ID NO: 10, or an amino acid sequence having one or more conservative amino acid substitutions as compared with the amino acid sequence set forth in SEQ ID NO: 10; and/or the VLa comprises an amino acid sequence set forth in SEQ ID NO: 12, or an amino acid sequence having at least 80% or 90% identity to the amino acid sequence set forth in SEQ ID NO: 12, or an amino acid sequence having one or more conservative amino acid substitutions as compared with the amino acid sequence set forth in SEQ ID NO: 12.

In some embodiments, the variable region b comprises a heavy chain variable region VHb and a light chain variable region VLb; wherein the VHb comprises an amino acid sequence set forth in SEQ ID NO: 11 or an amino acid sequence having at least 80% or 90% identity to the amino acid sequence set forth in SEQ ID NO: 11; and/or the VLb comprises an amino acid sequence set forth in SEQ ID NO: 12 or an amino acid sequence having at least 80% or 90% identity to the amino acid sequence set forth in SEQ ID NO: 12.

In some embodiments, the VHa comprises an amino acid sequence set forth in SEQ ID NO: 10, or an amino acid sequence having at least 80% or 90% identity to the amino acid sequence set forth in SEQ ID NO: 10, or an amino acid sequence having one or more conservative amino acid substitutions as compared with the amino acid sequence set forth in SEQ ID NO: 10; the VHb comprises an amino acid sequence set forth in SEQ ID NO: 11, or an amino acid sequence having at least 80% or 90% identity to the amino acid sequence set forth in SEQ ID NO: 11, or an amino acid sequence having one or more conservative amino acid substitutions as compared with the amino acid sequence set forth in SEQ ID NO: 11.

In some embodiments, the VLa or VLb comprises an amino acid sequence set forth in SEQ ID NO: 12, or an amino acid sequence having at least 80% or 90% identity to the amino acid sequence set forth in SEQ ID NO: 12, or an amino acid sequence having one or more conservative amino acid substitutions as compared with the amino acid sequence set forth in SEQ ID NO: 12.

In some embodiments, the variable region a comprises a VHa and a VLa; the variable region b comprises a VHb and a VLb. The VHa comprises an amino acid sequence set forth in SEQ ID NO: 10, or an amino acid sequence having at least 80% or 90% identity to the amino acid sequence set forth in SEQ ID NO: 10, or an amino acid sequence having one or more conservative amino acid substitutions as compared with the amino acid sequence set forth in SEQ ID NO: 10. The VHb comprises an amino acid sequence set forth in SEQ ID NO: 11, or an amino acid sequence having at least 80% or 90% identity to the amino acid sequence set forth in SEQ ID NO: 11, or an amino acid sequence having one or more conservative amino acid substitutions as compared with the amino acid sequence set forth in SEQ ID NO: 11. The VLa or VLb comprises an amino acid sequence set forth in SEQ ID NO: 12, or an amino acid sequence having at least 80% or 90% identity to the amino acid sequence set forth in SEQ ID NO: 12, or an amino acid sequence having one or more conservative amino acid substitutions as compared with the amino acid sequence set forth in SEQ ID NO: 12.

In some embodiments, the VLa and the VLb comprise identical amino acid sequences.

In some embodiments, the present invention provides the anti-PD-L1/CD47 bispecific antibody or the antigen-binding fragment, wherein the anti-PD-L1/CD47 bispecific antibody or the antigen-binding fragment further comprises a light chain constant region a (CLa), and a heavy chain constant region a (CHa), a light chain constant region b (CLb), and a heavy chain constant region b (CHb).

In some embodiments, the VHa is linked to the CHa to form a heavy chain a, and the VHb is linked to the CHb to form a heavy chain b. In some embodiments, the VLa is linked to the CLa to form a light chain a, and the VLb is linked to the CLb to form a light chain b.

In some embodiments, the CHa is of an IgG1 subtype. In some embodiments, the CHb is of an IgG1 subtype.

In some embodiments, the CHa and/or the CHb are of an IgG1 subtype comprising one or more of the following amino acid mutations: Y349C, S354C, T366W, T366S, L368A, and Y407V, wherein amino acid positions are numbered according to the Eu numbering scheme. In some embodiments, the CHa and/or the CHb is of an IgG1 subtype comprising the following amino acid mutation: N297A, wherein an amino acid position is numbered according to the Eu numbering scheme.

In some embodiments, one heavy chain constant region of the CHa and the CHb comprises one or more of the following amino acid mutations: N297A, Y349C, T366S, L368A, and Y407V

In some embodiments, the other heavy chain constant region of the CHa and the CHb comprises one or more of the following amino acid mutations: N297A, S354C, and T366W. In some embodiments, the CHa comprises an amino acid sequence set forth in SEQ ID NO: 13 or 19, or an amino acid sequence having at least 80% or 90% identity to the amino acid sequence set forth in SEQ ID NO: 13 or 19, or an amino acid sequence having one or more conservative amino acid substitutions as compared with the amino acid sequence set forth in SEQ ID NO: 13 or 19; the CHb comprises an amino acid sequence set forth in SEQ ID NO: 15 or 20, or an amino acid sequence having at least 80% or 90% identity to the amino acid sequence set forth in SEQ ID NO: 15 or 20, or an amino acid sequence having one or more conservative amino acid substitutions as compared with the amino acid sequence set forth in SEQ ID NO: 15 or 20.

In some embodiments, the CHa comprises an amino acid sequence set forth in SEQ ID NO: 13 or 19, or an amino acid sequence having at least 80% or 90% identity to the amino acid sequence set forth in SEQ ID NO: 13 or 19, or an amino acid sequence having one or more conservative amino acid substitutions as compared with the amino acid sequence set forth in SEQ ID NO: 13 or 19.

In some embodiments, the CHb comprises an amino acid sequence set forth in SEQ ID NO: 15 or 20, or an amino acid sequence having at least 80% or 90% identity to the amino acid sequence set forth in SEQ ID NO: 15 or 20, or an amino acid sequence having one or more conservative amino acid substitutions as compared with the amino acid sequence set forth in SEQ ID NO: 15 or 20.

In some embodiments, the CHa comprises an amino acid sequence set forth in SEQ ID NO: 13, and the CHb comprises an amino acid sequence set forth in SEQ ID NO: 15. In some embodiments, the CHa comprises an amino acid sequence set forth in SEQ ID NO: 19, and the CHb comprises an amino acid sequence set forth in SEQ ID NO: 20.

In some embodiments, the CLa comprises an amino acid sequence set forth in SEQ ID NO: 14, or an amino acid sequence having at least 80% or 90% identity to the amino acid sequence set forth in SEQ ID NO: 14, or an amino acid sequence having one or more conservative amino acid substitutions as compared with the amino acid sequence set forth in SEQ ID NO: 14. The CLb comprises an amino acid sequence set forth in SEQ ID NO: 14, or an amino acid sequence having at least 80% or 90% identity to the amino acid sequence set forth in SEQ ID NO: 14, or an amino acid sequence having one or more conservative amino acid substitutions as compared with the amino acid sequence set forth in SEQ ID NO: 14.

In some embodiments, the heavy chain a comprises an amino acid sequence set forth in SEQ ID NO: 16 or 21, or an amino acid sequence having at least 80% or 90% identity to the amino acid sequence set forth in SEQ ID NO: 16 or 21, or an amino acid sequence having one or more conservative amino acid substitutions as compared with the amino acid sequence set forth in SEQ ID NO: 16 or 21; the heavy chain b comprises an amino acid sequence set forth in SEQ ID NO: 17 or 22, or an amino acid sequence having at least 80% or 90% identity to the amino acid sequence set forth in SEQ ID NO: 17 or 22, or an amino acid sequence having one or more conservative amino acid substitutions as compared with the amino acid sequence set forth in SEQ ID NO: 17 or 22. In some embodiments, the light chain a and the light chain b comprise identical amino acid sequences. In some embodiments, the light chain a and the light chain b each comprise an amino acid sequence set forth in SEQ ID NO: 18, or an amino acid sequence having at least 80% or 90% identity to the amino acid sequence set forth in SEQ ID NO: 18, or an amino acid sequence having one or more conservative amino acid substitutions as compared with the amino acid sequence set forth in SEQ ID NO: 18.

In some embodiments, the anti-PD-L1/CD47 bispecific antibody or the antigen-binding fragment provided herein has an affinity index KD of ≤ 10 nM for PD-L1. In some embodiments, the anti-PD-L1/CD47 bispecific antibody or the antigen-binding fragment provided herein has an affinity index KD of ≤ 1 nM for PD-L1. In some embodiments, the anti-PD-L1/CD47 bispecific antibody or the antigen-binding fragment provided herein has an affinity index KD of ≤ 0.5 nM for PD-L1. In some embodiments, the anti-PD-L1/CD47 bispecific antibody or the antigen-binding fragment provided herein has an affinity index KD of ≤ 0.2 nM for PD-L1. In some embodiments, the anti-PD-L1/CD47 bispecific antibody or the antigen-binding fragment provided herein has an affinity index KD of ≤ 10 nM for CD47. In some embodiments, the anti-PD-L1/CD47 bispecific antibody or the antigen-binding fragment provided herein has an affinity index KD of ≤ 5 nM for CD47. In some embodiments, the anti-PD-L1/CD47 bispecific antibody or the antigen-binding fragment provided herein has an affinity index KD of ≤ 4 nM for CD47. In some embodiments, the anti-PD-L1/CD47 bispecific antibody or the antigen-binding fragment provided herein binds to PD-L1 with greater affinity than to CD47.

In another aspect, the present invention provides the anti-PD-L1/CD47 bispecific antibody or the antigen-binding fragment, wherein the anti-PD-L1/CD47 bispecific antibody or the antigen-binding fragment comprises a variable region a specifically binding to PD-L1, a variable region b specifically binding to CD47, a light chain constant region, and a heavy chain constant region; wherein the heavy chain constant region is of an IgG1 subtype, or the IgG1 subtype comprising one or more of the following amino acid mutations: Y349C, S354C, T366W, T366S, L368A and Y407V, wherein amino acid positions are numbered according to the Eu numbering scheme.

In some embodiments, the heavy chain constant region is of an IgG1 subtype further comprising the following amino acid mutation: N297A, wherein an amino acid position is numbered according to the Eu numbering scheme.

In some embodiments, the heavy chain constant region comprises a first heavy chain constant region and a second heavy chain constant region, wherein the first heavy chain constant region comprises one or more of the following amino acid mutations:

N297A, S354C, and T366W; and/or
the second heavy chain constant region comprises one or more of the following amino acid mutations:
N297A, Y349C, T366S, L368A, and Y407V

In some embodiments, the first heavy chain constant region comprises an amino acid sequence set forth in SEQ ID NO: 13 or 19, or an amino acid sequence having at least 80% or 90% identity to the amino acid sequence set forth in SEQ ID NO: 13 or 19, or an amino acid sequence having one or more conservative amino acid substitutions as compared with the amino acid sequence set forth in SEQ ID NO: 13 or 19; and/or the second heavy chain constant region comprises an amino acid sequence set forth in SEQ ID NO: 15 or 20, or an amino acid sequence having at least 80% or 90% identity to the amino acid sequence set forth in SEQ ID NO: 15 or 20, or an amino acid sequence having one or more conservative amino acid substitutions as compared with the amino acid sequence set forth in SEQ ID NO: 15 or 20.

In some embodiments, the VHa in the variable region a is linked to the first heavy chain constant region, and the VHb in the variable region b is linked to the second heavy chain constant region. In some embodiments, the VHa in the variable region a is linked to the second heavy chain constant region, and the VHb in the variable region b is linked to the first heavy chain constant region.

In some embodiments, the light chain constant region comprises an amino acid sequence set forth in SEQ ID NO: 14, or an amino acid sequence having at least 80% or 90% identity to the amino acid sequence set forth in SEQ ID NO: 14, or an amino acid sequence having one or more conservative amino acid substitutions as compared with the amino acid sequence set forth in SEQ ID NO: 14.

In some embodiments, the anti-PD-L1/CD47 bispecific antibody or the antigen-binding fragment comprises the variable region a described herein and the variable region b described herein.

In some embodiments, the anti-PD-L1/CD47 bispecific antibody or the antigen-binding fragment comprises one heavy chain a, one heavy chain b, and two identical light chains.

In some embodiments, the variable region (VL) of the light chain comprises VL CDR1, VL CDR2, and VL CDR3; wherein the VL CDR1 comprises an amino acid sequence set forth in SEQ ID NO: 4, or an amino acid sequence having at least 80% or 90% identity to the amino acid sequence set forth in SEQ ID NO: 4, or an amino acid sequence having one or more conservative amino acid substitutions as compared with the amino acid sequence set forth in SEQ ID NO: 4; the VL CDR2 comprises an amino acid sequence set forth in SEQ ID NO: 5, or an amino acid sequence having at least 80% or 90% identity to the amino acid sequence set forth in SEQ ID NO: 5, or an amino acid sequence having one or more conservative amino acid substitutions as compared with the amino acid sequence set forth in SEQ ID NO: 5; the VL CDR3 comprises an amino acid sequence set forth in SEQ ID NO: 6, or an amino acid sequence having at least 80% or 90% identity to the amino acid sequence set forth in SEQ ID NO: 6, or an amino acid sequence having one or more conservative amino acid substitutions as compared with the amino acid sequence set forth in SEQ ID NO: 6.

In some embodiments, the VL CDR1 comprises an amino acid sequence set forth in SEQ ID NO: 4 or an amino acid sequence having one or more conservative amino acid substitutions as compared with the amino acid sequence set forth in SEQ ID NO: 4. The VL CDR2 comprises an amino acid sequence set forth in SEQ ID NO: 5 or an amino acid sequence having one or more conservative amino acid substitutions as compared with the amino acid sequence set forth in SEQ ID NO: 5. The VL CDR3 comprises an amino acid sequence set forth in SEQ ID NO: 6 or an amino acid sequence having one or more conservative amino acid substitutions as compared with the amino acid sequence set forth in SEQ ID NO: 6.

In some embodiments, the VL comprises an amino acid sequence set forth in SEQ ID NO: 12, or an amino acid sequence having at least 80% or 90% identity to the amino acid sequence set forth in SEQ ID NO: 12, or an amino acid sequence having one or more conservative amino acid substitutions as compared with the amino acid sequence set forth in SEQ ID NO: 12.

In some embodiments, the light chain comprises an amino acid sequence set forth in SEQ ID NO: 18, or an amino acid sequence having at least 80% or 90% identity to the amino acid sequence set forth in SEQ ID NO: 18, or an amino acid sequence having one or more conservative amino acid substitutions as compared with the amino acid sequence set forth in SEQ ID NO: 18.

In some embodiments, the heavy chain a is paired with one of the light chains to form a PD-L1 antigen-combining site, and the heavy chain b is paired with the other of the light chains to form a CD47 antigen-combining site.

In some embodiments, the heavy chain a comprises an amino acid sequence set forth in SEQ ID NO: 16 or 21, or an amino acid sequence having at least 80% or 90% identity to the amino acid sequence set forth in SEQ ID NO: 16 or 21, or an amino acid sequence having one or more conservative amino acid substitutions as compared with the amino acid sequence set forth in SEQ ID NO: 16 or 21; the heavy chain b comprises an amino acid sequence set forth in SEQ ID NO: 17 or 22, or an amino acid sequence having at least 80% or 90% identity to the amino acid sequence set forth in SEQ ID NO: 17 or 22, or an amino acid sequence having one or more conservative amino acid substitutions as compared with the amino acid sequence set forth in SEQ ID NO: 17 or 22; the light chain comprises an amino acid sequence set forth in SEQ ID NO: 18, or an amino acid sequence having at least 80% or 90% identity to the amino acid sequence set forth in SEQ ID NO: 18, or an amino acid sequence having one or more conservative amino acid substitutions as compared with the amino acid sequence set forth in SEQ ID NO: 18.

In some embodiments, the heavy chain a comprises an amino acid sequence set forth in SEQ ID NO: 16 or 21, and the heavy chain b comprises an amino acid sequence set forth in SEQ ID NO: 17 or 22; the light chain comprises an amino acid sequence set forth in SEQ ID NO: 18. In some embodiments, the anti-PD-L1/CD47 bispecific antibody or the antigen-binding fragment has an affinity index KD of $\leq$ 1 nM for PD-L1.

In some embodiments, the anti-PD-L1/CD47 bispecific antibody or the antigen-binding fragment has an affinity index KD of $\leq$ 10 nM for CD47.

In some embodiments, the anti-PD-L1/CD47 bispecific antibody or the antigen-binding fragment of the present invention has one or more of the following properties:

(a) In some embodiments, the anti-PD-L1/CD47 bispecific antibody of the present invention can bind to PD-L1 and CD47 simultaneously, and maintains the affinity constant of the parent antibody, thereby being capable of blocking the PD1/PD-L1 signaling pathway and blocking the SIRPα/CD47 signaling pathway.

(b) In some embodiments, the anti-PD-L1/CD47 bispecific antibody of the present invention can bind to PD-L1 with ultra-high affinity (e.g., KD = 0.174 nM), and bind to CD47 with high affinity (e.g., KD = 3.78 nM); the selective binding of the anti-PD-L1/CD47 bispecific antibody of the present invention to tumor cells is promoted by the specific binding to PD-L1 on the tumor cells, the binding to CD47 expressed in many normal tissues is avoided, and the side effects are reduced; the effective dose range of the anti-PD-L1/CD47 bispecific antibody of the present invention can be significantly expanded by making the affinity for CD47 much lower than the affinity for PD-L1.

(c) In some embodiments, for the anti-PD-L1/CD47 bispecific antibody of the present invention, a common light chain capable of avoiding pairing of unrelated heavy and light chains is designed.

(d) In some embodiments, for the anti-PD-L1/CD47 bispecific antibody of the present invention, amino acid residues capable of stabilizing the structure of the tetra-chain antibody and facilitating correct coupling or pairing between the chains are designed. In some embodiments, the anti-PD-L1/CD47 bispecific antibody of the present invention comprises Y349C and S354C, or S354C and Y349C in the respective Fc domains; the anti-PD-L1/CD47 bispecific antibody of the present invention comprises a protuberance ("knob") or a cavity ("hole") in the respective Fc domains, and the protuberance or cavity in the Fc domain of the first polypeptide chain can be placed in the cavity or protuberance in the Fc domain of the third polypeptide chain, respectively, and thus the first polypeptide chain and the third polypeptide chain form a stable association of "knob-into-hole" with each other; wherein the first polypeptide chain comprises the structure VHa-CHa and can specifically recognize and bind to PD-L1, and the third polypeptide chain comprises the structure VHb-CHb and can specifically recognize and bind to CD47.

(e) In some embodiments, the anti-PD-L1/CD47 bispecific antibody of the present invention has such advantages as easy stable expression in culture cells *in vitro,* good thermal stability, high antibody yield and purity, and no need of a complex production process.

(f) The anti-PD-L1/CD47 bispecific antibody or the antigen-binding fragment of the present invention binds to cells expressing human PD-L1, in some embodiments, e.g., binds to cells expressing human PD-L1 with an $EC_{50}$ of less than or equal to about 2 nM, 1 nM, 0.9 nM, 0.8 nM, 0.7 nM, 0.6 nM, 0.5 nM, 0.4 nM, 0.3 nM, 0.2 nM, or 0.1 nM. In some embodiments, the binding is determined by flow cytometry (e.g., FACS). In some embodiments, the cells expressing human PD-L1 are CHO-S cells expressing human PD-L1.

(g) In some embodiments, the anti-PD-L1/CD47 bispecific antibody or the antigen-binding fragment of the present invention blocks the relevant activity of PD-L1/PD-1, e.g., blocks the relevant activity of PD-L1/PD-1 with an $EC_{50}$ of less than or equal to about 0.3 μg/mL, 0.2 μg/mL, or 0.1 μg/mL, or with an $EC_{50}$ of about 0.1-3 μg/mL, 0.1-0.4 μg/mL, or 0.1-0.3 μg/mL. In some embodiments, the relevant activity of PD-L1/PD-1 refers to the activation of the inhibitory signals in the intracellular domain of PD1 after PD-L1 binds to PD-1. In some embodiments, the anti-PD-L1/CD47 bispecific antibody or the antigen-binding fragment of the present invention inhibits the binding of PD-L1 to PD-1 in an MOA assay with an $EC_{50}$ of less than or equal to or about less than or equal to about 0.3 μg/mL, 0.2

μg/mL, or 0.1 μg/mL, or with an $EC_{50}$ of about 0.1-3 μg/mL, 0.1-0.4 μg/mL, or 0.1-0.3 μg/mL. In some embodiments, the cells are CHO cells overexpressing human PD-L1.

(h) The anti-PD-L1/CD47 bispecific antibody of the present invention does not cause significant cell agglutination, for example, the anti-PD-L1/CD47 bispecific antibody of the present invention does not cause significant hemagglutination of red blood cells. In some embodiments, if the level of agglutination in the presence of the anti-PD-L1/CD47 bispecific antibody of the present invention decreases by at least 5%, at least 10%, at least 20%, at least 30%, at least 40%, at least 50%, at least 60%, at least 70%, at least 80%, at least 90%, or at least 99% as compared with the level of agglutination in the presence of the anti-CD47 positive control antibody Hu5F9-G4, it indicates that the anti-PD-L1/CD47 bispecific antibody of the present invention does not cause significant agglutination. Preferably, the anti-PD-L1/CD47 bispecific antibody of the present invention does not cause significant cell agglutination when at an antibody concentration between 400 pM and 800 nM.

(i) The anti-PD-L1/CD47 bispecific antibody of the present invention does not bind to human red blood cells. In one embodiment, if the level of binding to human red blood cells in the presence of the anti-PD-L1/CD47 bispecific antibody of the present invention decreases by at least 5%, at least 10%, at least 20%, at least 30%, at least 40%, at least 50%, at least 60%, at least 70%, at least 80%, at least 90%, or at least 99% as compared with the level of binding to human red blood cells in the presence of the anti-CD47 positive control antibody Hu5F9-G4, it indicates that the anti-PD-L1/CD47 bispecific antibody of the present invention does not bind to human red blood cells significantly. Preferably, the anti-PD-L1/CD47 bispecific antibody of the present invention does not bind to human red blood cells significantly when at an antibody concentration between 200 pM and 100 nM.

(j) In some embodiments, the anti-PD-L1/CD47 bispecific antibody of the present invention is a blocking antibody that blocks the binding of CD47 to SIRPα. In some embodiments, the ability of macrophages to phagocytose tumor cells is increased by at least 5%, at least 10%, at least 20%, at least 30%, at least 40%, at least 50%, at least 60%, at least 70%, at least 80%, at least 90% or at least 99% in the presence of the anti-PD-L1/CD47 bispecific antibody of the present invention.

(k) In some embodiments, the anti-PD-L1/CD47 bispecific antibody or the antigen-binding fragment of the present invention can induce antibody-dependent cell-mediated cytotoxicity (ADCC). In one embodiment, the anti-PD-L1/CD47 bispecific antibody or the antigen-binding fragment of the present invention cannot induce antibody-dependent cell-mediated cytotoxicity (ADCC).

(l) The anti-PD-L1/CD47 bispecific antibody or the antigen-binding fragment of the present invention synergistically inhibits one or more activities of PD-L1 and CD47.

In some embodiments, the anti-PD-L1/CD47 bispecific antibody of the present invention has four peptide chains, namely two heavy chains and two light chains. One heavy chain comprises VHa, and the other heavy chain comprises VHb; one light chain comprises VLa, and the other light chain comprises VLb; wherein the variable region a composed of VHa and VLa specifically binds to PD-L1, and the variable region b composed of VHb and VLb specifically binds to CD47. In some embodiments, the two light chains are identical light chains.

In some embodiments, the "Knobs-into-Holes" technique (see, e.g., John B. B. Ridgway et al., 'Knobs-into-holes' engineering of antibody CH3 domains for heavy chain heterodimerization, Protein Engineering, 9(7): p.617-21 (1996); Shane Atwell et al., Stable heterodimers form remodeling the domain interface of a homodimer using a phage display library, J. mol. Biol., 270: p.26-35 (1997); Paul Carter, Bispecific human IgG by design, Journal of Immunological Methods, 248, 7-15 (2001); patent US8216805B2) is adopted in the anti-PD-L1/CD47 bispecific antibody of the present invention. This technique enables the modification of the interfaces between different chains of the anti-PD-L1/CD47 bispecific antibody of the present invention, thus facilitating the correct association of the chains of the anti-PD-L1/ CD47 bispecific antibody of the present invention. Generally, this technique involves introducing a "protuberance" (knob) at the interface of one chain" and introducing a corresponding "cavity"("hole") at the interface of the other chain to be paired with, such that the protuberance can be placed at the cavity. The protuberance can be constructed by replacing an amino acid side chain at the interface of the CH3 domain from the heavy chain constant domains of one chain with a relatively large side chain (e.g., amino acid replacement T366W (EU numbering)). The compensating cavity of the same size as, or a similar size to, the protuberance is constructed at the interface of the CH3 domain from the heavy chain constant domains of the other chain to be paired with by replacing a large amino acid side chain with a relatively small side chain (e.g., amino acid replacements T366S, L368A, and Y407V (Eu numbering)). In some embodiments, the Fc domain of one heavy chain comprises Y349C, T366S, L368A, and Y407V, and the Fc domain of the other heavy chain comprises S354C and T366W, thus forming a stable association of "knob-into-hole".

In some embodiments, the Fc region of the anti-PD-L1/CD47 bispecific antibody of the present invention comprises a modification of binding affinity for the Fc receptor. In some embodiments, the Fc receptor is a Fcγ receptor, particularly a human Fcγ receptor. In one embodiment, the Fc receptor is an activating Fc receptor. In some embodiments, the modification reduces the effector function of the bispecific antibody of the present invention. In some embodiments, the effector function is antibody-dependent cell-mediated cytotoxicity (ADCC). In some embodiments, the modification is in the Fc region of the immunoglobulin molecule, particularly in its CH2 region. In some embodiments, the immunoglobulin molecule comprises an amino acid replacement at position 297 (Eu numbering) of an immunoglobulin heavy chain. In a specific embodiment, the amino acid replacement is N297A (see, e.g., J. Lund et al., Oligosaccharide-protein interactions in IgG can modulate recognition by Fc gamma receptors, FASEB. J. 9, 115-119 (1995)).

In some embodiments, the heavy chain of the anti-PD-L1/CD47 bispecific antibody of the present invention further comprises a signal peptide sequence, e.g., MEFGLSWVFLUAII,KGVQC (SEQ ID NO: 23). In some embodiments, the light chain of the anti-PD-L1/CD47 bispecific antibody of the present invention further comprises a signal peptide sequence, e.g., MDMRVLAQLLGLLLLCFPGARC (SEQ ID NO: 24).

In some embodiments, the anti-PD-L1/CD47 bispecific antibody is an antibody BsAb-71-N297A, the heavy chain a of the antibody BsAb-71-N297A comprises an amino acid sequence set forth in SEQ ID NO: 16, the heavy chain b of the antibody BsAb-71-N297A comprises an amino acid sequence set forth in SEQ ID NO: 17, the light chain a and the light chain b of the antibody BsAb-71-N297A have identical amino acid sequences (common light chains), and the light chain a and the light chain b of the antibody BsAb-71-N297A comprises an amino acid sequence set forth in SEQ ID NO: 18.

In some embodiments, the anti-PD-L1/CD47 bispecific antibody is an antibody BsAb-71, the heavy chain a of the antibody BsAb-71 comprises an amino acid sequence set forth in SEQ ID NO: 21, the heavy chain b of the antibody BsAb-71 comprises an amino acid sequence set forth in SEQ ID NO: 22, the light chain a and the light chain b of the antibody BsAb-71 have identical amino acid sequences (common light chains), and the light chain a and the light chain b of the antibody BsAb-71 comprise an amino acid sequence set forth in SEQ ID NO: 18.

The anti-PD-L1/CD47 bispecific antibody or the antigen-binding fragment can be expressed in a host cell by genetic engineering and obtained by purification; the purification can be performed by conventional methods, such as performing centrifugation on the cell suspension and collecting the supernatant, and then performing centrifugation again to further remove impurities. Protein A affinity column, ion exchange column, and other methods can be used for purifying antibody protein. Host cells include, but are not limited to, Chinese hamster ovary cells CHO, or CHO-S, CHO-dhfr-, CHO/DG44 or ExpiCHO obtained from the modification of CHO cells; and human embryonic kidney cells HEK293, or HEK293T, HEK293F or HEK293E obtained from the modification of HEK293 cells.

Antibodies can be purified by well-known techniques, for example, affinity chromatography using protein A or protein G. In addition, the specific antigen targeted by the immunoglobulin, or an epitope thereof, can be immobilized on a column to purify the immune-specific antibody by immunoaffinity chromatography. For purification of immunoglobulins, reference can be made to D. Wilkinson's article (The Scientist, published by the Scientist, Inc., Philadelphia Pa., Vol. 14, No. 8 (Apr. 17, 2000), pp. 25-28).

In some embodiments, the anti-PD-L1/CD47 bispecific antibody is expressed by a CHO cell line. In some embodiments, the method or use comprises: administering to a patient in need thereof an effective amount of the anti-PD-L1/CD47 bispecific antibody or the antigen-binding fragment. In some embodiments, the anti-PD-L1/CD47 bispecific antibody is an antibody BsAb-71-N297A or BsAb-71. In some embodiments, the anti-PD-L1/CD47 bispecific antibody or the antigen-binding fragment is administered at a dose ranging from about 0.1 mg/kg to about 100 mg/kg, and may be administered, for example, twice a week (BIW) to once a month. In some embodiments, the anti-PD-L1/CD47 bispecific antibody or the antigen-binding fragment is administered at a dose of about 0.1 mg/kg to 100 mg/kg each time. In some embodiments, the anti-PD-L1/CD47 bispecific antibody or the antigen-binding fragment is administered once every two days to once every six weeks. In some embodiments, the anti-PD-L1/CD47 bispecific antibody or the antigen-binding fragment is administered once every two days, once every three days, once every four days, once every five days, twice a week, once a week, once every two weeks, once every three weeks, once a month, or once every six weeks. In some embodiments, the anti-PD-L1/CD47 bispecific antibody or the antigen-binding fragment is administered once every two weeks after four weeks of the first administration. In some embodiments, the anti-PD-L1/CD47 bispecific antibody or the antigen-binding fragment is administered at a dose of about 0.1 mg/kg to 100 mg/kg each time. In some embodiments, the anti-PD-L1/CD47 bispecific antibody or the antigen-binding fragment is administered at a dose of about 6 mg to 3000 mg each time.

In some embodiments, the patient has a disorder associated with PD-L1 and CD47. In some embodiments, the patient has an autoimmune disease, an inflammatory disease, an infectious disease (e.g., chronic infectious disease or sepsis), or a tumor (including benign tumor or cancer). In some embodiments, the autoimmune and inflammatory diseases include arthritis, rheumatoid arthritis, multiple sclerosis, psoriasis, psoriatic arthritis, Crohn's disease, inflammatory bowel disease, ulcerative colitis, lupus, systemic lupus erythematosus, juvenile rheumatoid arthritis, juvenile idiopathic arthritis, Graves' disease, Hashimoto's thyroiditis, Addison's disease, celiac sprue, dermatomyositis, multiple sclerosis, myasthenia gravis, pernicious anemia, Sjogren syndrome, type I diabetes, vasculitis, uveitis, and ankylosing spondylitis; the autoinflammatory diseases include familial mediterranean fever, neonatal-onset multisystem inflammatory disease, tumor necrosis factor (TNF) receptor-associated periodic syndrome, deficiency of interleukin-1 receptor antagonist, behcet disease; the cardiovascular diseases include coronary heart disease, coronary artery disease, atherosclerosis, myocardial infarction, heart failure, and left ventricular heart failure. In some embodiments, the cancer includes, but is not limited to, a solid tumor, a hematologic cancer, or a metastatic lesion. In some embodiments, the hematologic cancer includes, but is not limited to, leukemia, lymphoma, or myeloma. In some embodiments, the leukemia includes, but is not limited to, acute lymphocytic leukemia (ALL), acute myeloid leukemia (AML), chronic lymphocytic leukemia (CLL), chronic myelogenous leukemia (CML), myeloproliferative diseases/tumors (MPDs), and myelodysplastic syndrome. In some embodiments, the lymphoma includes, but is not limited to, diffuse large B-cell lymphoma, Hodgkin's lymphoma, non-Hodgkin's lymphoma, Burkitt's lymphoma, and follicular lymphoma (both small and large cell lymphomas). In some embodiments, the myeloma includes, but is not limited to, multiple myeloma (MM), giant cell myeloma, heavy chain myeloma, and light chain or Bence-Jones myeloma. In some embodiments, the solid tumor includes, but is not limited to, breast cancer, ovarian cancer, lung cancer, prostate cancer, melanoma, colorectal cancer, head and neck cancer, bladder cancer, esophageal cancer, liver cancer, renal cancer, gastric cancer, cervical cancer, pancreatic cancer, thyroid cancer, neuroglioma, salivary gland cancer, leiomyosarcoma, thymus cancer, or epithelial cancer. In some embodiments, the cancer is a pathologically confirmed locally advanced or metastatic malignant solid tumor for which there is no effective treatment available.

In some embodiments, the present invention discloses a method for treating an autoimmune disease, an acute or chronic inflammatory disease, an infectious disease, or a tumor in a patient in need thereof, which comprises administering an effective amount of the anti-PD-L1/CD47 bispecific antibody or the antigen-binding fragment, wherein the anti-PD-L1/CD47 bispecific antibody or the antigen-binding fragment is administered at a dose of about 6 mg to 3000 mg per treatment cycle. In some embodiments, one treatment cycle is for one week, two weeks, three weeks, four weeks, five weeks, six weeks, seven weeks, or a range between any two of these values (inclusive) or any value therein. In some embodiments, the anti-PD-L1/CD47 bispecific antibody is BsAb-71-N297A or BsAb-71.

In some embodiments, the anti-PD-L1/CD47 bispecific antibody may be formulated into a pharmaceutical composition and administered to a patient in a variety of forms suitable for the selected route of administration, for example, orally, rectally, parenterally, intracisternally, intravaginally, intraperitoneally, topically (e.g., via powders, ointments, drops, or transdermal patches), bucally, or by oral or nasal spray. In some embodiments, the anti-PD-L1/CD47 bispecific antibody may be infused intravenously or injected subcutaneously.

The term "parenteral" as used herein refers to modes of administration including intravenous, intramuscular, intraperitoneal, intrasternal, subcutaneous and intraarticular injections and infusions.

The mode of administration may be systemic or local. In addition, it may be desired to introduce the antibody of the present invention into the central nervous system by any suitable route, including intracerebroventricular and intrathecal injections; intracerebroventricular injection may be assisted by an intracerebroventricular catheter connected to, for example, a reservoir (which may be an Ommaya reservoir). Pulmonary administration is also possible, for example by use of an inhaler or nebulizer, and by use of nebulized formulations.

In some embodiments, the anti-PD-L1/CD47 bispecific antibody of the present invention may be administered locally to an area in need of treatment; this may be achieved by (but not limited to) the following: local infusion during surgery (e.g., local application in combination with a post-operative wound dressing), by injection, by means of a catheter, by means of a suppository, or by means of an implant, the implant being of a porous, non-porous, or gelatinous material, including membranes (such as silicone rubber membranes) or fibers. Preferably, when administering a protein (including an antibody) of the present invention, care must be taken to use materials that do not absorb the protein.

Various known delivery systems can be used to administer the anti-PD-L1/CD47 bispecific antibody of the present invention, e.g., encapsulation in liposomes, microparticles, microcapsules, recombinant cells capable of expressing the compound, receptor-mediated endocytosis (see, e.g., Wu and Wu, 1987, J. Biol. Chem. 262:4429-4432), construction of nucleic acids as part of a retrovirus or other vectors, or the like. The dose of the anti-PD-L1/CD47 bispecific antibody will depend on the properties of the drug, the extent to which the internalization, transport and release of the drug are triggered at the cell surface, and the disease being treated and the conditions of the patient (e.g., age, sex, and body weight).

In some embodiments, the anti-PD-L1/CD47 bispecific antibody (such as antibody BsAb-71-N297A or BsAb-71) at a dose of about 0.1 mg/kg to 100 mg/kg, about 0.1 mg/kg to 40 mg/kg, about 0.1 mg/kg to 20 mg/kg, about 0.1 mg/kg to 10 mg/kg, about 0.1 mg/kg to 3 mg/kg, about 0.1 mg/kg to 1 mg/kg, about 0.1 mg/kg to 0.3 mg/kg, about 0.3 mg/kg to 100 mg/kg, about 0.3 mg/kg to 40 mg/kg, about 0.3 mg/kg to 20 mg/kg, about 0.3 mg/kg to 10 mg/kg, about 0.3 mg/kg to 3 mg/kg, about 0.3 mg/kg to 1 mg/kg, about 1 mg/kg to 100 mg/kg, about 1 mg/kg to 40 mg/kg, about 1 mg/kg to 20 mg/kg, about 1 mg/kg to 10 mg/kg, about 1 mg/kg to 3 mg/kg, about 3 mg/kg to 100 mg/kg, about 3 mg/kg to 40 mg/kg, about 3 mg/kg to 20 mg/kg, about 3 mg/kg to 10 mg/kg, about 10 mg/kg to 100 mg/kg, about 10 mg/kg to 40 mg/kg, about 10 mg/kg to 20 mg/kg, about 20 mg/kg to 100 mg/kg, about 20 mg/kg to 40 mg/kg, about 40 mg/kg to 100 mg/kg, or a formulation containing the anti-PD-L1/CD47 bispecific antibody at this dose is administered each time. In some embodiments, the anti-PD-L1/CD47 bispecific antibody at a dose of about 0.1 mg/kg, about 0.2 mg/kg, about 0.3 mg/kg, about 0.5 mg/kg, about 0.9 mg/kg, about 1 mg/kg, about 2 mg/kg, about 2.5 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg, about 20 mg/kg, about 21 mg/kg, about 22 mg/kg, about 23 mg/kg, about 24 mg/kg, about 25 mg/kg, about 26 mg/kg, about 27 mg/kg, about 28 mg/kg, about 29 mg/kg, about 30 mg/kg, about 31 mg/kg, about 32 mg/kg, about 33 mg/kg, about 34 mg/kg, about 35 mg/kg, about 36 mg/kg, about 37 mg/kg, about 38 mg/kg, about 39 mg/kg, about 40 mg/kg, about 41 mg/kg, about 42 mg/kg, about 43 mg/kg, about 44 mg/kg, about 45 mg/kg, about 46 mg/kg, about 47 mg/kg, about 48 mg/kg, about 49 mg/kg, about 50 mg/kg, about 51 mg/kg, about 52 mg/kg, about 53 mg/kg, about 54 mg/kg, about 55 mg/kg, about 56 mg/kg, about 57 mg/kg, about 58 mg/kg, about 59 mg/kg, about 60 mg/kg, about 61 mg/kg, about 62 mg/kg, about 63 mg/kg, about 64 mg/kg, about 65 mg/kg, about 66 mg/kg, about 67 mg/kg, about 68 mg/kg, about 69 mg/kg, about 70 mg/kg, about 71 mg/kg, about 72 mg/kg, about 73 mg/kg, about 74 mg/kg, about 75 mg/kg, about 76 mg/kg, about 77 mg/kg, about 78 mg/kg, about 79 mg/kg, about 80 mg/kg, about 81 mg/kg, about 82 mg/kg, about 83 mg/kg, about 84 mg/kg, about 85 mg/kg, about 86 mg/kg, about 87 mg/kg, about 88 mg/kg, about 89 mg/kg, about 90 mg/kg, about 91 mg/kg, about 92 mg/kg, about 93 mg/kg, about 94 mg/kg, about 95 mg/kg, about 96 mg/kg, about 97 mg/kg, about 98 mg/kg, about 99 mg/kg, about 100 mg/kg, or a range between any two of these values (inclusive) or any value therein, or a formulation containing the anti-PD-L1/CD47 bispecific antibody at this dose is administered each time. In some embodiments, the administration is performed once about a week, once about every two weeks, once about every three weeks, once about every four weeks, once about every five weeks, once about every six weeks, or once about every seven weeks.

In some embodiments, the present invention discloses a method for treating an autoimmune disease, an acute or chronic inflammatory disease, an infectious disease, or a tumor, which comprises: administering to a patient in need thereof the antibody BsAb-71-N297A or BsAb-71 at a dose of about 0.1 mg/kg, about 0.2 mg/kg, about 0.3 mg/kg, about 0.5 mg/kg, about 0.9 mg/kg, about 1 mg/kg, about 2 mg/kg, about 2.5 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg, about 20 mg/kg, about 21 mg/kg, about 22 mg/kg, about 23 mg/kg, about 24 mg/kg, about 25 mg/kg, about 26 mg/kg, about 27 mg/kg, about 28 mg/kg, about 29 mg/kg, about 30 mg/kg, about 31 mg/kg, about 32 mg/kg, about 33 mg/kg, about 34 mg/kg, about 35 mg/kg, about 36 mg/kg, about 37 mg/kg, about 38 mg/kg, about 39 mg/kg, about 40 mg/kg, about 41 mg/kg, about 42 mg/kg, about 43 mg/kg, about 44 mg/kg, about 45 mg/kg, about 46 mg/kg, about 47 mg/kg, about 48 mg/kg, about 49 mg/kg, about 50 mg/kg, about 51 mg/kg, about 52 mg/kg, about 53 mg/kg, about 54 mg/kg, about 55 mg/kg, about 56 mg/kg, about 57 mg/kg, about 58 mg/kg, about 59 mg/kg, about 60 mg/kg, about 61 mg/kg, about 62 mg/kg, about 63 mg/kg, about 64 mg/kg, about 65 mg/kg, about 66 mg/kg, about 67 mg/kg, about 68 mg/kg, about 69 mg/kg, about 70 mg/kg, about 71 mg/kg, about 72 mg/kg, about 73 mg/kg, about 74 mg/kg, about 75 mg/kg, about 76 mg/kg, about 77 mg/kg, about 78 mg/kg, about 79 mg/kg, about 80 mg/kg, about 81 mg/kg, about 82 mg/kg, about 83 mg/kg, about 84 mg/kg, about 85 mg/kg, about 86 mg/kg, about 87 mg/kg, about 88 mg/kg, about 89 mg/kg, about 90 mg/kg, about 91 mg/kg, about 92 mg/kg, about 93 mg/kg, about 94 mg/kg, about 95 mg/kg, about 96 mg/kg, about 97 mg/kg, about 98 mg/kg, about 99 mg/kg, about 100 mg/kg, or a range between any two of these values (inclusive) or any value therein, or a formulation containing the antibody BsAb-71-N297A or BsAb-71 at this dose twice a week.

In some embodiments, the present invention discloses a method for treating an autoimmune disease, an acute or chronic inflammatory disease, an infectious disease, or a tumor, which comprises: administering to a patient in need thereof the antibody BsAb-71-N297A or BsAb-71 at a dose of about 0.1 mg/kg, about 0.2 mg/kg, about 0.3 mg/kg, about 0.5 mg/kg, about 0.9 mg/kg, about 1 mg/kg, about 2 mg/kg, about 2.5 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg, about 20 mg/kg, about 21 mg/kg, about 22 mg/kg, about 23 mg/kg, about 24 mg/kg, about 25 mg/kg, about 26 mg/kg, about 27 mg/kg, about 28 mg/kg, about 29 mg/kg, about 30 mg/kg, about 31 mg/kg, about 32 mg/kg, about 33 mg/kg, about 34 mg/kg, about 35 mg/kg, about 36 mg/kg, about 37 mg/kg, about 38 mg/kg, about 39 mg/kg, about 40 mg/kg, about 41 mg/kg, about 42 mg/kg, about 43 mg/kg, about 44 mg/kg, about 45 mg/kg, about 46 mg/kg, about 47 mg/kg, about 48 mg/kg, about 49 mg/kg, about 50 mg/kg, about 51 mg/kg, about 52 mg/kg, about 53 mg/kg, about 54 mg/kg, about 55 mg/kg, about 56 mg/kg, about 57 mg/kg, about 58 mg/kg, about 59 mg/kg, about 60 mg/kg, about 61 mg/kg, about 62 mg/kg, about 63 mg/kg, about 64 mg/kg, about 65 mg/kg, about 66 mg/kg, about 67 mg/kg, about 68 mg/kg, about 69 mg/kg, about 70 mg/kg, about 71 mg/kg, about 72 mg/kg, about 73 mg/kg, about 74 mg/kg, about 75 mg/kg, about 76 mg/kg, about 77 mg/kg, about 78 mg/kg, about 79 mg/kg, about 80 mg/kg, about 81 mg/kg, about 82 mg/kg, about 83 mg/kg, about 84 mg/kg, about 85 mg/kg, about 86 mg/kg, about 87 mg/kg, about 88 mg/kg, about 89 mg/kg, about 90 mg/kg, about 91 mg/kg, about 92 mg/kg, about 93 mg/kg, about 94 mg/kg, about 95 mg/kg, about 96 mg/kg, about 97 mg/kg, about 98 mg/kg, about 99 mg/kg, about 100 mg/kg, or a range between any two of these values (inclusive) or any value therein, or a formulation containing the antibody BsAb-71-N297A or BsAb-71 at this dose once a week.

In some embodiments, the present invention discloses a method for treating an autoimmune disease, an acute or chronic inflammatory disease, an infectious disease, or a tumor, which comprises: administering to a patient in need thereof the antibody BsAb-71-N297A or BsAb-71 at a dose of about 0.1 mg/kg, about 0.2 mg/kg, about 0.3 mg/kg, about 0.5 mg/kg, about 0.9 mg/kg, about 1 mg/kg, about 2 mg/kg, about 2.5 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg, about 20 mg/kg, about 21 mg/kg, about 22 mg/kg, about 23 mg/kg, about 24 mg/kg, about 25 mg/kg, about 26 mg/kg, about 27 mg/kg, about 28 mg/kg, about 29 mg/kg, about 30 mg/kg, about 31 mg/kg, about 32 mg/kg, about 33 mg/kg, about 34 mg/kg, about 35 mg/kg, about 36 mg/kg, about 37 mg/kg, about 38 mg/kg, about 39 mg/kg, about 40 mg/kg, about 41 mg/kg, about 42 mg/kg, about 43 mg/kg, about 44 mg/kg, about 45 mg/kg, about 46 mg/kg, about 47 mg/kg, about 48 mg/kg, about 49 mg/kg, about 50 mg/kg, about 51 mg/kg, about 52 mg/kg, about 53 mg/kg, about 54 mg/kg, about 55 mg/kg, about 56 mg/kg, about 57 mg/kg, about 58 mg/kg, about 59 mg/kg, about 60 mg/kg, about 61 mg/kg, about 62 mg/kg, about 63 mg/kg, about 64 mg/kg, about 65 mg/kg, about 66 mg/kg, about 67 mg/kg, about 68 mg/kg, about 69 mg/kg, about 70 mg/kg, about 71 mg/kg, about 72 mg/kg, about 73 mg/kg, about 74 mg/kg, about 75 mg/kg, about 76 mg/kg, about 77 mg/kg, about 78 mg/kg, about 79 mg/kg, about 80 mg/kg, about 81 mg/kg, about 82 mg/kg, about 83 mg/kg, about 84 mg/kg, about 85 mg/kg, about 86 mg/kg, about 87 mg/kg, about 88 mg/kg, about 89 mg/kg, about 90 mg/kg, about 91 mg/kg, about 92 mg/kg, about 93 mg/kg, about 94 mg/kg, about 95 mg/kg, about 96 mg/kg, about 97 mg/kg, about 98 mg/kg, about 99 mg/kg, about 100 mg/kg, or a range between any two of these values (inclusive) or any value therein, or a formulation containing the antibody BsAb-71-N297A or BsAb-71 at this dose once every two weeks.

In some embodiments, the present invention discloses a method for treating an autoimmune disease, an acute or chronic inflammatory disease, an infectious disease, or a tumor, which comprises: administering to a patient in need thereof the antibody BsAb-71-N297A or BsAb-71 at a dose of about 0.1 mg/kg, about 0.2 mg/kg, about 0.3 mg/kg, about 0.5 mg/kg, about 0.9 mg/kg, about 1 mg/kg, about 2 mg/kg, about 2.5 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg, about 20 mg/kg, about 21 mg/kg, about 22 mg/kg, about 23 mg/kg, about 24 mg/kg, about 25 mg/kg, about 26 mg/kg, about 27 mg/kg, about 28 mg/kg, about 29 mg/kg, about 30 mg/kg, about 31 mg/kg, about 32 mg/kg, about 33 mg/kg, about 34 mg/kg, about 35 mg/kg, about 36 mg/kg, about 37 mg/kg, about 38 mg/kg, about 39 mg/kg, about 40 mg/kg, about 41 mg/kg, about 42 mg/kg, about 43 mg/kg, about 44 mg/kg, about 45 mg/kg, about 46 mg/kg, about 47 mg/kg, about 48 mg/kg, about 49 mg/kg, about 50 mg/kg, about 51 mg/kg, about 52 mg/kg, about 53 mg/kg, about 54 mg/kg, about 55 mg/kg, about 56 mg/kg, about 57 mg/kg, about 58 mg/kg, about 59 mg/kg, about 60 mg/kg, about 61 mg/kg, about 62 mg/kg, about 63 mg/kg, about 64 mg/kg, about 65 mg/kg, about 66 mg/kg, about 67 mg/kg, about 68 mg/kg, about 69 mg/kg, about 70 mg/kg, about 71 mg/kg, about 72 mg/kg, about 73 mg/kg, about 74 mg/kg, about 75 mg/kg, about 76 mg/kg, about 77 mg/kg, about 78 mg/kg, about 79 mg/kg, about 80 mg/kg, about 81 mg/kg, about 82 mg/kg, about 83 mg/kg, about 84 mg/kg, about 85 mg/kg, about 86 mg/kg, about 87 mg/kg, about 88 mg/kg, about 89 mg/kg, about 90 mg/kg, about 91 mg/kg, about 92 mg/kg, about 93 mg/kg, about 94 mg/kg, about 95 mg/kg, about 96 mg/kg, about 97 mg/kg, about 98 mg/kg, about 99 mg/kg, about 100 mg/kg, or a range between any two of these values (inclusive) or any value therein, or a formulation containing the antibody BsAb-71-N297A or BsAb-71 at this dose once every two weeks after four weeks of the first administration.

In some embodiments, the present invention discloses a method for treating an autoimmune disease, an acute or chronic inflammatory disease, an infectious disease, or a tumor, which comprises: administering to a patient in need thereof the antibody BsAb-71-N297A or BsAb-71 at a dose of about 0.1 mg/kg, about 0.2 mg/kg, about 0.3 mg/kg, about 0.5 mg/kg, about 0.9 mg/kg, about 1 mg/kg, about 2 mg/kg, about 2.5 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg, about 20 mg/kg, about 21 mg/kg, about 22 mg/kg, about 23 mg/kg, about 24 mg/kg, about 25 mg/kg, about 26 mg/kg, about 27 mg/kg, about 28 mg/kg, about 29 mg/kg, about 30 mg/kg, about 31 mg/kg, about 32 mg/kg, about 33 mg/kg, about 34 mg/kg, about 35 mg/kg, about 36 mg/kg, about 37 mg/kg, about 38 mg/kg, about 39 mg/kg, about 40 mg/kg, about 41 mg/kg, about 42 mg/kg, about 43 mg/kg, about 44 mg/kg, about 45 mg/kg, about 46 mg/kg, about 47 mg/kg, about 48 mg/kg, about 49 mg/kg, about 50 mg/kg, about 51 mg/kg, about 52 mg/kg, about 53 mg/kg, about 54 mg/kg, about 55 mg/kg, about 56 mg/kg, about 57 mg/kg, about 58 mg/kg, about 59 mg/kg, about 60 mg/kg, about 61 mg/kg, about 62 mg/kg, about 63 mg/kg, about 64 mg/kg, about 65 mg/kg, about 66 mg/kg, about 67 mg/kg, about 68 mg/kg, about 69 mg/kg, about 70 mg/kg, about 71 mg/kg, about 72 mg/kg, about 73 mg/kg, about 74 mg/kg, about 75 mg/kg, about 76 mg/kg, about 77 mg/kg, about 78 mg/kg, about 79 mg/kg, about 80 mg/kg, about 81 mg/kg, about 82 mg/kg, about 83 mg/kg, about 84 mg/kg, about 85 mg/kg, about 86 mg/kg, about 87 mg/kg, about 88 mg/kg, about 89 mg/kg, about 90 mg/kg, about 91 mg/kg, about 92 mg/kg, about 93 mg/kg, about 94 mg/kg, about 95 mg/kg, about 96 mg/kg, about 97 mg/kg, about 98 mg/kg, about 99 mg/kg, about 100 mg/kg, or a range between any two of these values (inclusive) or any value therein, or a formulation containing the antibody BsAb-71-N297A or BsAb-71 at this dose once every three weeks.

In some embodiments, the present invention discloses a method for treating an autoimmune disease, an acute or chronic inflammatory disease, an infectious disease, or a tumor, which comprises: administering to a patient in need thereof the antibody BsAb-71-N297A or BsAb-71 at a dose of about 0.1 mg/kg, about 0.2 mg/kg, about 0.3 mg/kg, about 0.5 mg/kg, about 0.9 mg/kg, about 1 mg/kg, about 2 mg/kg, about 2.5 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg, about 20 mg/kg, about 21 mg/kg, about 22 mg/kg, about 23 mg/kg, about 24 mg/kg, about 25 mg/kg, about 26 mg/kg, about 27 mg/kg, about 28 mg/kg, about 29 mg/kg, about 30 mg/kg, about 31 mg/kg, about 32 mg/kg, about 33 mg/kg, about 34 mg/kg, about 35 mg/kg, about 36 mg/kg, about 37 mg/kg, about 38 mg/kg, about 39 mg/kg, about 40 mg/kg, about 41 mg/kg, about 42 mg/kg, about 43 mg/kg, about 44 mg/kg, about 45 mg/kg, about 46 mg/kg, about 47 mg/kg, about 48 mg/kg, about 49 mg/kg, about 50 mg/kg, about 51 mg/kg, about 52 mg/kg, about 53 mg/kg, about 54 mg/kg, about 55 mg/kg, about 56 mg/kg, about 57 mg/kg, about 58 mg/kg, about 59 mg/kg, about 60 mg/kg, about 61 mg/kg, about 62 mg/kg, about 63 mg/kg, about 64 mg/kg, about 65 mg/kg, about 66 mg/kg, about 67 mg/kg, about 68 mg/kg, about 69 mg/kg, about 70 mg/kg, about 71 mg/kg, about 72 mg/kg, about 73 mg/kg, about 74 mg/kg, about 75 mg/kg, about 76 mg/kg, about 77 mg/kg, about 78 mg/kg, about 79 mg/kg, about 80 mg/kg, about 81 mg/kg, about 82 mg/kg, about 83 mg/kg, about 84 mg/kg, about 85 mg/kg, about 86 mg/kg, about 87 mg/kg, about 88 mg/kg, about 89 mg/kg, about 90 mg/kg, about 91 mg/kg, about 92 mg/kg, about 93 mg/kg, about 94 mg/kg, about 95 mg/kg, about 96 mg/kg, about 97 mg/kg, about 98 mg/kg, about 99 mg/kg, about 100 mg/kg, or a range between any two of these values (inclusive) or any value therein, or a formulation containing the antibody BsAb-71-N297A or BsAb-71 at this dose once every four weeks.

In some embodiments, the method comprises at least one, at least two, at least three, at least four, at least five, at least six, or at least seven treatment cycles. In some embodiments, one treatment cycle is for at least one week, at least two weeks, at least three weeks, at least four weeks, at least five weeks, at least six weeks, or at least seven weeks. In some embodiments, one treatment cycle is for one week to seven weeks. In some embodiments, one treatment cycle is for one week to six weeks. In some embodiments, one treatment cycle is for one week to five weeks. In some embodiments, one treatment cycle is for two weeks to six weeks. In some embodiments, one treatment cycle is for two weeks to four weeks. In some embodiments, one treatment cycle is for two weeks to three weeks. In some embodiments, the administration is performed twice about a week, or once about a week, once about every two weeks, once about every three weeks, once about every four weeks, once about every five weeks, once about every six weeks, or once about every seven weeks. In some embodiments, the administration is performed once about every two weeks after four weeks of the first administration. In some embodiments, the anti-PD-L1/CD47 bispecific antibody is administered once every two days to once every six weeks. In some embodiments, the anti-PD-L1/CD47 bispecific antibody is administered once every two days, once every three days, once every four days, once every five days, twice a week, once a week, once every two weeks, once every three weeks, once a month, or once every six weeks. In some embodiments, the anti-PD-L1/CD47 bispecific antibody is administered once every two weeks after four weeks of the first administration.

In some embodiments, the present invention discloses a method for treating an autoimmune disease, an acute or chronic inflammatory disease, an infectious disease, or a tumor, which comprises administering to a patient in need thereof an effective amount of the anti-PD-L1/CD47 bispecific antibody or the antigen-binding fragment (or formulation); wherein the anti-PD-L1/CD47 bispecific antibody or the antigen-binding fragment at a dose of about 6 mg to 3000 mg (or a formulation containing the anti-PD-L1/CD47 bispecific antibody or the antigen-binding fragment at this dose) is administered each time. The dosage schedule and administration mode depend on benefit-risk assessment and general clinical practice guidelines for the anti-PD-L1/CD47 bispecific antibody or the antigen-binding fragment (or formulation) in certain patient populations.

In some embodiments, the anti-PD-L1/CD47 bispecific antibody or the antigen-binding fragment at a dose of about 6 mg to 3000 mg (or a formulation containing the anti-PD-L1/CD47 bispecific antibody or the antigen-binding fragment at this dose) is administered to the patient per treatment cycle. In some embodiments, the anti-PD-L1/CD47 bispecific antibody (such as antibody BsAb-71-N297A or BsAb-71) at a dose of about 6 mg, about 7 mg, about 10 mg, about 14 mg, about 18 mg, about 21 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 435 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, about 600 mg, about 610 mg, about 620 mg, about 630 mg, about 640 mg, about 650 mg, about 660 mg, about 670 mg, about 680 mg, about 690 mg, about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, about 1450 mg, about 1500 mg, about 1550 mg, about 1600 mg, about 1650 mg, about 1700 mg, about 1750 mg, about 1800 mg, about 1850 mg, about 1900 mg, about 1950 mg, about 2000 mg, about 2050 mg, about 2100 mg, about 2150 mg, about 2200 mg, about 2250 mg, about 2300 mg, about 2350 mg, about 2400 mg, about 2450 mg, about 2500 mg, about 2550 mg, about 2600 mg, about 2650 mg, about 2700 mg, about 2750 mg, about 2800 mg, about 2850 mg, about 2900 mg, about 2950 mg, or about 3000 mg, or a range between any two of these values (inclusive) or any value therein, or a formulation containing the anti-PD-L1/CD47 bispecific antibody at this dose is administered to the patient per treatment cycle. In some embodiments, one treatment cycle is the administration performed once every one week to seven weeks. In some embodiments, one treatment cycle is the administration performed once every two weeks to four weeks.

In some embodiments, the anti-PD-L1/CD47 bispecific antibody or the antigen-binding fragment at a dose of about 6 mg to 2800 mg, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks). In some embodiments, one treatment cycle is about one week, about two weeks, about three weeks, about four weeks, about five weeks, about six weeks, or about seven weeks. In some embodiments, the anti-PD-L1/CD47 bispecific antibody (such as antibody BsAb-71-N297A or BsAb-71) at a dose of about 6 mg, about 7 mg, about 10 mg, about 18 mg, about 21 mg, about 30 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 434 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 567 mg, about 570 mg, about 580 mg, about 590 mg, about 600 mg, about 610 mg, about 620 mg, about 630 mg, about 640 mg, about 650 mg, about 660 mg, about 670 mg, about 680 mg, about 690 mg, about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, about 1450 mg, about 1500 mg, about 1550 mg, about 1600 mg, about 1650 mg, about 1700 mg, about 1750 mg, about 1800 mg, about 1850 mg, about 1900 mg, about 1950 mg, about 2000 mg, about 2050 mg, about 2100 mg, about 2150 mg, about 2200 mg, about 2250 mg, about 2300 mg, about 2350 mg, about 2400 mg, about 2450 mg, about 2500 mg, about 2550 mg, about 2600 mg, about 2650 mg, about 2700 mg, about 2750 mg, about 2800 mg, or a range between any two of these values (inclusive) or any value therein, or a formulation containing the anti-PD-L1/CD47 bispecific antibody at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody or the antigen-binding fragment at a dose of about 6 mg to 18 mg, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks). In some embodiments, the anti-PD-L1/CD47 bispecific antibody or the antigen-binding fragment at a dose of about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, or a range between any two of these values (inclusive) or any value therein, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody or the antigen-binding fragment at a dose of about 18 mg to 60 mg, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks). In some embodiments, the anti-PD-L1/CD47 bispecific antibody or the antigen-binding fragment at a dose of about 18 mg, about 19 mg, about 20 mg, about 21 mg, about 22 mg, about 23 mg, about 25 mg, about 26 mg, about 27 mg, about 28 mg, about 29 mg, about 30 mg, about 31 mg, about 32 mg, about 33 mg, about 35 mg, 36 mg, about 37 mg, about 38 mg, about 39 mg, about 40 mg, about 41 mg, about 42 mg, about 43 mg, about 45 mg, about 46 mg, about 47 mg, about 48 mg, about 50 mg, about 53 mg, about 55 mg, about 58 mg, about 60 mg, or a range between any two of these values (inclusive) or any value therein, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody or the antigen-binding fragment at a dose of about 60 mg to 180 mg, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks). In some embodiments, the anti-PD-L1/CD47 bispecific antibody or the antigen-binding fragment at a dose of about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, or a range between any two of these values (inclusive) or any value therein, or a formulation containing the anti-PD-L1/CD47 bispecific antibody at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody or the antigen-binding fragment at a dose of about 180 mg to 600 mg, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks). In some embodiments, the anti-PD-L1/CD47 bispecific antibody or the antigen-binding fragment at a dose of about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 320 mg, about 350 mg, about 400 mg, about 450 mg, about 480 mg, about 500 mg, about 520 mg, about 550 mg, about 580 mg, about 600 mg, or a range between any two of these values (inclusive) or any value therein, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody or the antigen-binding fragment at a dose of about 600 mg to 1200 mg, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks). In some embodiments, the anti-PD-L1/CD47 bispecific antibody or the antigen-binding fragment at a dose of about 600 mg, about 650 mg, about 700 mg, about 750 mg, about 800 mg, about 850 mg, about 900 mg, about 950 mg, about 1000 mg, about 1050 mg, about 1100 mg, about 1150 mg, about 1200 mg, or a range between any two of these values (inclusive) or any value therein, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody or the antigen-binding fragment at a dose of about 1200 mg to 2400 mg, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks). In some embodiments, the anti-PD-L1/CD47 bispecific antibody at a dose of about 1200 mg, about 1250 mg, about 1350 mg, about 1400 mg, about 1450 mg, about 1500 mg, about 1600 mg, about 1700 mg, about 1800 mg, about 1900 mg, about 2000 mg, about 2100 mg, about 2200 mg, about 2300 mg, about 2400 mg, or a range between any two of these values (inclusive) or any value therein, or a formulation containing the anti-PD-L1/CD47 bispecific antibody at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody or the antigen-binding fragment at a dose of about 7 mg to 21 mg, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks). In some embodiments, the anti-PD-L1/CD47 bispecific antibody or the antigen-binding fragment at a dose of about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, or a range between any two of these values (inclusive) or any value therein, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody or the antigen-binding fragment at a dose of about 21 mg to 70 mg, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks). In some embodiments, the anti-PD-L1/CD47 bispecific antibody or the antigen-binding fragment at a dose of about 21 mg, about 22 mg, about 23 mg, about 25 mg, about 26 mg, about 27 mg, about 28 mg, about 29 mg, about 30 mg, about 31 mg, about 32 mg, about 33 mg, about 35 mg, 36 mg, about 37 mg, about 38 mg, about 39 mg, about 40 mg, about 41 mg, about 42 mg, about 43 mg, about 45 mg, about 46 mg, about 47 mg, about 48 mg, about 50 mg, about 53 mg, about 55 mg, about 58 mg, about 60 mg, about 61 mg, about 63 mg, about 65 mg, about 68 mg, about 70 mg, or a range between any two of these values (inclusive) or any value therein, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody or the antigen-binding fragment at a dose of about 70 mg to 210 mg, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks). In some embodiments, the anti-PD-L1/CD47 bispecific antibody or the antigen-binding fragment at a dose of about 70 mg, about 75 mg, about 80 mg, about 85 mg, about 90 mg, about 95 mg, about 100 mg, about 105 mg, about 110 mg, about 115 mg, about 120 mg, about 125 mg, about 130 mg, about 135 mg, about 140 mg, about 145 mg, about 150 mg, about 155 mg, about 160 mg, about 165 mg, about 170 mg, about 175 mg, about 180 mg, about 185 mg, about 190 mg, about 195 mg, about 200 mg, about 205 mg, about 210 mg, or a range between any two of these values (inclusive) or any value therein, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody or the antigen-binding fragment at a dose of about 210 mg to 700 mg, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks). In some embodiments, the anti-PD-L1/CD47 bispecific antibody or the antigen-binding fragment at a dose of about 210 mg, about 220 mg, about 230 mg, about 240 mg, about 250 mg, about 260 mg, about 270 mg, about 280 mg, about 290 mg, about 300 mg, about 320 mg, about 350 mg, about 400 mg, about 450 mg, about 480 mg, about 500 mg, about 520 mg, about 550 mg, about 580 mg, about 600 mg, about 620 mg, about 650 mg, about 680 mg, about 700 mg, or a range between any two of these values (inclusive) or any value therein, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody or the antigen-binding fragment at a dose of about 700 mg to 1400 mg, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks). In some embodiments, the anti-PD-L1/CD47 bispecific antibody or the antigen-binding fragment at a dose of about 700 mg, about 720 mg, about 750 mg, about 780 mg, about 800 mg, about 820 mg, about 850 mg, about 880 mg, about 900 mg, about 920 mg, about 950 mg, about 980 mg, about 1000 mg, about 1050 mg, about 1100 mg, about 1150 mg, about 1200 mg, about 1250 mg, about 1300 mg, about 1350 mg, about 1400 mg, or a range between any two of these values (inclusive) or any value therein, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody or the antigen-binding fragment at a dose of about 1400 mg to 2800 mg, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks). In some embodiments, the anti-PD-L1/CD47 bispecific antibody or the antigen-binding fragment at a dose of about 1400 mg, about 1420 mg, about 1450 mg, about 1480 mg, about 1500 mg, about 1550 mg, about 1600 mg, about 1650 mg, about 1700 mg, about 1750 mg, about 1800 mg, about 1850 mg, about 1900 mg, about 1950 mg, about 2000 mg, about 2050 mg, about 2100 mg, about 2150 mg, about 2200 mg, about 2250 mg, about 2300 mg, about 2350 mg, about 2400 mg, about 2450 mg, about 2500 mg, about 2550 mg, about 2600 mg, about 2650 mg, about 2700 mg, about 2750 mg, about 2800 mg, or a range between any two of these values (inclusive) or any value therein, or a formulation containing the anti-PD-L1/CD47 bispecific antibody or the antigen-binding fragment at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody (such as antibody BsAb-71-N297A or BsAb-71) at a dose of about 1 mg to 10 mg (such as about 6 mg for 1 administration or about 7 mg for 1 administration), or a formulation containing the anti-PD-L1/CD47 bispecific antibody at this dose is administered to the patient per treatment cycle. In some embodiments, the anti-PD-L1/CD47 bispecific antibody at a dose of about 6 mg, or a formulation containing the anti-PD-L1/CD47 bispecific antibody at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks). In some embodiments, the anti-PD-L1/CD47 bispecific antibody at a dose of about 7 mg, or a formulation containing the anti-PD-L1/CD47 bispecific antibody at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody (such as antibody BsAb-71-N297A or BsAb-71) at a dose of about 15 mg to 25 mg (such as about 18 mg for 1 administration or about 21 mg for 1 administration), or a formulation containing the anti-PD-L1/CD47 bispecific antibody at this dose is administered to the patient per treatment cycle. In some embodiments, the anti-PD-L1/CD47 bispecific antibody at a dose of about 18 mg, or a formulation containing the anti-PD-L1/CD47 bispecific antibody at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks). In some embodiments, the anti-PD-L1/CD47 bispecific antibody at a dose of about 21 mg, or a formulation containing the anti-PD-L1/CD47 bispecific antibody at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody (such as antibody BsAb-71-N297A or BsAb-71) at a dose of about 55 mg to 75 mg (such as about 60 mg for 1 administration or about 70 mg for 1 administration), or a formulation containing the anti-PD-L1/CD47 bispecific antibody at this dose is administered to the patient per treatment cycle. In some embodiments, the anti-PD-L1/CD47 bispecific antibody at an effective amount of about 60 mg, or a formulation containing the anti-PD-L1/CD47 bispecific antibody at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks). In some embodiments, the anti-PD-L1/CD47 bispecific antibody at an effective amount of about 70 mg, or a formulation containing the anti-PD-L1/CD47 bispecific antibody at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody (such as antibody BsAb-71-N297A or BsAb-71) at a dose of about 150 mg to 230 mg (such as about 180 mg for 1 administration or about 210 mg for 1 administration), or a formulation containing the anti-PD-L1/CD47 bispecific antibody at this dose is administered to the patient per treatment cycle. In some embodiments, the anti-PD-L1/CD47 bispecific antibody at an effective amount of about 180 mg, or a formulation containing the anti-PD-L1/CD47 bispecific antibody at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks). In some embodiments, the anti-PD-L1/CD47 bispecific antibody at an effective amount of about 210 mg, or a formulation containing the anti-PD-L1/CD47 bispecific antibody at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody (such as antibody BsAb-71-N297A or BsAb-71) at a dose of about 590 mg to 610 mg (such as about 600 mg for 1 administration), or a formulation containing the anti-PD-L1/CD47 bispecific antibody at this dose is administered to the patient per treatment cycle. In some embodiments, the anti-PD-L1/CD47 bispecific antibody at a dose of about 600 mg, or a formulation containing the anti-PD-L1/CD47 bispecific antibody at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody (such as antibody BsAb-71-N297A or BsAb-71) at a dose of about 650 mg to 750 mg (such as about 700 mg for 1 administration), or a formulation containing the anti-PD-L1/CD47 bispecific antibody at this dose is administered to the patient per treatment cycle. In some embodiments, the anti-PD-L1/CD47 bispecific antibody at a dose of about 700 mg, or a formulation containing the anti-PD-L1/CD47 bispecific antibody at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody (such as antibody BsAb-71-N297A or BsAb-71) at a dose of about 1150 mg to 1250 mg (such as about 1200 mg for 1 administration), or a formulation containing the anti-PD-L1/CD47 bispecific antibody at this dose is administered to the patient per treatment cycle. In some embodiments, the anti-PD-L1/CD47 bispecific antibody at a dose of about 1200 mg, or a formulation containing the anti-PD-L1/CD47 bispecific antibody at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody (such as antibody BsAb-71-N297A or BsAb-71) at a dose of about 1350 mg to 1450 mg (such as about 1400 mg for 1 administration), or a formulation containing the anti-PD-L1/CD47 bispecific antibody at this dose is administered to the patient per treatment cycle. In some embodiments, the anti-PD-L1/CD47 bispecific antibody at a dose of about 1400 mg, or a formulation containing the anti-PD-L1/CD47 bispecific antibody at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody (such as antibody BsAb-71-N297A or BsAb-71) at a dose of about 2370 mg to 2490 mg (such as about 2400 mg for 1 administration), or a formulation containing the anti-PD-L1/CD47 bispecific antibody at this dose is administered to the patient per treatment cycle. In some embodiments, the anti-PD-L1/CD47 bispecific antibody at a dose of about 2400 mg, or a formulation containing the anti-PD-L1/CD47 bispecific antibody at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody (such as antibody BsAb-71-N297A or BsAb-71) at a dose of about 2750 mg to 2850 mg (such as about 2800 mg for 1 administration), or a formulation containing the anti-PD-L1/CD47 bispecific antibody at this dose is administered to the patient per treatment cycle. In some embodiments, the anti-PD-L1/CD47 bispecific antibody at a dose of about 2800 mg, or a formulation containing the anti-PD-L1/CD47 bispecific antibody at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody (such as antibody BsAb-71-N297A or BsAb-71) at a dose of about 2970 mg to 3050 mg (such as about 3000 mg for 1 administration), or a formulation containing the anti-PD-L1/CD47 bispecific antibody at this dose is administered to the patient per treatment cycle. In some embodiments, the anti-PD-L1/CD47 bispecific antibody at a dose of about 3000 mg, or a formulation containing the anti-PD-L1/CD47 bispecific antibody at this dose is administered to the patient per treatment cycle; wherein one treatment cycle is about one week, about two weeks, about three weeks, about four weeks, about five weeks, about six weeks, about seven weeks, or a range between any two of these values (inclusive), or any value therein (e.g., about two weeks to four weeks).

In some embodiments, the anti-PD-L1/CD47 bispecific antibody (such as antibody BsAb-71-N297A or BsAb-71) is administered at a dose of about 6 mg to 3000 mg once every two weeks. In some embodiments, the anti-PD-L1/CD47 bispecific antibody is administered at a dose of about 6 mg, about 7 mg, about 10 mg, about 14 mg, about 18 mg, about 21 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 435 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, about 600 mg, about 610 mg, about 620 mg, about 630 mg, about 640 mg, about 650 mg, about 660 mg, about 670 mg, about 680 mg, about 690 mg, about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, about 1450 mg, about 1500 mg, about 1550 mg, about 1600 mg, about 1650 mg, about 1700 mg, about 1750 mg, about 1800 mg, about 1850 mg, about 1900 mg, about 1950 mg, about 2000 mg, about 2050 mg, about 2100 mg, about 2150 mg, about 2200 mg, about 2250 mg, about 2300 mg, about 2350 mg, about 2400 mg, about 2450 mg, about 2500 mg, about 2550 mg, about 2600 mg, about 2650 mg, about 2700 mg, about 2750 mg, about 2800 mg, about 2850 mg, about 2900 mg, about 2950 mg, about 3000 mg, once every two weeks.

In some embodiments, the anti-PD-L1/CD47 bispecific antibody (such as antibody BsAb-71-N297A or BsAb-71) is administered at a dose of about 6 mg to 2800 mg once every two weeks. In some embodiments, the anti-PD-L1/CD47 bispecific antibody is administered at a dose of about 6 mg, about 7 mg, about 10 mg, about 14 mg, about 18 mg, about 21 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 435 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, about 600 mg, about 610 mg, about 620 mg, about 630 mg, about 640 mg, about 650 mg, about 660 mg, about 670 mg, about 680 mg, about 690 mg, about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, about 1450 mg, about 1500 mg, about 1550 mg, about 1600 mg, about 1650 mg, about 1700 mg, about 1750 mg, about 1800 mg, about 1850 mg, about 1900 mg, about 1950 mg, about 2000 mg, about 2050 mg, about 2100 mg, about 2150 mg, about 2200 mg, about 2250 mg, about 2300 mg, about 2350 mg, about 2400 mg, about 2450 mg, about 2500 mg, about 2550 mg, about 2600 mg, about 2650 mg, about 2700 mg, about 2750 mg, about 2800 mg, once every two weeks.

In some embodiments, the anti-PD-L1/CD47 bispecific antibody (such as antibody BsAb-71-N297A or BsAb-71) is administered at a dose of about 6 mg to 18 mg once every two weeks. In some embodiments, the anti-PD-L1/CD47 bispecific antibody is administered at a dose of about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, once every two weeks.

In some embodiments, the anti-PD-L1/CD47 bispecific antibody (such as antibody BsAb-71-N297A or BsAb-71) is administered at a dose of about 18 mg to 60 mg once every two weeks. In some embodiments, the anti-PD-L1/CD47 bispecific antibody is administered at a dose of about 18 mg, about 19 mg, about 20 mg, about 21 mg, about 22 mg, about 23 mg, about 25 mg, about 26 mg, about 27 mg, about 28 mg, about 29 mg, about 30 mg, about 31 mg, about 32 mg, about 33 mg, about 35 mg, 36 mg, about 37 mg, about 38 mg, about 39 mg, about 40 mg, about 41 mg, about 42 mg, about 43 mg, about 45 mg, about 46 mg, about 47 mg, about 48 mg, about 50 mg, about 53 mg, about 55 mg, about 58 mg, about 60 mg, once every two weeks.

In some embodiments, the anti-PD-L1/CD47 bispecific antibody (such as antibody BsAb-71-N297A or BsAb-71) is administered at a dose of about 60 mg to 180 mg once every two weeks. In some embodiments, the anti-PD-L1/CD47 bispecific antibody is administered at a dose of about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, once every two weeks.

In some embodiments, the anti-PD-L1/CD47 bispecific antibody (such as antibody BsAb-71-N297A or BsAb-71) is administered at a dose of about 180 mg to 600 mg once every two weeks. In some embodiments, the anti-PD-L1/CD47 bispecific antibody is administered at a dose of about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 320 mg, about 350 mg, about 400 mg, about 450 mg, about 480 mg, about 500 mg, about 520 mg, about 550 mg, about 580 mg, about 600 mg, once every two weeks.

In some embodiments, the anti-PD-L1/CD47 bispecific antibody (such as antibody

BsAb-71-N297A or BsAb-71) is administered at a dose of about 600 mg to 1200 mg once every two weeks. In some embodiments, the anti-PD-L1/CD47 bispecific antibody is administered at a dose of about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 434 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 567 mg, about 570 mg, about 580 mg, about 590 mg, about 600 mg, about 610 mg, about 620 mg, about 630 mg, about 640 mg, about 650 mg, about 660 mg, about 670 mg, about 680 mg, about 690 mg, about 700 mg, about 750 mg, about 800 mg, about 850 mg, about 900 mg, about 950 mg, about 1000 mg, about 1050 mg, about 1100 mg, about 1150 mg, about 1200 mg, once every two weeks.

In some embodiments, the anti-PD-L1/CD47 bispecific antibody (such as antibody BsAb-71-N297A or BsAb-71) is administered at a dose of about 1200 mg to 2400 mg once every two weeks. In some embodiments, the anti-PD-L1/CD47 bispecific antibody is administered at a dose of about 1200 mg, about 1250 mg, about 1300 mg, about 1350 mg, about 1400 mg, about 1450 mg, about 1500 mg, about 1550 mg, about 1600 mg, about 1650 mg, about 1700 mg, about 1750 mg, about 1800 mg, about 1850 mg, about 1900 mg, about 1950 mg, about 2000 mg, about 2050 mg, about 2100 mg, about 2150 mg, about 2200 mg, about 2250 mg, about 2300 mg, about 2350 mg, about 2400 mg, once every two weeks.

In some embodiments, the anti-PD-L1/CD47 bispecific antibody (such as antibody BsAb-71-N297A or BsAb-71) is administered at a dose of about 7 mg to 2800 mg once every two weeks. In some embodiments, the anti-PD-L1/CD47 bispecific antibody is administered at a dose of about 7 mg, about 10 mg, about 14 mg, about 18 mg, about 21 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 435 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, about 600 mg, about 610 mg, about 620 mg, about 630 mg, about 640 mg, about 650 mg, about 660 mg, about 670 mg, about 680 mg, about 690 mg, about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, about 1450 mg, about 1500 mg, about 1550 mg, about 1600 mg, about 1650 mg, about 1700 mg, about 1750 mg, about 1800 mg, about 1850 mg, about 1900 mg, about 1950 mg, about 2000 mg, about 2050 mg, about 2100 mg, about 2150 mg, about 2200 mg, about 2250 mg, about 2300 mg, about 2350 mg, about 2400 mg, about 2450 mg, about 2500 mg, about 2550 mg, about 2600 mg, about 2650 mg, about 2700 mg, about 2750 mg, about 2800 mg, once every two weeks. In some embodiments, the anti-PD-L1/CD47 bispecific antibody (such as antibody BsAb-71-N297A or BsAb-71) is administered at a dose of about 7 mg to 21 mg once every two weeks. In some embodiments, the anti-PD-L1/CD47 bispecific antibody is administered at a dose of about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, once every two weeks.

In some embodiments, the anti-PD-L1/CD47 bispecific antibody (such as antibody BsAb-71-N297A or BsAb-71) is administered at a dose of about 21 mg to 70 mg once every two weeks. In some embodiments, the anti-PD-L1/CD47 bispecific antibody is administered at a dose of about 21 mg, about 22 mg, about 23 mg, about 25 mg, about 26 mg, about 27 mg, about 28 mg, about 29 mg, about 30 mg, about 31 mg, about 32 mg, about 33 mg, about 35 mg, about 36 mg, about 37 mg, about 38 mg, about 39 mg, about 40 mg, about 41 mg, about 42 mg, about 43 mg, about 45 mg, about 46 mg, about 47 mg, about 48 mg, about 50 mg, about 53 mg, about 55 mg, about 58 mg, about 60 mg, about 63 mg, about 65 mg, about 68 mg, about 70 mg, once every two weeks.

In some embodiments, the anti-PD-L1/CD47 bispecific antibody (such as antibody BsAb-71-N297A or BsAb-71) is administered at a dose of about 70 mg to 210 mg once every two weeks. In some embodiments, the anti-PD-L1/CD47 bispecific antibody is administered at a dose of about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, once every two weeks.

In some embodiments, the anti-PD-L1/CD47 bispecific antibody (such as antibody BsAb-71-N297A or BsAb-71) is administered at a dose of about 210 mg to 700 mg once every two weeks. In some embodiments, the anti-PD-L1/CD47 bispecific antibody is administered at a dose of about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 434 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 567 mg, about 570 mg, about 580 mg, about 590 mg, about 600 mg, about 610 mg, about 620 mg, about 630 mg, about 640 mg, about 650 mg, about 660 mg, about 670 mg, about 680 mg, about 690 mg, about 700 mg, once every two weeks. In some embodiments, the anti-PD-L1/CD47 bispecific antibody (such as antibody BsAb-71-N297A or BsAb-71) is administered at a dose of about 700 mg to 1400 mg once every two weeks. In some embodiments, the anti-PD-L1/CD47 bispecific antibody is administered at a dose of about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, once every two weeks.

In some embodiments, the anti-PD-L1/CD47 bispecific antibody (such as antibody BsAb-71-N297A or BsAb-71) is administered at a dose of about 1400 mg to 2800 mg once every two weeks. In some embodiments, the anti-PD-L1/CD47 bispecific antibody is administered at a dose of about 1400 mg, about 1450 mg, about 1500 mg, about 1550 mg, about 1600 mg, about 1650 mg, about 1700 mg, about 1750 mg, about 1800 mg, about 1850 mg, about 1900 mg, about 1950 mg, about 2000 mg, about 2050 mg, about 2100 mg, about 2150 mg, about 2200 mg, about 2250 mg, about 2300 mg, about 2350 mg, about 2400 mg, about 2430 mg, about 2450 mg, about 2480 mg, about 2500 mg, about 2530 mg, about 2550 mg, about 2580 mg, about 2600 mg, about 2630 mg, about 2650 mg, about 2680 mg, about 2700 mg, about 2730 mg, about 2750 mg, about 2780 mg, about 2800 mg, once every two weeks.

In some embodiments, the anti-PD-L1/CD47 bispecific antibody (such as antibody BsAb-71-N297A or BsAb-71) at a dose of about 1 mg to 10 mg, or a formulation containing the anti-PD-L1/CD47 bispecific antibody at this dose is administered once every two weeks. In some embodiments, the anti-PD-L1/CD47 bispecific antibody at a dose of about 6 mg, or a formulation containing the anti-PD-L1/CD47 bispecific antibody at this dose is administered once every two weeks. In some embodiments, the anti-PD-L1/CD47 bispecific antibody at a dose of about 7 mg, or a formulation containing the anti-PD-L1/CD47 bispecific antibody at this dose is administered once every two weeks.

In some embodiments, the anti-PD-L1/CD47 bispecific antibody (such as antibody BsAb-71-N297A or BsAb-71) at a dose of about 15 mg to 25 mg, or a formulation containing the anti-PD-L1/CD47 bispecific antibody at this dose is administered once every two weeks. In some embodiments, the anti-PD-L1/CD47 bispecific antibody at a dose of about 18 mg, or a formulation containing the anti-PD-L1/CD47 bispecific antibody at this dose is administered once every two weeks. In some embodiments, the anti-PD-L1/CD47 bispecific antibody at a dose of about 21 mg, or a formulation containing the anti-PD-L1/CD47 bispecific antibody at this dose is administered once every two weeks.

In some embodiments, the anti-PD-L1/CD47 bispecific antibody (such as antibody BsAb-71-N297A or BsAb-71) at a dose of about 55 mg to 75 mg, or a formulation containing the anti-PD-L1/CD47 bispecific antibody at this dose is administered once every two weeks. In some embodiments, the anti-PD-L1/CD47 bispecific antibody at a dose of about 60 mg, or a formulation containing the anti-PD-L1/CD47 bispecific antibody at this dose is administered once every two weeks. In some embodiments, the anti-PD-L1/CD47 bispecific antibody at a dose of about 70 mg, or a formulation containing the anti-PD-L1/CD47 bispecific antibody at this dose is administered once every two weeks.

In some embodiments, the anti-PD-L1/CD47 bispecific antibody (such as antibody BsAb-71-N297A or BsAb-71) at a dose of about 150 mg to 230 mg, or a formulation containing the anti-PD-L1/CD47 bispecific antibody at this dose is administered once every two weeks. In some embodiments, the anti-PD-L1/CD47 bispecific antibody at a dose of about 180 mg, or a formulation containing the anti-PD-L1/CD47 bispecific antibody at this dose is administered once every two weeks. In some embodiments, the anti-PD-L1/CD47 bispecific antibody at a dose of about 210 mg, or a formulation containing the anti-PD-L1/CD47 bispecific antibody at this dose is administered once every two weeks.

In some embodiments, the anti-PD-L1/CD47 bispecific antibody (such as antibody BsAb-71-N297A or BsAb-71) at a dose of about 590 mg to 610 mg, or a formulation containing the anti-PD-L1/CD47 bispecific antibody at this dose is administered once every two weeks. In some embodiments, the anti-PD-L1/CD47 bispecific antibody at a dose of about 600 mg, or a formulation containing the anti-PD-L1/CD47 bispecific antibody at this dose is administered once every two weeks.

In some embodiments, the anti-PD-L1/CD47 bispecific antibody (such as antibody BsAb-71-N297A or BsAb-71) at a dose of about 690 mg to 710 mg, or a formulation containing the anti-PD-L1/CD47 bispecific antibody at this dose is administered once every two weeks. In some embodiments, the anti-PD-L1/CD47 bispecific antibody at a dose of about 700 mg, or a formulation containing the anti-PD-L1/CD47 bispecific antibody at this dose is administered once every two weeks.

In some embodiments, the anti-PD-L1/CD47 bispecific antibody (such as antibody BsAb-71-N297A or BsAb-71) at a dose of about 1150 mg to 1250 mg, or a formulation containing the anti-PD-L1/CD47 bispecific antibody at this dose is administered once every two weeks. In some embodiments, the anti-PD-L1/CD47 bispecific antibody at a dose of about 1200 mg, or a formulation containing the anti-PD-L1/CD47 bispecific antibody at this dose is administered once every two weeks.

In some embodiments, the anti-PD-L1/CD47 bispecific antibody (such as antibody BsAb-71-N297A or BsAb-71) at a dose of about 1350 mg to 1450 mg, or a formulation containing the anti-PD-L1/CD47 bispecific antibody at this dose is administered once every two weeks. In some embodiments, the anti-PD-L1/CD47 bispecific antibody at a dose of about 1400 mg, or a formulation containing the anti-PD-L1/CD47 bispecific antibody at this dose is administered once every two weeks.

In some embodiments, the anti-PD-L1/CD47 bispecific antibody (such as antibody BsAb-71-N297A or BsAb-71) at a dose of about 2370 mg to 2490 mg, or a formulation containing the anti-PD-L1/CD47 bispecific antibody at this dose is administered once every two weeks. In some embodiments, the anti-PD-L1/CD47 bispecific antibody at a dose of about 2400 mg, or a formulation containing the anti-PD-L1/CD47 bispecific antibody at this dose is administered once every two weeks.

In some embodiments, the anti-PD-L1/CD47 bispecific antibody (such as antibody BsAb-71-N297A or BsAb-71) at a dose of about 2770 mg to 2890 mg, or a formulation containing the anti-PD-L1/CD47 bispecific antibody at this dose is administered once every two weeks. In some embodiments, the anti-PD-L1/CD47 bispecific antibody at a dose of about 2800 mg, or a formulation containing the anti-PD-L1/CD47 bispecific antibody at this dose is administered once every two weeks.

In some embodiments, the anti-PD-L1/CD47 bispecific antibody (such as antibody BsAb-71-N297A or BsAb-71) at a dose of about 2970 mg to 3050 mg, or a formulation containing the anti-PD-L1/CD47 bispecific antibody at this dose is administered once every two weeks. In some embodiments, the anti-PD-L1/CD47 bispecific antibody at a dose of about 3000 mg, or a formulation containing the anti-PD-L1/CD47 bispecific antibody at this dose is administered once every two weeks.

In some embodiments, the anti-PD-L1/CD47 bispecific antibody (such as antibody BsAb-71-N297A or BsAb-71) is administered at a dose of about 6 mg to 3000 mg once every two weeks after four weeks of the first administration. In some embodiments, the anti-PD-L1/CD47 bispecific antibody is administered at a dose of about 6 mg, about 7 mg, about 10 mg, about 14 mg, about 18 mg, about 21 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 435 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, about 600 mg, about 610 mg, about 620 mg, about 630 mg, about 640 mg, about 650 mg, about 660 mg, about 670 mg, about 680 mg, about 690 mg, about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, about 1450 mg, about 1500 mg, about 1550 mg, about 1600 mg, about 1650 mg, about 1700 mg, about 1750 mg, about 1800 mg, about 1850 mg, about 1900 mg, about 1950 mg, about 2000 mg, about 2050 mg, about 2100 mg, about 2150 mg, about 2200 mg, about 2250 mg, about 2300 mg, about 2350 mg, about 2400 mg, about 2450 mg, about 2500 mg, about 2550 mg, about 2600 mg, about 2650 mg, about 2700 mg, about 2750 mg, about 2800 mg, about 2850 mg, about 2900 mg, about 2950 mg, about 3000 mg, once every two weeks after four weeks of the first administration.

In some embodiments, the anti-PD-L1/CD47 bispecific antibody (such as antibody BsAb-71-N297A or BsAb-71) is administered at a dose of about 6 mg to 2800 mg once every two weeks after four weeks of the first administration. In some embodiments, the anti-PD-L1/CD47 bispecific antibody is administered at a dose of about 6 mg, about 7 mg, about 10 mg, about 14 mg, about 18 mg, about 21 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 435 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, about 600 mg, about 610 mg, about 620 mg, about 630 mg, about 640 mg, about 650 mg, about 660 mg, about 670 mg, about 680 mg, about 690 mg, about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, about 1450 mg, about 1500 mg, about 1550 mg, about 1600 mg, about 1650 mg, about 1700 mg, about 1750 mg, about 1800 mg, about 1850 mg, about 1900 mg, about 1950 mg, about 2000 mg, about 2050 mg, about 2100 mg, about 2150 mg, about 2200 mg, about 2250 mg, about 2300 mg, about 2350 mg, about 2400 mg, about 2450 mg, about 2500 mg, about 2550 mg, about 2600 mg, about 2650 mg, about 2700 mg, about 2750 mg, about 2800 mg, once every two weeks after four weeks of the first administration.

In some embodiments, the anti-PD-L1/CD47 bispecific antibody (such as antibody BsAb-71-N297A or BsAb-71) is administered at a dose of about 6 mg to 18 mg once every two weeks after four weeks of the first administration. In some embodiments, the anti-PD-L1/CD47 bispecific antibody is administered at a dose of about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, once every two weeks after four weeks of the first administration.

In some embodiments, the anti-PD-L1/CD47 bispecific antibody (such as antibody BsAb-71-N297A or BsAb-71) is administered at a dose of about 18 mg to 60 mg once every two weeks after four weeks of the first administration. In some embodiments, the anti-PD-L1/CD47 bispecific antibody is administered at a dose of about 18 mg, about 19 mg, about 20 mg, about 21 mg, about 22 mg, about 23 mg, about 25 mg, about 26 mg, about 27 mg, about 28 mg, about 29 mg, about 30 mg, about 31 mg, about 32 mg, about 33 mg, about 35 mg, 36 mg, about 37 mg, about 38 mg, about 39 mg, about 40 mg, about 41 mg, about 42 mg, about 43 mg, about 45 mg, about 46 mg, about 47 mg, about 48 mg, about 50 mg, about 53 mg, about 55 mg, about 58 mg, about 60 mg, once every two weeks after four weeks of the first administration.

In some embodiments, the anti-PD-L1/CD47 bispecific antibody (such as antibody BsAb-71-N297A or BsAb-71) is administered at a dose of about 60 mg to 180 mg once every two weeks after four weeks of the first administration. In some embodiments, the anti-PD-L1/CD47 bispecific antibody is administered at a dose of about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, once every two weeks after four weeks of the first administration.

In some embodiments, the anti-PD-L1/CD47 bispecific antibody (such as antibody BsAb-71-N297A or BsAb-71) is administered at a dose of about 180 mg to 600 mg once every two weeks after four weeks of the first administration. In some embodiments, the anti-PD-L1/CD47 bispecific antibody is administered at a dose of about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 320 mg, about 350 mg, about 400 mg, about 450 mg, about 480 mg, about 500 mg, about 520 mg, about 550 mg, about 580 mg, about 600 mg, once every two weeks after four weeks of the first administration.

In some embodiments, the anti-PD-L1/CD47 bispecific antibody (such as antibody BsAb-71-N297A or BsAb-71) is administered at a dose of about 600 mg to 1200 mg once every two weeks after four weeks of the first administration. In some embodiments, the anti-PD-L1/CD47 bispecific antibody is administered at a dose of about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 434 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 567 mg, about 570 mg, about 580 mg, about 590 mg, about 600 mg, about 610 mg, about 620 mg, about 630 mg, about 640 mg, about 650 mg, about 660 mg, about 670 mg, about 680 mg, about 690 mg, about 700 mg, about 750 mg, about 800 mg, about 850 mg, about 900 mg, about 950 mg, about 1000 mg, about 1050 mg, about 1100 mg, about 1150 mg, about 1200 mg, once every two weeks after four weeks of the first administration.

In some embodiments, the anti-PD-L1/CD47 bispecific antibody (such as antibody BsAb-71-N297A or BsAb-71) is administered at a dose of about 1200 mg to 2400 mg once every two weeks after four weeks of the first administration. In some embodiments, the anti-PD-L1/CD47 bispecific antibody is administered at a dose of about 1200 mg, about 1250 mg, about 1300 mg, about 1350 mg, about 1400 mg, about 1450 mg, about 1500 mg, about 1550 mg, about 1600 mg, about 1650 mg, about 1700 mg, about 1750 mg, about 1800 mg, about 1850 mg, about 1900 mg, about 1950 mg, about 2000 mg, about 2050 mg, about 2100 mg, about 2150 mg, about 2200 mg, about 2250 mg, about 2300 mg, about 2350 mg, about 2400 mg, once every two weeks after four weeks of the first administration.

In some embodiments, the anti-PD-L1/CD47 bispecific antibody (such as antibody BsAb-71-N297A or BsAb-71) is administered at a dose of about 7 mg to 2800 mg once every two weeks after four weeks of the first administration. In some embodiments, the anti-PD-L1/CD47 bispecific antibody is administered at a dose of about 7 mg, about 10 mg, about 14 mg, about 18 mg, about 21 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 435 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, about 600 mg, about 610 mg, about 620 mg, about 630 mg, about 640 mg, about 650 mg, about 660 mg, about 670 mg, about 680 mg, about 690 mg, about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, about 1450 mg, about 1500 mg, about 1550 mg, about 1600 mg, about 1650 mg, about 1700 mg, about 1750 mg, about 1800 mg, about 1850 mg, about 1900 mg, about 1950 mg, about 2000 mg, about 2050 mg, about 2100 mg, about 2150 mg, about 2200 mg, about 2250 mg, about 2300 mg, about 2350 mg, about 2400 mg, about 2450 mg, about 2500 mg, about 2550 mg, about 2600 mg, about 2650 mg, about 2700 mg, about 2750 mg, about 2800 mg, once every two weeks after four weeks of the first administration.

In some embodiments, the anti-PD-L1/CD47 bispecific antibody (such as antibody BsAb-71-N297A or BsAb-71) is administered at a dose of about 7 mg to 21 mg once every two weeks after four weeks of the first administration. In some embodiments, the anti-PD-L1/CD47 bispecific antibody is administered at a dose of about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, once every two weeks after four weeks of the first administration.

In some embodiments, the anti-PD-L1/CD47 bispecific antibody (such as antibody BsAb-71-N297A or BsAb-71) is administered at a dose of about 21 mg to 70 mg once every two weeks after four weeks of the first administration. In some embodiments, the anti-PD-L1/CD47 bispecific antibody is administered at a dose of about 21 mg, about 22 mg, about 23 mg, about 25 mg, about 26 mg, about 27 mg, about 28 mg, about 29 mg, about 30 mg, about 31 mg, about 32 mg, about 33 mg, about 35 mg, 36 mg, about 37 mg, about 38 mg, about 39 mg, about 40 mg, about 41 mg, about 42 mg, about 43 mg, about 45 mg, about 46 mg, about 47 mg, about 48 mg, about 50 mg, about 53 mg, about 55 mg, about 58 mg, about 60 mg, about 63 mg, about 65 mg, about 68 mg, about 70 mg, once every two weeks after four weeks of the first administration.

In some embodiments, the anti-PD-L1/CD47 bispecific antibody (such as antibody BsAb-71-N297A or BsAb-71) is administered at a dose of about 70 mg to 210 mg once every two weeks after four weeks of the first administration. In some embodiments, the anti-PD-L1/CD47 bispecific antibody is administered at a dose of about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, once every two weeks after four weeks of the first administration.

In some embodiments, the anti-PD-L1/CD47 bispecific antibody (such as antibody BsAb-71-N297A or BsAb-71) is administered at a dose of about 210 mg to 700 mg once every two weeks after four weeks of the first administration. In some embodiments, the anti-PD-L1/CD47 bispecific antibody is administered at a dose of about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 434 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 567 mg, about 570 mg, about 580 mg, about 590 mg, about 600 mg, about 610 mg, about 620 mg, about 630 mg, about 640 mg, about 650 mg, about 660 mg, about 670 mg, about 680 mg, about 690 mg, about 700 mg, once every two weeks after four weeks of the first administration.

In some embodiments, the anti-PD-L1/CD47 bispecific antibody (such as antibody BsAb-71-N297A or BsAb-71) is administered at a dose of about 700 mg to 1400 mg once every two weeks after four weeks of the first administration. In some embodiments, the anti-PD-L1/CD47 bispecific antibody is administered at a dose of about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, once every two weeks after four weeks of the first administration.

In some embodiments, the anti-PD-L1/CD47 bispecific antibody (such as antibody BsAb-71-N297A or BsAb-71) is administered at a dose of about 1400 mg to 2800 mg once every two weeks after four weeks of the first administration. In some embodiments, the anti-PD-L1/CD47 bispecific antibody is administered at a dose of about 1400 mg, about 1450 mg, about 1500 mg, about 1550 mg, about 1600 mg, about 1650 mg, about 1700 mg, about 1750 mg, about 1800 mg, about 1850 mg, about 1900 mg, about 1950 mg, about 2000 mg, about 2050 mg, about 2100 mg, about 2150 mg, about 2200 mg, about 2250 mg, about 2300 mg, about 2350 mg, about 2400 mg, about 2430 mg, about 2450 mg, about 2480 mg, about 2500 mg, about 2530 mg, about 2550 mg, about 2580 mg, about 2600 mg, about 2630 mg, about 2650 mg, about 2680 mg, about 2700 mg, about 2730 mg, about 2750 mg, about 2780 mg, about 2800 mg, once every two weeks after four weeks of the first administration.

In some embodiments, the anti-PD-L1/CD47 bispecific antibody (such as antibody BsAb-71-N297A or BsAb-71) at a dose of about 1 mg to 10 mg, or a formulation containing the anti-PD-L1/CD47 bispecific antibody at this dose is administered once every two weeks after four weeks of the first administration. In some embodiments, the anti-PD-L1/CD47 bispecific antibody at a dose of about 6 mg, or a formulation containing the anti-PD-L1/CD47 bispecific antibody at this dose is administered once every two weeks after four weeks of the first administration. In some embodiments, the anti-PD-L1/CD47 bispecific antibody at a dose of about 7 mg, or a formulation containing the anti-PD-L1/CD47 bispecific antibody at this dose is administered once every two weeks after four weeks of the first administration.

In some embodiments, the anti-PD-L1/CD47 bispecific antibody (such as antibody BsAb-71-N297A or BsAb-71) at a dose of about 15 mg to 25 mg, or a formulation containing the anti-PD-L1/CD47 bispecific antibody at this dose is administered once every two weeks after four weeks of the first administration. In some embodiments, the anti-PD-L1/CD47 bispecific antibody at a dose of about 18 mg, or a formulation containing the anti-PD-L1/CD47 bispecific antibody at this dose is administered once every two weeks after four weeks of the first administration. In some embodiments, the anti-PD-L1/CD47 bispecific antibody at a dose of about 21 mg, or a formulation containing the anti-PD-L1/CD47 bispecific antibody at this dose is administered once every two weeks after four weeks of the first administration.

In some embodiments, the anti-PD-L1/CD47 bispecific antibody (such as antibody BsAb-71-N297A or BsAb-71) at a dose of about 55 mg to 75 mg, or a formulation containing the anti-PD-L1/CD47 bispecific antibody at this dose is administered once every two weeks after four weeks of the first administration. In some embodiments, the anti-PD-L1/CD47 bispecific antibody at a dose of about 60 mg, or a formulation containing the anti-PD-L1/CD47 bispecific antibody at this dose is administered once every two weeks after four weeks of the first administration. In some embodiments, the anti-PD-L1/CD47 bispecific antibody at a dose of about 70 mg, or a formulation containing the anti-PD-L1/CD47 bispecific antibody at this dose is administered once every two weeks after four weeks of the first administration.

In some embodiments, the anti-PD-L1/CD47 bispecific antibody (such as antibody BsAb-71-N297A or BsAb-71) at a dose of about 150 mg to 230 mg, or a formulation containing the anti-PD-L1/CD47 bispecific antibody at this dose is administered once every two weeks after four weeks of the first administration. In some embodiments, the anti-PD-L1/CD47 bispecific antibody at a dose of about 180 mg, or a formulation containing the anti-PD-L1/CD47 bispecific antibody at this dose is administered once every two weeks after four weeks of the first administration. In some embodiments, the anti-PD-L1/CD47 bispecific antibody at a dose of about 210 mg, or a formulation containing the anti-PD-L1/CD47 bispecific antibody at this dose is administered once every two weeks after four weeks of the first administration.

In some embodiments, the anti-PD-L1/CD47 bispecific antibody (such as antibody BsAb-71-N297A or BsAb-71) at a dose of about 590 mg to 610 mg, or a formulation containing the anti-PD-L1/CD47 bispecific antibody at this dose is administered once every two weeks after four weeks of the first administration. In some embodiments, the anti-PD-L1/CD47 bispecific antibody at a dose of about 600 mg, or a formulation containing the anti-PD-L1/CD47 bispecific antibody at this dose is administered once every two weeks after four weeks of the first administration.

In some embodiments, the anti-PD-L1/CD47 bispecific antibody (such as antibody BsAb-71-N297A or BsAb-71) at a dose of about 690 mg to 710 mg, or a formulation containing the anti-PD-L1/CD47 bispecific antibody at this dose is administered once every two weeks after four weeks of the first administration. In some embodiments, the anti-PD-L1/CD47 bispecific antibody at a dose of about 700 mg, or a formulation containing the anti-PD-L1/CD47 bispecific antibody at this dose is administered once every two weeks after four weeks of the first administration.

In some embodiments, the anti-PD-L1/CD47 bispecific antibody (such as antibody BsAb-71-N297A or BsAb-71) at a dose of about 1150 mg to 1250 mg, or a formulation containing the anti-PD-L1/CD47 bispecific antibody at this dose is administered once every two weeks after four weeks of the first administration. In some embodiments, the anti-PD-L1/CD47 bispecific antibody at a dose of about 1200 mg, or a formulation containing the anti-PD-L1/CD47 bispecific antibody at this dose is administered once every two weeks after four weeks of the first administration.

In some embodiments, the anti-PD-L1/CD47 bispecific antibody (such as antibody BsAb-71-N297A or BsAb-71) at a dose of about 1350 mg to 1450 mg, or a formulation containing the anti-PD-L1/CD47 bispecific antibody at this dose is administered once every two weeks after four weeks of the first administration. In some embodiments, the anti-PD-L1/CD47 bispecific antibody at a dose of about 1400 mg, or a formulation containing the anti-PD-L1/CD47 bispecific antibody at this dose is administered once every two weeks after four weeks of the first administration.

In some embodiments, the anti-PD-L1/CD47 bispecific antibody (such as antibody BsAb-71-N297A or BsAb-71) at a dose of about 2370 mg to 2490 mg, or a formulation containing the anti-PD-L1/CD47 bispecific antibody at this dose is administered once every two weeks after four weeks of the first administration. In some embodiments, the anti-PD-L1/CD47 bispecific antibody at a dose of about 2400 mg, or a formulation containing the anti-PD-L1/CD47 bispecific antibody at this dose is administered once every two weeks after four weeks of the first administration.

In some embodiments, the anti-PD-L1/CD47 bispecific antibody (such as antibody BsAb-71-N297A or BsAb-71) at a dose of about 2770 mg to 2890 mg, or a formulation containing the anti-PD-L1/CD47 bispecific antibody at this dose is administered once every two weeks after four weeks of the first administration. In some embodiments, the anti-PD-L1/CD47 bispecific antibody at a dose of about 2800 mg, or a formulation containing the anti-PD-L1/CD47 bispecific antibody at this dose is administered once every two weeks after four weeks of the first administration.

In some embodiments, the anti-PD-L1/CD47 bispecific antibody (such as antibody BsAb-71-N297A or BsAb-71) at a dose of about 2970 mg to 3050 mg, or a formulation containing the anti-PD-L1/CD47 bispecific antibody at this dose is administered once every two weeks after four weeks of the first administration. In some embodiments, the anti-PD-L1/CD47 bispecific antibody at a dose of about 3000 mg, or a formulation containing the anti-PD-L1/CD47 bispecific antibody at this dose is administered once every two weeks after four weeks of the first administration.

In some embodiments, the anti-PD-L1/CD47 bispecific antibody or the antigen-binding fragment (or formulation) is administered to the patient once per treatment cycle. In some embodiments, the anti-PD-L1/CD47 bispecific antibody or the antigen-binding fragment (or formulation) is administered multiple times, e.g., 2, 3, 4, or 5 times, per treatment cycle. In some embodiments, the administration is performed on the patient only 1 or 4 times per treatment cycle.

In some embodiments, the patient is treated for one treatment cycle. In some embodiments, the patient is treated for multiple (e.g., 2, 3, 4, 5, 6, 7, 8, 9, 10, 11, 12, 13, 14, 15, 16, 17, 18, 19, 20, 21, 22, 23, 24, 25, or 26) treatment cycles. In some embodiments, one treatment cycle is about one week, about two weeks, about three weeks, about four weeks, about five weeks, about six weeks, about seven weeks, or a range between any two of these values (inclusive) or any value therein (e.g., about two weeks to four weeks). In some embodiments, the patient is treated until the disorder is alleviated and no treatment is required.

In some embodiments, the present invention discloses a method for treating an autoimmune disease, an acute or chronic inflammatory disease, an infectious disease, or a tumor, which comprises: administering to a patient in need thereof the anti-PD-L1/CD47 bispecific antibody or the antigen-binding fragment at a dose of about 6 mg to 18 mg, about 18 mg to 60 mg, about 60 mg to 180 mg, about 180 mg to 600 mg, about 600 mg to 1200 mg, about 1200 mg to 2400 mg, such as about 6 mg, about 10 mg, about 12 mg, about 18 mg, about 30 mg, about 50 mg, about 60 mg, about 80 mg, about 110 mg, about 130 mg, about 160 mg, about 180 mg, about 210 mg, about 230 mg, about 250 mg, about 300 mg, about 400 mg, about 500 mg, about 600 mg, about 700 mg, about 800 mg, about 900 mg, about 1000 mg, about 1100 mg, about 1200 mg, about 1300 mg, about 1500 mg, about 1600 mg, about 1700 mg, about 1800 mg, about 1900 mg, about 2000 mg, about 2100 mg, about 2200 mg, about 2300 mg, or about 2400 mg, or a formulation containing the anti-PD-L1/CD47 bispecific antibody or the antigen-binding fragment at this dose once every two weeks. In some embodiments, the anti-PD-L1/CD47 bispecific antibody is an antibody BsAb-71-N297A. In some embodiments, the anti-PD-L1/CD47 bispecific antibody is an antibody BsAb-71.

In some embodiments, the present invention discloses a method for treating an autoimmune disease, an acute or chronic inflammatory disease, an infectious disease, or a tumor, which comprises: administering to a patient in need thereof the anti-PD-L1/CD47 bispecific antibody or the antigen-binding fragment at a dose of about 7 mg to 21 mg, about 21 mg to 70 mg, about 70 mg to 210 mg, about 210 mg to 700 mg, about 700 mg to 1400 mg, about 1400 mg to 2800 mg, such as about 7 mg, about 10 mg, about 12 mg, about 18 mg, about 21 mg, about 30 mg, about 50 mg, about 60 mg, about 70 mg, about 80 mg, about 110 mg, about 130 mg, about 160 mg, about 180 mg, about 210 mg, about 230 mg, about 250 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, about 1800 mg, about 1900 mg, about 2000 mg, about 2100 mg, about 2200 mg, about 2300 mg, about 2400 mg, about 2500 mg, about 2600 mg, about 2700 mg, or about 2800 mg, or a formulation containing the anti-PD-L1/CD47 bispecific antibody or the antigen-binding fragment at this dose once every two weeks. In some embodiments, the anti-PD-L1/CD47 bispecific antibody is an antibody BsAb-71-N297A. In some embodiments, the anti-PD-L1/CD47 bispecific antibody is an antibody BsAb-71.

In some embodiments, the anti-PD-L1/CD47 bispecific antibody or the antigen-binding fragment is administered at a dose of about 6 mg once every two weeks. In some embodiments, the anti-PD-L1/CD47 bispecific antibody or the antigen-binding fragment is administered at a dose of about 7 mg once every two weeks. In some embodiments, the anti-PD-L1/CD47 bispecific antibody or the antigen-binding fragment is administered at a dose of about 18 mg once every two weeks. In some embodiments, the anti-PD-L1/CD47 bispecific antibody or the antigen-binding fragment is administered at a dose of about 21 mg once every two weeks. In some embodiments, the anti-PD-L1/CD47 bispecific antibody or the antigen-binding fragment is administered at a dose of about 60 mg once every two weeks. In some embodiments, the anti-PD-L1/CD47 bispecific antibody or the antigen-binding fragment is administered at a dose of about 70 mg once every two weeks. In some embodiments, the anti-PD-L1/CD47 bispecific antibody or the antigen-binding fragment is administered at a dose of about 180 mg once every two weeks. In some embodiments, the anti-PD-L1/CD47 bispecific antibody or the antigen-binding fragment is administered at a dose of about 210 mg once every two weeks. In some embodiments, the anti-PD-L1/CD47 bispecific antibody or the antigen-binding fragment is administered at a dose of about 600 mg once every two weeks. In some embodiments, the anti-PD-L1/CD47 bispecific antibody or the antigen-binding fragment is administered at a dose of about 700 mg once every two weeks. In some embodiments, the anti-PD-L1/CD47 bispecific antibody or the antigen-binding fragment is administered at a dose of about 1200 mg once every two weeks. In some embodiments, the anti-PD-L1/CD47 bispecific antibody or the antigen-binding fragment is administered at a dose of about 1400 mg once every two weeks. In some embodiments, the anti-PD-L1/CD47 bispecific antibody or the antigen-binding fragment is administered at a dose of about 2400 mg once every two weeks. In some embodiments, the anti-PD-L1/CD47 bispecific antibody or the antigen-binding fragment is administered at a dose of about 2800 mg once every two weeks.

In some embodiments, the anti-PD-L1/CD47 bispecific antibody or the antigen-binding fragment is administered at a dose of about 0.1 mg/kg once every two weeks. In some embodiments, the anti-PD-L1/CD47 bispecific antibody or the antigen-binding fragment is administered at a dose of about 0.3 mg/kg once every two weeks. In some embodiments, the anti-PD-L1/CD47 bispecific antibody or the antigen-binding fragment is administered at a dose of about 1 mg/kg once every two weeks. In some embodiments, the anti-PD-L1/CD47 bispecific antibody or the antigen-binding fragment is administered at a dose of about 3 mg/kg once every two weeks. In some embodiments, the anti-PD-L1/CD47 bispecific antibody or the antigen-binding fragment is administered at a dose of about 10 mg/kg once every two weeks. In some embodiments, the anti-PD-L1/CD47 bispecific antibody or the antigen-binding fragment is administered at a dose of about 20 mg/kg once every two weeks. In some embodiments, the anti-PD-L1/CD47 bispecific antibody or the antigen-binding fragment is administered at a dose of about 40 mg/kg once every two weeks.

In some embodiments, the present invention discloses a method for treating an autoimmune disease, an acute or chronic inflammatory disease, an infectious disease, or a tumor, which comprises: administering to a patient in need thereof the anti-PD-L1/CD47 bispecific antibody or the antigen-binding fragment at a dose of about 6 mg to 18 mg, about 18 mg to 60 mg, about 60 mg to 180 mg, about 180 mg to 600 mg, about 600 mg to 1200 mg, about 1200 mg to 2400 mg, such as about 6 mg, about 10 mg, about 12 mg, about 18 mg, about 30 mg, about 50 mg, about 60 mg, about 80 mg, about 110 mg, about 130 mg, about 160 mg, about 180 mg, about 210 mg, about 230 mg, about 250 mg, about 300 mg, about 400 mg, about 500 mg, about 600 mg, about 700 mg, about 800 mg, about 900 mg, about 1000 mg, about 1100 mg, about 1200 mg, about 1300 mg, about 1500 mg, about 1600 mg, about 1700 mg, about 1800 mg, about 1900 mg, about 2000 mg, about 2100 mg, about 2200 mg, about 2300 mg, or about 2400 mg, or a formulation containing the anti-PD-L1/CD47 bispecific antibody or the antigen-binding fragment at this dose once every two weeks after four weeks of the first administration. In some embodiments, the anti-PD-L1/CD47 bispecific antibody is an antibody BsAb-71-N297A. In some embodiments, the anti-PD-L1/CD47 bispecific antibody is an antibody BsAb-71.

In some embodiments, the present invention discloses a method for treating an autoimmune disease, an acute or chronic inflammatory disease, an infectious disease, or a tumor, which comprises: administering to a patient in need thereof the anti-PD-L1/CD47 bispecific antibody or the antigen-binding fragment at a dose of about 7 mg to 21 mg, about 21 mg to 70 mg, about 70 mg to 210 mg, about 210 mg to 700 mg, about 700 mg to 1400 mg, about 1400 mg to 2800 mg, such as about 7 mg, about 10 mg, about 12 mg, about 18 mg, about 21 mg, about 30 mg, about 50 mg, about 60 mg, about 70 mg, about 80 mg, about 110 mg, about 130 mg, about 160 mg, about 180 mg, about 210 mg, about 230 mg, about 250 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, about 1800 mg, about 1900 mg, about 2000 mg, about 2100 mg, about 2200 mg, about 2300 mg, about 2400 mg, about 2500 mg, about 2600 mg, about 2700 mg, or about 2800 mg, or a formulation containing the anti-PD-L1/CD47 bispecific antibody or the antigen-binding fragment at this dose once every two weeks after four weeks of the first administration. In some embodiments, the anti-PD-L1/CD47 bispecific antibody is an antibody BsAb-71-N297A. In some embodiments, the anti-PD-L1/CD47 bispecific antibody is an antibody BsAb-71.

In some embodiments, the anti-PD-L1/CD47 bispecific antibody or the antigen-binding fragment is administered at a dose of about 6 mg once every two weeks. In some embodiments, the anti-PD-L1/CD47 bispecific antibody or the antigen-binding fragment is administered at a dose of about 7 mg once every two weeks. In some embodiments, the anti-PD-L1/CD47 bispecific antibody or the antigen-binding fragment is administered at a dose of about 18 mg once every two weeks. In some embodiments, the anti-PD-L1/CD47 bispecific antibody or the antigen-binding fragment is administered at a dose of about 21 mg once every two weeks. In some embodiments, the anti-PD-L1/CD47 bispecific antibody or the antigen-binding fragment is administered at a dose of about 60 mg once every two weeks. In some embodiments, the anti-PD-L1/CD47 bispecific antibody or the antigen-binding fragment is administered at a dose of about 70 mg once every two weeks. In some embodiments, the anti-PD-L1/CD47 bispecific antibody or the antigen-binding fragment is administered at a dose of about 180 mg once every two weeks. In some embodiments, the anti-PD-L1/CD47 bispecific antibody or the antigen-binding fragment is administered at a dose of about 210 mg once every two weeks. In some embodiments, the anti-PD-L1/CD47 bispecific antibody or the antigen-binding fragment is administered at a dose of about 600 mg once every two weeks. In some embodiments, the anti-PD-L1/CD47 bispecific antibody or the antigen-binding fragment is administered at a dose of about 700 mg once every two weeks. In some embodiments, the anti-PD-L1/CD47 bispecific antibody or the antigen-binding fragment is administered at a dose of about 1200 mg once every two weeks. In some embodiments, the anti-PD-L1/CD47 bispecific antibody or the antigen-binding fragment is administered at a dose of about 1400 mg once every two weeks. In some embodiments, the anti-PD-L1/CD47 bispecific antibody or the antigen-binding fragment is administered at a dose of about 2400 mg once every two weeks. In some embodiments, the anti-PD-L1/CD47 bispecific antibody or the antigen-binding fragment is administered at a dose of about 2800 mg once every two weeks.

In some embodiments, the anti-PD-L1/CD47 bispecific antibody or the antigen-binding fragment is administered at a dose of about 6 mg once every two weeks after four weeks of the first administration. In some embodiments, the anti-PD-L1/CD47 bispecific antibody or the antigen-binding fragment is administered at a dose of about 7 mg once every two weeks after four weeks of the first administration. In some embodiments, the anti-PD-L1/CD47 bispecific antibody or the antigen-binding fragment is administered at a dose of about 18 mg once every two weeks after four weeks of the first administration. In some embodiments, the anti-PD-L1/CD47 bispecific antibody or the antigen-binding fragment is administered at a dose of about 21 mg once every two weeks after four weeks of the first administration. In some embodiments, the anti-PD-L1/CD47 bispecific antibody or the antigen-binding fragment is administered at a dose of about 60 mg once every two weeks after four weeks of the first administration. In some embodiments, the anti-PD-L1/CD47 bispecific antibody or the antigen-binding fragment is administered at a dose of about 70 mg once every two weeks after four weeks of the first administration. In some embodiments, the anti-PD-L1/CD47 bispecific antibody or the antigen-binding fragment is administered at a dose of about 180 mg once every two weeks after four weeks of the first administration. In some embodiments, the anti-PD-L1/CD47 bispecific antibody or the antigen-binding fragment is administered at a dose of about 210 mg once every two weeks after four weeks of the first administration. In some embodiments, the anti-PD-L1/CD47 bispecific antibody or the antigen-binding fragment is administered at a dose of about 600 mg once every two weeks after four weeks of the first administration. In some embodiments, the anti-PD-L1/CD47 bispecific antibody or the antigen-binding fragment is administered at a dose of about 700 mg once every two weeks after four weeks of the first administration. In some embodiments, the anti-PD-L1/CD47 bispecific antibody or the antigen-binding fragment is administered at a dose of about 1200 mg once every two weeks after four weeks of the first administration. In some embodiments, the anti-PD-L1/CD47 bispecific antibody or the antigen-binding fragment is administered at a dose of about 1400 mg once every two weeks after four weeks of the first administration. In some embodiments, the anti-PD-L1/CD47 bispecific antibody or the antigen-binding fragment is administered at a dose of about 2400 mg once every two weeks after four weeks of the first administration. In some embodiments, the anti-PD-L1/CD47 bispecific antibody or the antigen-binding fragment is administered at a dose of about 2800 mg once every two weeks after four weeks of the first administration.

In some embodiments, the anti-PD-L1/CD47 bispecific antibody or the antigen-binding fragment is administered at a dose of about 0.1 mg/kg once every two weeks. In some embodiments, the anti-PD-L1/CD47 bispecific antibody or the antigen-binding fragment is administered at a dose of about 0.3 mg/kg once every two weeks. In some embodiments, the anti-PD-L1/CD47 bispecific antibody or the antigen-binding fragment is administered at a dose of about 1 mg/kg once every two weeks. In some embodiments, the anti-PD-L1/CD47 bispecific antibody or the antigen-binding fragment is administered at a dose of about 3 mg/kg once every two weeks. In some embodiments, the anti-PD-L1/CD47 bispecific antibody or the antigen-binding fragment is administered at a dose of about 10 mg/kg once every two weeks. In some embodiments, the anti-PD-L1/CD47 bispecific antibody or the antigen-binding fragment is administered at a dose of about 20 mg/kg once every two weeks. In some embodiments, the anti-PD-L1/CD47 bispecific antibody or the antigen-binding fragment is administered at a dose of about 40 mg/kg once every two weeks.

In some embodiments, the anti-PD-L1/CD47 bispecific antibody or the antigen-binding fragment is administered at a dose of about 0.1 mg/kg once every two weeks after four weeks of the first administration. In some embodiments, the anti-PD-L1/CD47 bispecific antibody or the antigen-binding fragment is administered at a dose of about 0.3 mg/kg once every two weeks after four weeks of the first administration. In some embodiments, the anti-PD-L1/CD47 bispecific antibody or the antigen-binding fragment is administered at a dose of about 1 mg/kg once every two weeks after four weeks of the first administration. In some embodiments, the anti-PD-L1/CD47 bispecific antibody or the antigen-binding fragment is administered at a dose of about 3 mg/kg once every two weeks after four weeks of the first administration. In some embodiments, the anti-PD-L1/CD47 bispecific antibody or the antigen-binding fragment is administered at a dose of about 10 mg/kg once every two weeks after four weeks of the first administration. In some embodiments, the anti-PD-L1/CD47 bispecific antibody or the antigen-binding fragment is administered at a dose of about 20 mg/kg once every two weeks after four weeks of the first administration. In some embodiments, the anti-PD-L1/CD47 bispecific antibody or the antigen-binding fragment is administered at a dose of about 40 mg/kg once every two weeks after four weeks of the first administration.

In some embodiments, the anti-PD-L1/CD47 bispecific antibody is an antibody BsAb-71-N297A. In some embodiments, the anti-PD-L1/CD47 bispecific antibody is an antibody BsAb-71.

In some embodiments, the patient is relieved of symptoms after a single dose is administered. In some embodiments, the patient is not relieved of symptoms as expected after a single dose is administered, and then the anti-PD-L1/CD47 bispecific antibody or the antigen-binding fragment at a dose of about 6 mg to 3000 mg is administered to the patient until the patient is relieved of the symptoms.

In some embodiments, the anti-PD-L1/CD47 bispecific antibody or the antigen-binding fragment is administered by at least one route selected from: parenteral, subcutaneous, intramuscular, intravenous, intraarticular, intrabronchial, intraperitoneal, intracapsular, intracartilaginous, intracavitary, intracoelomic, intracelebellar, intracerebroventricular, intracolic, intracervical, intragastric, intrahepatic, intramyocardial, intraosteal, intrapelvic, intrapericardial, intraperitoneal, intrapleural, intraprostatic, intrapulmonary, intrarectal, intrarenal, intraretinal, intraspinal, intrasynovial, intrathoracic, intrauterine, intravesical, bolus, vaginal, rectal, buccal, sublingual, intranasal, and transdermal administrations.

In some embodiments, the anti-PD-L1/CD47 bispecific antibody or the antigen-binding fragment (or formulation) is administered by subcutaneous (s.c.) injection, intraperitoneal (i.p.) injection, parenteral injection, intra-arterial injection, intravenous (i.v.) injection, or the like. In some embodiments, the anti-PD-L1/CD47 bispecific antibody or the antigen-binding fragment (or formulation) is administered by infusion. In some embodiments, the anti-PD-L1/CD47 bispecific antibody or the antigen-binding fragment (or formulation) is administered by bolus injection.

In some embodiments, the anti-PD-L1/CD47 bispecific antibody or the antigen-binding fragment (or formulation) is administered by intravenous (i.v.) infusion (i.e., i.v. infusion). In some embodiments, the intravenous infusion is performed for about 50 min, about 55 min, about 60 min, about 65 min, about 70 min, about 75 min, about 81 min, about 87 min, about 90 min, about 95 min, or a range between any two of these values (inclusive) or any value therein. In some embodiments, the intravenous infusion is performed for ≥ 60 min.

In some embodiments, the anti-PD-L1/CD47 bispecific antibody or the antigen-binding fragment (or formulation) is used in combination with other treatment methods for treating a tumor (including a benign tumor or cancer), e.g., chemotherapy, radiation therapy, immunotherapy, hormonal therapy, targeted therapy, biological therapy, surgical therapy, and the like. In some embodiments, the anti-PD-L1/CD47 bispecific antibody or the antigen-binding fragment (or formulation) is used in combination with other tumor or cancer therapeutic agents for treating a tumor (including a benign tumor or cancer), such as hormones and antibodies treating a tumor (including a benign tumor or cancer).

In some embodiments, the present invention discloses use of the anti-PD-L1/CD47 bispecific antibody or the antigen-binding fragment in the treatment of an autoimmune disease, an inflammatory disease, an infectious disease, or a tumor (including a benign tumor or cancer). In some embodiments, the anti-PD-L1/CD47 bispecific antibody or the antigen-binding fragment is administered in an amount of 0.1 mg/kg to 100 mg/kg once every two days to once every six weeks. In some embodiments, the administration is performed once every two days, once every three days, once every four days, once every five days, twice a week, once a week, once every two weeks, once every three weeks, once a month, or once every six weeks. In some embodiments, the administration is performed once every two weeks. In some embodiments, the administration is performed once every two weeks after four weeks of the first administration. In some embodiments, the anti-PD-L1/CD47 bispecific antibody is an antibody BsAb-71-N297A. In some embodiments, the anti-PD-L1/CD47 bispecific antibody is an antibody BsAb-71.

In some embodiments, the present invention discloses use of the anti-PD-L1/CD47 bispecific antibody or the antigen-binding fragment in the preparation of a medicament for treating an autoimmune disease, an inflammatory disease, an infectious disease, or a tumor (including a benign tumor or cancer). In some embodiments, the medicament for treating an autoimmune disease, an inflammatory disease, an infectious disease, or a tumor (including a benign tumor or cancer) includes the anti-PD-L1/CD47 bispecific antibody or the antigen-binding fragment. In some embodiments, the anti-PD-L1/CD47 bispecific antibody or the antigen-binding fragment is administered in an amount of 0.1 mg/kg to 100 mg/kg once every two days to once every six weeks. In some embodiments, the administration is performed once every two days, once every three days, once every four days, once every five days, twice a week, once a week, once every two weeks, once every three weeks, once a month, or once every six weeks. In some embodiments, the administration is performed once every two weeks after four weeks of the first administration. In some embodiments, the anti-PD-L1/CD47 bispecific antibody is an antibody BsAb-71-N297A. In some embodiments, the anti-PD-L1/CD47 bispecific antibody is an antibody BsAb-71.

In another aspect, the present invention also discloses a container comprising the anti-PD-L1/CD47 bispecific antibody or the antigen-binding fragment (or formulation). In some embodiments, the container is a syringe, an infusion bag, or a vial. In some embodiments, the container is an ampoule, a pre-filled disposable syringe, a multi-dose vial made of glass or plastic, or an infusion bag. In some embodiments, the anti-PD-L1/CD47 bispecific antibody is an antibody BsAb-71-N297A. In some embodiments, the anti-PD-L1/CD47 bispecific antibody is an antibody BsAb-71.

In another aspect, the present invention also discloses a kit comprising the anti-PD-L1/CD47 bispecific antibody or the antigen-binding fragment (or formulation) and instructions for directing administration of the anti-PD-L1/CD47 bispecific antibody or the antigen-binding fragment (or formulation) to a patient in need thereof. In some embodiments, the instructions are instructions for directing administration of the anti-PD-L1/CD47 bispecific antibody or the antigen-binding fragment (or formulation) at 0.1 mg/kg to 100 mg/kg to a patient in need thereof once every two days to once every six weeks. In some embodiments, the anti-PD-L1/CD47 bispecific antibody is an antibody BsAb-71-N297A. In some embodiments, the anti-PD-L1/CD47 bispecific antibody is an antibody BsAb-71.

In some embodiments, the present invention discloses use of the anti-PD-L1/CD47 bispecific antibody for the method described above.

In some embodiments, the present invention discloses use of the anti-PD-L1/CD47 bispecific antibody in the preparation of a medicament for the method described above.

In another aspect, the present invention also discloses a pharmaceutical composition, which comprises the anti-PD-L1/CD47 bispecific antibody or the antigen-binding fragment and is suitable for injection, e.g., a bolus injection or infusion (drip) pharmaceutical composition. The pharmaceutical composition suitable for injection includes a sterile aqueous solution (herein water-soluble solution) or dispersion and sterile powders for the instant preparation of a sterile injectable solution or dispersion. For intravenous administration, a suitable carrier includes normal saline, bacteriostatic water or phosphate-buffered saline (PBS), ethanol, a solvent or dispersion medium for polyol (e.g., glycerol, propylene glycol and liquid polyethylene glycol), and a suitable mixture thereof. In some embodiments, the pharmaceutical composition further comprises a pharmaceutically acceptable carrier. In some embodiments, the pharmaceutically acceptable carrier may include an antibacterial and/or antifungal agent, e.g., p-hydroxylbenzoate, chlorobutanol, phenol, ascorbic acid and thimerosal. In some embodiments, the pharmaceutically acceptable carrier may include an isotonic agent, such as a sugar, a polyol (such as mannitol and sorbitol), and sodium chloride. In some embodiments, the pharmaceutical composition comprises at least 0.1% of the anti-PD-L1/CD47 bispecific antibody or the antigen-binding fragment. The content of the antibody may vary, and the pharmaceutical composition comprises at least about 2% to 90% of the anti-PD-L1/CD47 bispecific antibody or the antigen-binding fragment. The amount of the anti-PD-L1/CD47 bispecific antibody or the antigen-binding fragment in such a therapeutically useful pharmaceutical composition may be an effective amount for administration. In some embodiments, the anti-PD-L1/CD47 bispecific antibody is an antibody BsAb-71-N297A. In some embodiments, the anti-PD-L1/CD47 bispecific antibody is an antibody BsAb-71.

In another aspect, the present invention also discloses a method for preparing the pharmaceutical composition described above, which comprises: mixing the anti-PD-L1/CD47 bispecific antibody or the antigen-binding fragment described herein with a pharmaceutically acceptable carrier suitable for injection (e.g., water for injection, normal saline, PBS, etc.). The method for mixing the anti-PD-L1/CD47 bispecific antibody described above with the pharmaceutically acceptable carrier is generally known in the art. The anti-PD-L1/CD47 bispecific antibody or the antigen-binding fragment (or formulation) of the present invention can be used for treating an autoimmune disease, an inflammatory disease, an infectious disease, or a tumor (including a benign tumor or cancer).

## BRIEF DESCRIPTION OF THE DRAWINGS

FIG. 1 shows the effect of the antibody BsAb-71, the positive control antibody Hu5F9-G4, and the negative control antibody IgG-Isotype on the agglutination of red blood cells.
FIG. 2 shows the inhibition of tumor cell proliferation by the antibody BsAb-71-N297A; wherein the abscissa represents the days of treatment and the ordinate represents the tumor volume.
FIG. 3 shows the inhibition of tumor cell proliferation by the antibody BsAb-71; wherein the abscissa represents the days of treatment and the ordinate represents the tumor volume.

## DETAILED DESCRIPTION

Unless otherwise stated, each of the following terms shall have the meaning described below. Unless otherwise defined, scientific and technical terms used in the present invention have the meanings that are commonly understood by those skilled in the art. Unless otherwise stated herein, amino acid residues in the constant regions are numbered according to the EU numbering scheme described in, for example, Kabat et al., Sequences of Proteins of Immunological Interes, 5th Edition, Public Health Service, National Institutes of Health, Bethesda, MD, 1991.

*Definitions*

It should be noted that the term "an" entity refers to one or more of the entities. For example, "an antibody" should be interpreted as one or more antibodies. As such, the terms "a" (or "an"), "one or more" and "at least one" can be used interchangeably herein.

"About" or "approximately" refers to a general error range for corresponding values as readily understood by those skilled in the relevant art. In some embodiments, "about" or "approximately" mentioned herein refers to the numerical value described as well as its ranges of $\pm 10\%$, $\pm 5\%$, $\pm 1\%$ or $\pm 0.1\%$.

As used herein, the term "contain" or "comprise" means that the compositions, methods and the like comprise the recited elements, such as components or steps and do not exclude the others.

The term "polypeptide" is intended to encompass both the singular form "polypeptide" and the plural form "polypeptides", and refers to a molecule consisting of amino acid monomers linearly linked by amide bonds (also known as peptide bonds). The term "polypeptide" refers to any single chain or multiple chains of two or more amino acids and does not refer to a specific length of the product. Thus, included within the definition of "polypeptide" are peptides, dipeptides, tripeptides, oligopeptides, "proteins", "amino acid chains", or any other term used to refer to chains of two or more amino acids, and the term "polypeptide" may be used in place of, or interchangeably with, any of the above terms. The term "polypeptide" is also intended to refer to a product of post-expression modification of a polypeptide, including but not limited to glycosylation, acetylation, phosphorylation, amidation, derivatization by known protecting/blocking groups, proteolytic cleavage or non-naturally occurring amino acid modification. The polypeptide may be derived from a natural biological source or produced by recombinant techniques, but it is not necessarily translated from a specified nucleic acid sequence. It may be produced in any manner, including chemical synthesis.

As used herein, the twenty conventional amino acids and their abbreviations follow conventional usage. See Immunology-A Synthesis (second edition, Eds. E. S. Golub and D. R. Gren, Sinauer Associates, Sunderland 7 Mass. (1991)). Stereoisomers (e.g., D-amino acids) of the twenty conventional amino acids, unnatural amino acids (such as $\alpha$- or $\alpha$-disubstituted amino acids), N-alkyl amino acids, lactic acid, and other unconventional amino acids can also be suitable components for the polypeptides of the present disclosure. Examples of unconventional amino acids include: 4-hydroxyproline, $\gamma$-carboxyglutamate, $\varepsilon$-N,N,N-trimethyllysine, $\varepsilon$-N-acetyllysine, O-phosphoserine, N-acetylserine, N-formylmethionine, 3-methylhistidine, 5-hydroxylysine, $\sigma$-N-methylarginine and other similar amino acids and imino acids (e.g., 4-hydroxyproline). In the polypeptide notation used herein, the left-hand direction is the amino-terminal direction and the right-hand direction is the carboxy-terminal direction, in accordance with standard usage and convention. The conventional (natural) amino acids include alanine (three-letter code: Ala, one-letter code: A), arginine (Arg, R), asparagine (Asn, N), aspartic acid (Asp, D), cysteine (Cys, C), glutamine (Gln, Q), glutamic acid (Glu, E), glycine (Gly, G), histidine (His, H), isoleucine (Ile, I ), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y) and valine (Val, V).

Minor variations in the amino acid sequences of antibodies or immunoglobulin molecules are contemplated by the present disclosure, provided that the identity of the amino acid sequences is maintained to be at least 75%, as an example, at least 80%, 90%, or 95%, and as another example, 99%. In some embodiments, the variations are conservative amino acid substitutions. Conservative amino acid substitutions are those that occur within a family of amino acids that are related in their side chains. Genetically encoded amino acids are generally classified into the following categories: (1) acidic amino acids: aspartate, and glutamate; (2) basic amino acids: lysine, arginine, and histidine; (3) non-polar amino acids: alanine, valine, leucine, isoleucine, proline, phenylalanine, methionine, and tryptophan; and (4) uncharged polar amino acids: glycine, asparagine, glutamine, cysteine, serine, threonine, and tyrosine. Amino acids of the other families include (i) serine and threonine of the aliphatic-hydroxy family; (ii) asparagine and glutamine of the amide-containing family; (iii) alanine, valine, leucine, and isoleucine of the aliphatic family; and (iv) phenylalanine, tryptophan, and tyrosine of the aromatic family. In some embodiments, conservative amino acid substitution groups are valine-leucine-isoleucine, phenylalanine-tyrosine, lysine-arginine, alanine-valine, glutamic acid-aspartic acid, and asparagine-glutamine. For example, it is reasonable to expect that the single replacement of leucine with isoleucine or valine, aspartate with glutamate, threonine with serine, or the similar replacement of an amino acid with a structurally related amino acid, will not have a major effect on the binding or properties of the resulting molecule, particularly when the replacement does not involve an amino acid within a binding site. Whether an amino acid change results in a functional peptide can be readily determined by measuring the specific activity of the polypeptide derivative. The measurement is described in detail herein. Fragments or analogs of antibodies or immunoglobulin molecules can be readily prepared by those of ordinary skill in the art.

In some embodiments, the amino acid substitutions have the following effects: (1) reducing susceptibility to proteolysis, (2) reducing susceptibility to oxidation, (3) altering the binding affinity for formation of protein complexes, (4) altering binding affinity, and (5) conferring or improving other physicochemical or functional properties of such analogs. Analogs can include various mutant proteins with sequences different from those of the naturally occurring peptide sequences. For example, single or multiple amino acid substitutions (preferably conservative amino acid substitutions) may be made in the naturally occurring sequence (preferably in a portion of the polypeptide outside the domains that form intermolecular contacts). A conservative amino acid substitution should not significantly alter the structural characteristics of the parent sequence (e.g., an amino acid for the replacement should not tend to disrupt a helix that occurs in the parent sequence, or disrupt other types of secondary structures that characterize the parent sequence). Examples of secondary and tertiary structures of artificially recognized polypeptides are described in Proteins: Structures and Molecular Principles (Ed. Creighton, W. H. Freeman and Company, New York (1984)); Introduction to Protein Structure (Eds. C. Branden and J. Tooze, Garland Publishing, New York, N.Y(1991)); and Thornton et al., Nature 354:105(1991).

The number of amino acids of "conservative amino acid substitutions of VL or VH" may be about 1, about 2, about 3, about 4, about 5, about 6, about 8, about 9, about 10, about 11, about 13, about 14, about 15, or a range between any two of these values (inclusive) or any value therein. The number of amino acids of "conservative amino acid substitutions of a heavy chain or a light chain" may be about 1, about 2, about 3, about 4, about 5, about 6, about 8, about 9, about 10, about 11, about 13, about 14, about 15, about 18, about 19, about 22, about 24, about 25, about 29, about 31, about 35, about 38, about 41, about 45, or a range between any two of these values (inclusive) or any value therein.

The term "isolated" as used herein with respect to cells, nucleic acids, polypeptides, antibodies and the like, e.g., "isolated" DNA, RNA, polypeptides, or antibodies, refers to molecules that are separated from one or more other components, e.g., DNA or RNA, in the natural environment of the cell. The term "isolated" as used herein also refers to nucleic acids or peptides that are substantially free of cellular materials, viral materials or cell media when produced by recombinant DNA techniques, or free of chemical precursors or other chemicals when chemically synthesized. The term "isolated" is also used herein to refer to cells or polypeptides that are separated from other cellular proteins or tissues. Isolated polypeptides are intended to include both purified and recombinant polypeptides. Isolated nucleic acids, polypeptides, antibodies and the like are usually prepared by at least one purification step. In some embodiments, the purity of the isolated nucleic acids, polypeptides, antibodies, etc. is at least about 50%, about 60%, about 70%, about 80%, about 90%, about 95%, about 99%, or a range between any two of these values (inclusive) or any value therein.

The term "encoding" as applied to a polynucleotide refers to a polynucleotide known to "encode" a polypeptide. When in its native state or manipulated by methods well known to those skilled in the art, the polynucleotide can be transcribed and/or translated to produce the polypeptide and/or a fragment thereof.

The term "recombinant", with regard to a polypeptide or polynucleotide, is intended to refer to a polypeptide or polynucleotide that does not occur in nature, and non-limiting examples can be combined to produce a polynucleotide or polypeptide that does not normally occur.

"Homology", "identity" or "similarity" refers to sequence similarity between two peptides or between two nucleic acid molecules. Homology or identity can be determined by comparing the positions that can be aligned in the sequences. When a position of the compared sequences is occupied by the same base or amino acid, then the molecules are homologous at that position. The degree of homology between the sequences is a function of the number of matching or homologous positions shared by the sequences.

"At least 80% identity" refers to about 80% identity, about 81% identity, about 82% identity, about 83% identity, about 85% identity, about 86% identity, about 87% identity, about 88% identity, about 90% identity, about 91% identity, about 92% identity, about 94% identity, about 95% identity, about 98% identity, about 99% identity, or a range between any two of these values (inclusive) or any value therein.

"At least 90% identity" refers to about 90% identity, about 91% identity, about 92% identity, about 93% identity, about 95% identity, about 96% identity, about 97% identity, about 98% identity, about 99% identity, or a range between any two of these values (inclusive) or any value therein.

A nucleic acid or a polynucleotide sequence (or a polypeptide or an antibody sequence) having a certain percentage (e.g., 90%, 95%, 98% or 99%) of "identity" or "sequence identity" to another sequence means that when the sequences are aligned, the percentage of bases (or amino acids) in the sequences are the same. This percentage of alignment identity or sequence identity can be determined using visual inspection or software programs known in the art, such as the software programs described in Ausubel et al. eds., (2007), Current Protocols in Molecular Biology. Preferably, the alignment is performed using default parameters. One alignment program is BLAST using default parameters, such as BLASTN and BLASTP, both using the following default parameters: Genetic code=standard; filter=none; strand=both; cutoff=60; expect=10; Matrix=BLOSUM62; Descriptions=50 sequences; sort by=HIGHSCORE; Databases=non-redundant; GenBank+EMBL+DDBJ+PDB+GenBank CDS translations+Swiss Protein+SPupdate+PIR. Biologically equivalent polynucleotides are polynucleotides having the above-specified percentage identity and encoding polypeptides having identical or similar biological activity. A polynucleotide consists of a specific sequence of four nucleotide bases: adenine (A), cytosine (C), guanine (G) and thymine (T)/uracil (U, instead of thymine when the polynucleotide is RNA). A "polynucleotide sequence" can be a letter representation of a polynucleotide molecule. The letter representation can be input into a database in a computer with a central processing unit and used for bioinformatics applications, such as functional genomics and homology searches.

The terms "polynucleotide", "nucleic acid", "polynucleotide" and "oligonucleotide" are used interchangeably to refer to a polymeric form of nucleotides of any length, whether deoxyribonucleotides or ribonucleotides or analogs thereof. The polynucleotide may have any three-dimensional structure and may perform any function, known or unknown. The following are non-limiting examples of polynucleotides: genes or gene fragments (such as probes, primers, EST or SAGE tags), exons, introns, messenger RNA (mRNA), transfer RNA, ribosomal RNA, ribozymes, cDNA, dsRNA, siRNA, miRNA, recombinant polynucleotides, branched polynucleotides, plasmids, vectors, isolated DNA of any sequence, isolated RNA, nucleic acid probes and primers of any sequence, Polynucleotides can include modified nucleotides, such as methylated nucleotides and nucleotide analogs. If present, structural modifications to the nucleotide can be made before or after the assembly of the polynucleotide. The sequence of nucleotides can be interrupted by non-nucleotide components. The polynucleotide may be further modified after polymerization, such as by conjugation with a labeling component. This term also refers to double-stranded and single-stranded molecules. Unless otherwise stated or required, examples of any polynucleotide disclosed herein include a double-stranded form and each of the two complementary single-stranded forms known or predicted to constitute the double-stranded form.

"Antibody" or "antigen-binding fragment" refers to a polypeptide or polypeptide complex that specifically recognizes and binds to an antigen. The antibody may be an intact antibody and any antigen-binding fragment or single chain thereof. The term "antibody" thus includes any protein or peptide comprising, in its molecule, at least a portion of an immunoglobulin molecule that has biological activity for binding to an antigen. The antibody and the antigen-binding fragment include, but are not limited to, complementarity determining regions (CDRs) of a heavy or light chain or a ligand binding portion thereof, heavy chain variable regions (VHs), light chain variable regions (VLs), heavy chain constant regions (CHs), light chain constant regions (CLs), framework regions (FRs) or any portion thereof, or at least a portion of a binding protein. The CDRs include light chain CDRs (VL CDR1-3) and heavy chain CDRs (VH CDR1-3). The antibody or the antigen-binding fragment described herein is bispecific antibody comprising an antibody fragment specifically binding to antigen a and antigen b. In some embodiments, a first polypeptide chain comprises the structure VHa-CHa, a second polypeptide chain comprises the structure VLa-CLa, a third polypeptide chain comprises the structure VHb-CHb, and a fourth polypeptide chain comprises the structure VLb-CLb. In some embodiments, the second polypeptide chain is identical to the fourth polypeptide chain in amino acid sequence.

The term "antibody fragment" or "antigen-binding fragment" refers to a part of an antibody, and the composition of the antibody fragment of the present invention may be similar to $F(ab')_2$, $F(ab)_2$, Fab', Fab, Fv, scFv and the like in monospecific antibody fragments. Regardless of its structure, the antibody fragment binds to the same antigen recognized by an intact antibody. The term "antibody fragment" includes aptamers, mirror image isomers and bivalent antibodies. The term "antigen-binding fragment" also includes any synthetic or genetically engineered protein that functions as an antibody by binding to a specific antigen to form a complex.

"Single chain variable fragment" or "scFv" refers to a fusion protein of a heavy chain variable region (VH) and a light chain variable region (VL) of an immunoglobulin. In some aspects, these regions are linked to a short linker peptide having 10 to about 25 amino acids. The linker may be enriched with glycine to improve flexibility, and enriched with serine or threonine to improve solubility, and may link the N terminus of VH and the C terminus of VL, or vice versa. Although the protein has the constant region removed and the linker introduced, it retains the specificity of the original immunoglobulin. ScFv molecules are generally known in the art and are described, for example, in U.S. patent No. 5,892,019.

The term "antibody" includes a wide variety of polypeptides that can be biochemically distinguished. Those skilled in the art will appreciate that the classes of heavy chains include gamma, mu, alpha, delta, or epsilon ($\gamma$, $\mu$, $\alpha$, $\delta$, or $\epsilon$) and some subclasses (e.g., $\gamma1$-$\gamma4$). The nature of this chain determines the "type" of the antibody as IgG, IgM, IgA, IgG or IgE. Immunoglobulin subclasses (isotypes), such as IgG1, IgG2, IgG3, IgG4, IgG5, etc., have been well characterized and the functional specificity imparted is also known. All types of immunoglobulins are within the scope of the present invention. In some embodiments, the immunoglobulin molecule is an IgG species. These four chains are connected in a "Y" configuration through disulfide bonds, wherein the light chain starts at the opening of "Y" configuration and extends through the variable region to surround the heavy chain.

The antibodies, antigen-binding fragments or derivatives disclosed herein include, but are not limited to, polyclonal antibodies, monoclonal antibodies, multispecific antibodies, fully human antibodies, humanized antibodies, primatized antibodies, chimeric antibodies, single-chain antibodies, epitope-binding fragments (e.g., Fab, Fab' and $F(ab')_2$), and single chain Fv (scFv).

Light chains can be classified into kappa ($\kappa$) or lambda ($\lambda$). Each heavy chain may bind to a x or $\lambda$ light chain. In general, when an immunoglobulin is produced by a hybridoma, a B cell or a genetically engineered host cell, the light and heavy chains are bound by covalent bonds, and the "tail" portions of the two heavy chains are bound by covalent disulfide bonds or non-covalent bonds. In the heavy chains, the amino acid sequence extends from the N terminus at the forked end of the Y configuration to the C terminus at the bottom of each chain. The immunoglobulin x light chain variable region is $V\kappa$; and the immunoglobulin $\lambda$ light chain variable region is $V_\lambda$.

The terms "constant" and "variable" are used in accordance with function. The light chain variable region (VL) and heavy chain variable region (VH) determine the antigen recognition and specificity. The light chain constant region (CL) and the heavy chain constant region (CH) impart important biological properties such as secretion, transplacental movement, Fc receptor binding, and complement fixation. By convention, the numbering of amino acids in the constant regions increases as they become further away from the antigen-binding site of the antibody or amino terminus. The N-terminal portion is the variable region, and the C-terminal portion is the constant region; such as the CH3 and CL domains of IgG1 actually comprise the carboxyl termini of the heavy chain and light chain, respectively.

In naturally occurring antibodies, the six "complementarity determining regions" or "CDRs" present in each antigen-binding domain are short, non-contiguous, antigen-specific binding amino acid sequences that form the antigen-binding domain, assuming that the antibody is present in its three-dimensional configuration in an aqueous environment. The remaining amino acids in the antigen-binding domain, referred to as the "framework" region, exhibit little intermolecular variability. Most of the framework regions adopt a $\beta$-sheet conformation, with the CDRs forming a loop structure connected to, or in some cases forming part of, the $\beta$-sheet structure. Thus, the framework regions position the CDRs in a correct orientation by interchain non-covalent interactions through forming a scaffold. The antigen-binding domain with the specifically positioned CDRs forms a surface complementary to an epitope on the antigen that facilitates non-covalent binding of the antibody to its antigenic epitope. For a given heavy or light chain variable region, amino acids comprising the CDRs and the framework regions may be identified by one of ordinary skills in the art according to known methods (see, Kabat, E., et al., U.S. Department of Health and Human Services, Sequences of Proteins of Immunological Interest, (1983) and Chothia and Lesk, J. Mol. Biol., 196:901-917 (1987)).

Based on different assignment systems, the boundaries of the CDRs of the variable regions of the same antibody may differ. Thus, when it comes to defining an antibody with a particular CDR sequence defined in the present invention, the scope of the antibody also encompasses antibodies whose variable region sequences comprise the CDR sequence of the present invention but whose claimed CDR boundaries differ from the particular CDR boundaries defined in the present invention due to the application of different schemes. CDRs defined according to Kabat and Chothia schemes include overlaps or subsets of amino acid residues when compared with each other. Nevertheless, it is within the scope of the present invention to apply any definition to refer to the CDRs of an antibody or a variant thereof. The exact number of residues comprising a particular CDR will vary according to the sequence and size of the CDR. Those skilled in the art can generally determine which particular residues a CDR comprises based on the variable region amino acid sequence of an antibody.

Where the terms used and/or accepted in the art have two or more definitions, the definitions of the terms used herein include all of these meanings unless explicitly indicated as opposite. One specific example is the use of the term "complementarity determining regions" ("CDRs") to describe non-contiguous antigen-combining sites found within the variable regions of heavy and light chain polypeptides. This particular region is described in Kabat et al., U.S. Dept. of Health and Human Services, Sequences of Proteins of Immunological Interest (1983) and Chothia et al., J. Mol. Biol. 196:901-917 (1987), which are incorporated herein by reference in their entireties.

Kabat et al. also define a numbering scheme applicable to the variable region sequence of any antibody. One of ordinary skills in the art can apply the "Kabat numbering" scheme to any variable region sequence without depending on other experimental data beyond the sequence itself. "Kabat numbering" refers to the numbering scheme proposed by Kabat et al., U.S. Dept. of Health and Human Services in Sequence of Proteins of Immunological Interest (1983). EU or Chothia numbering scheme can also be applicable to the antibody.

The antibodies disclosed herein may be derived from any animal, including birds and mammals. Preferably, the antibody is derived from a human, a mouse, a donkey, a rabbit, a goat, a camel, a llama, a horse, or a chicken source. In another embodiment, the variable region may be derived from a condricthoid source (e.g., from a shark).

"Heavy chain constant region" comprises at least one of a CH1 domain, a hinge (e.g., upper, middle and/or lower hinge region) domain, a CH2 domain, a CH3 domain, or a variant or a fragment. The heavy chain constant region of the antibody may be derived from different immunoglobulin molecules. For example, the heavy chain constant region of the polypeptide may comprise a CH1 domain derived from an IgG1 molecule and a hinge region derived from an IgG3 molecule. In another embodiment, the heavy chain constant region may comprise a hinge region derived partially from an IgG1 molecule and partially from an IgG3 molecule. In another embodiment, a portion of the heavy chain may comprise a chimeric hinge region derived partially from an IgG1 molecule and partially from an IgG4 molecule.

"Light chain constant region" includes a part of amino acid sequence from the light chain of an antibody. Preferably, the light chain constant region comprises at least one of a constant x domain or a constant $\lambda$ domain. "Light chain-heavy chain pair" refers to a collection of light and heavy chains that can form dimers through disulfide bonds between the CL domain of the light chain and the CH1 domain of the heavy chain.

"VH domain" includes the variable domain at the amino terminus of an immunoglobulin heavy chain. "CH1 domain" includes the first constant region of an immunoglobulin heavy chain. The CH2 domain is not closely paired with other domains, but rather two N-linked branched carbohydrate chains are inserted between the two CH2 domains of an intact native IgG molecule. The CH3 domain extends from the CH2 domain to the C terminus of the IgG molecule and comprises about 108 residues. "Hinge region" includes a portion of the heavy chain region connecting the CH1 domain and CH2 domain. The hinge region comprises about 25 residues and is flexible, thereby enabling independent movement of the two N-terminal antigen-binding regions. The hinge region can be subdivided into three distinct domains: upper, middle and lower hinge domains (Roux et al., J. Immunol., 161:4083 (1998)).

"Disulfide bond" refers to a covalent bond formed between two sulfur atoms. The thiol group of cysteine can form a disulfide bond or a bridge with a second thiol group. In most naturally occurring IgG molecules, the CH1 and CL regions are linked by a disulfide bond.

"Chimeric antibody" refers to any antibody in which the variable region of the antibody is obtained or derived from a first species, and the constant region thereof (which may be intact, partial or modified) is derived from a second species. In certain embodiments, the variable region is derived from a non-human source (e.g., mouse or primate) and the constant region is derived from a human source.

"Specifically bind to" generally means that an antibody or an antigen-binding fragment forms a relatively stable complex with a specific antigen, through complementary binding of its antigen-binding domain and epitope. "Specificity" can be expressed by relative affinity of an antibody or an antigen-binding fragment for binding to a specific antigen or epitope. For example, an antibody "A" can be considered to have a higher specificity for an antigen than an antibody "B" if the antibody "A" has a greater relative affinity for the antigen than the antibody "B". Specific binding can be described by the equilibrium dissociation constant (KD). A smaller KD means a tighter binding. Methods for determining whether two molecules specifically bind to each other are well known in the art, and include, for example, equilibrium dialysis, surface plasmon resonance, biofilm layer interferometry, and the like. Antibodies that "specifically bind" to antigen a include antibodies that have an equilibrium dissociation constant KD of less than or equal to about 100 nM, less than or equal to about 10 nM, less than or equal to about 5 nM with the antigen a.

"EC$_{50}$", i.e., concentration for 50% of maximal effect, refers to the concentration that can cause 50% of maximal effect.

"Bispecific" antibody refers to an antibody having two antigen-binding sites, which may be different epitopes of the same antigen or different epitopes of different antigens.

The term "epitope" includes any protein determining region that can specifically bind to an immunoglobulin or a fragment thereof or a T cell receptor. The epitope determinant generally consists of chemically active surface groups of molecules (such as amino acids or sugar side chains), and generally has particular three-dimensional structural properties and specific charge properties.

The term "common light chain" refers to a light chain that is capable of being assembled simultaneously with different heavy chains into a complete antibody with corresponding function; the light chain can be used in expressing bispecific antibodies, or in expressing a mixture of two antibodies.

The term "effector function" refers to biological activities attributed to an immunoglobulin Fc region. Examples of immunoglobulin effector functions include: C1q binding and complement-dependent cytotoxicity (CDC), antibody-dependent cell-mediated cytotoxicity (ADCC), antibody-dependent cellular phagocytosis (ADCP), cytokine secretion, Fc receptor binding, immune complex-mediated antigen uptake by antigen-presenting cells, down regulation of cell surface receptors (such as B-cell receptors), and B-cell activation.

As used herein, unless otherwise stated, the following terms shall be understood to have the following meanings: The terms "red blood cell" and "erythrocyte" are synonymous and are used interchangeably.

The term "agglutination" refers to clumping of cells, while the term "hemagglutination" refers to clumping of a particular type of cells (i.e., red blood cells). Therefore, hemagglutination is one type of agglutination. In the hemagglutination test described herein, red blood cells form in a given well a form that can be a "button", a "halo" or an intermediate between the two. The term "in a straight line" means that masses of red blood cells flow in a straight line after the 96-well plate is tilted 45°.

"Treatment" refers to both therapeutic treatment and prophylactic or preventative measures, the purpose of which is to prevent, slow down, ameliorate, and halt undesirable physiological changes or disorders, such as the progression of a disease, including, but not limited to, alleviation of symptoms, diminishment of extent of disease, stabilized (i.e., not worsening) state of disease, delay or slowing of disease progression, amelioration, palliation, alleviation, or elimination of the disease state (whether partial or total) of state of disease, prolongation of life expectancy as compared with that in the absence of treatment, and the like, as either detectable or undetectable. Patients in need of treatment include patients already with a condition or disorder, patients susceptible to a condition or disorder, or patients in need of prevention of the condition or disorder, or patients who may or are expected to benefit from administration of the antibody or the composition disclosed herein for detection, diagnostic procedures, and/or treatment.

"Autoimmune disease" refers to the condition in which the body's immune system targets itself and mistakenly attacks healthy cells.

"Inflammatory disease" refers to a disease characterized by inflammation, which is a fundamental pathological process consisting of: complex processes of histologically clear cytological changes, cellular infiltration, and mediator release, including local reactions and the resulting morphological changes, that occur in affected vessels and adjacent tissues in response to loss or abnormal stimulation by physical, chemical, or biological agents; destruction or removal of hazardous materials; and elicitation of repair and healing responses. "Autoinflammatory disease" refers to a disease that occurs when the innate immune system causes inflammation for unknown reasons.

"Infectious disease" refers to a disease caused by a pathogen, including, for example, a viral infection, a bacterial infection, a fungal infection, or a protozoan infection such as parasitic infection.

"Tumor" refers to all neoplastic cell growth and proliferation, whether malignant or benign, as well as all precancerous and cancerous cells and tissues.

"Cancer" and "cancerous" refer to or describe the physiological condition typically characterized by abnormal cell growth/proliferation in mammals.

"Cancer", "cancerous", and "tumor" are not mutually exclusive when referred to herein.

"Patient" refers to any mammal in need of diagnosis, prognosis or treatment, including humans, dogs, cats, cavies, rabbits, rats, mice, horses, cattle and the like.

"Effective amount" refers to the amount of an active compound or medicament that results in a biological or medical response in a tissue, system, animal, subject, or human.

As used herein, the term "in need" means that the patient has been identified as in need of a particular method or treatment. In some embodiments, identification may be made by any diagnostic mode. The patient may be in need of any methods and treatments described herein.

The term "administering" or "administration" as used herein refers to administering a substance (e.g., an anti-PD-L1/CD47 bispecific antibody) for therapeutic purposes (e.g., treating an autoimmune disease, an inflammatory disease, an infectious disease, or a tumor).

The term "agent" as used herein means a chemical compound, a mixture of chemical compounds, a biological macromolecule, or an extract made from biological material.

The term "medicament" or "drug" refers to a compound or composition that is capable of inducing the desired therapeutic effect when properly administered to a patient.

Other chemical terms herein are used according to conventional usage in the art, such as the McGraw-Hill Dictionary of Chemical Terms (Ed. Parker, S., McGraw-Hill, San Francisco (1985)).

All the publications, patents, and patent applications cited herein are incorporated herein by reference in their entirety for all purposes.

## Polynucleotide Encoding Anti-PD-L1/CD47 Bispecific Antibody, Expression Vector, and Preparation Method Thereof

The anti-PD-L1/CD47 bispecific antibody of the present invention can be prepared by recombinant expression of the heavy and light chain genes of the anti-PD-L1/CD47 bispecific antibody in a host cell. For example, a host cell is transfected with one or more recombinant expression vectors carrying heavy and light chain DNA segments encoding the antibody such that the heavy and light chains are expressed in the host cell, the expressed antibody can be secreted into the medium in which the host cell is cultured, and the antibody can be isolated from the medium. "Transfect" refers to a wide variety of techniques commonly used to introduce a foreign DNA into eukaryotic host cells, such as electroporation, lipofection, calcium phosphate precipitation, DEAE-dextran transfection, and the like. Standard recombinant DNA methods for obtaining antibody heavy and light chain genes, incorporating these genes into expression vectors and introducing the vectors into host cells are well known in the art, e.g., Molecular Cloning: A Laboratory Manual (second edition, Eds. Sambrook, Fritsch and Maniatis, Cold Spring Harbor, N. Y, 1989), Current Protocols in Molecular Biology (Eds. Ausubel, F.M. Et al., Greene Publishing Associates, 1989), and those methods described in U.S. Pat. No. 4,816,397. The DNAs expressing the heavy chain and the light chain of the antibody may be placed in the same vector or placed in different vectors; if placed in different vectors, the vectors expressing the heavy and light chains of the antibody may transfect host cells in an appropriate ratio (e.g., Tihomir S. Dodev et al., A tool kit for rapid cloning and expression of recombinant antibodies, Scientific Reports, volume 4, Article number: 5885 (2014); Stefan Schlatter et al., On the Optimal Ratio of Heavy to Light Chain Genes for Efficient Recombinant Antibody Production by CHO Cells, Biotechnol Progress, 21: 122-133 (2005); Hadi Bayat et al., Evaluation of different vector design strategies for the expression of recombinant monoclonal antibody in CHO cells, Preparative Biochemistry & Biotechnology, 48(8): 822-829 (2018)). In some embodiments, the vector expressing the antibody comprises at least one promoter element, an antibody encoding sequence, a transcription termination signal and a polyA tail. Other elements include enhancers, Kozak sequences, and donor and recipient sites flanking the inserted sequence for RNA splicing. Efficient transcription can be obtained by early and late promoters of SV40, long terminal repeats from retroviruses such as RSV, HTLV1 and HIVI, and early promoters of cytomegalovirus, and promoters from other cells such as actin promoter can also be used. Suitable expression vectors may include pIRES1neo, pRetro-Off, pRetro-On, PLXSN, PIncx, pcDNA3.1 (+/-), pcDNA/Zeo (+/-), pcDNA3.1/Hygro(+/-), PSVL, PMSG, pRSVcat, pSV2dhfr, pBC12MI, pCS2, or pCHO1.0, etc. Commonly used mammalian cells (host cells) include 293 cells, Cos1 cells, Cos7 cells, CV1 cells, murine L cells, CHO cells and the like.

To express the anti-PD-L1/CD47 bispecific antibody of the present invention, the DNA encoding the full-length light and heavy chains can be inserted into an expression vector such that the genes are operably linked to transcriptional and translational control sequences. "Operably linked" means that the antibody genes are linked into a vector such that transcriptional and translational control sequences within the vector perform their intended functions of regulating the transcription and translation of the antibody genes.

Antibody genes can be inserted into an expression vector by standard methods (e.g., linking the antibody gene fragment to complementary restriction sites on the vector, or blunt end ligation if no restriction sites are present). The expression vector may already carry antibody constant region sequences prior to insertion of the light or heavy chain gene sequences related to the anti-PD-L1/CD47 bispecific antibody. For example, one method for converting the VH and VL sequences related to the anti-PD-L1/CD47 bispecific antibody into full-length antibody genes is to insert them into an expression vector that already encodes a heavy chain constant region and a light chain constant region, such that the VH segment is operably linked to the CH segment within the vector and the VL segment is operably linked to the CL segment within the vector. Alternatively, the recombinant expression vector may encode a signal peptide that facilitates secretion of antibody chains from a host cell. Alternatively, the antibody chain genes may be cloned into a vector encoding a signal peptide that facilitates secretion of antibody chains from a host cell, such that the signal peptide is linked in frame to the amino termini of the antibody chain genes. The signal peptide may be an immunoglobulin signal peptide or a heterologous signal peptide (i.e., a signal peptide from a non-immunoglobulin protein).

In addition to the antibody chain genes, the recombinant expression vector may also carry regulatory sequences that control expression of the antibody chain genes in the host cell. Regulatory sequences include promoters, enhancers and other expression control elements (e.g., polyadenylation signals) that control the transcription or translation of antibody chain genes. Such regulatory sequences are described, for example, in Goeddel, Gene Expression Technology: Methods in Enzymology 185, Academic Press, San Diego, CA, 1990. Those skilled in the art will understand that the design of an expression vector, including the choice of regulatory sequences, may depend on such factors as the selection of a host cell to be transformed, the expression level of the desired protein, and the like. Suitable regulatory sequences for expression in mammalian host cells include viral elements that direct high-level protein expression in mammalian cells, such as promoters and/or enhancers derived from cytomegalovirus (CMV) (e.g., the CMV promoter/enhancer), simian virus 40 (SV40) (e.g., the SV40 promoter/enhancer), adenovirus (e.g., the adenovirus major late promoter (AdMLP)), and polyoma. For further description of viral regulatory elements and sequences thereof, see, e.g., U.S. Pat. Nos. 5,168,062, 4,510,245 and 4,968,615.

In addition to the antibody chain genes and regulatory sequences, the recombinant expression vector may carry other sequences, such as a sequence that regulates replication of the vector in a host cell (e.g., an origin of replication) and a selectable marker gene. The selectable marker gene facilitates selection of a host cell into which the vector has been introduced (see, e.g., U.S. Pat. Nos. 4,399,216, 4,634,665 and 5,179,017). For example, generally a marker gene that imparts resistance to drugs (such as G418, hygromycin or methotrexate) to a host cell into which the vector has been introduced can be selected. Suitable selectable marker genes include the dihydrofolate reductase (DHFR) gene (for use in DHFR-host cells with methotrexate selection/amplification), the neo gene (for G418 selection), and the GS gene. For expression of heavy and light chains, an expression vector encoding the heavy and light chains is transfected into a host cell by standard techniques.

The anti-PD-L1/CD47 bispecific antibody of the present invention can be expressed in eukaryotic host cells. In certain embodiments, the antibody is expressed in eukaryotic cells, such as mammalian host cells. Exemplary mammalian host cells for expressing the recombinant antibodies of the present invention include Chinese hamster ovary cells (CHO cells) or CHO-S, CHO-dhfr-, CHO/DG44 or ExpiCHO obtained by modification of CHO cells, NSO myeloma cells, COS cells, SP2 cells, CV1 cells, murine L cells, human embryonic kidney HEK293 cells, or HEK293T, HEK293F or HEK293E cells obtained by modification of HEK293 cells. After a recombinant expression vector encoding an antibody gene is introduced into a mammalian host cell, the host cell is cultured in a medium for a period of time sufficient to allow the antibody to be expressed in the host cell or allow the antibody to be secreted into the medium to produce the antibody. The antibody can be isolated from the medium using standard protein purification methods.

For recombinant expression of the anti-PD-L1/CD47 bispecific antibodies of the present invention, a host cell can be co-transfected with three recombinant expression vectors, a first recombinant expression vector encoding the anti-PD-L1 heavy chain, a second recombinant expression vector encoding the anti-CD47 heavy chain, and a third recombinant expression vector encoding the common light chain. The three recombinant expression vectors may contain the same selectable marker, or they may each contain separate selectable markers. Alternatively, the host cell may be co-transfected with a recombinant expression vector encoding the anti-PD-L1 and the common light chain and a recombinant expression vector encoding the anti-CD47 heavy chain and the common light chain. The two vectors may contain the same selectable marker, or they may each contain separate selectable markers. The anti-PD-L1/CD47 bispecific antibodies of the present invention can also be produced by chemical synthesis (e.g., by the methods described in Solid Phase Peptide Synthesis, second edition, 1984, The Pierce Chemical Co., Rockford, Ill.). Variant antibodies can also be generated using a cell-free platform (see, e.g., Chu et al., Biochemia No. 2, 2001 (Roche Molecular Biologicals) and Murray et al., 2013, Current Opinion in Chemical Biology, 17: 420-426).

The anti-PD-L1/CD47 bispecific antibodies of the present invention produced by recombinant expression can be purified by any method known in the art for purifying immunoglobulin molecules, for example, by chromatography (e.g., ion exchange chromatography, affinity chromatography, and sizing column chromatography), centrifugation, differential solubility, or any other standard technique for purifying proteins. Further, the anti-PD-L1/ CD47 bispecific antibodies of the present invention can be fused to heterologous polypeptide sequences known in the art to facilitate purification.

## Examples

The technical schemes of the present invention will be further illustrated below through specific examples, which are not intended to limit the protection scope of the present invention. Some nonessential modifications and adjustments made by other people according to the concept of the present invention still fall within the protection scope of the present invention.

The materials, reagents, and the like used in the following examples can be commercially available unless otherwise stated.

Example 1. Preparation Method for Antibodies

Genes encoding the heavy and light chains of the anti-PD-L1/CD47 bispecific antibody were cloned into expression vectors, and the correct construction of the recombinant plasmid was verified by sequencing analysis. The recombinant plasmid was transfected into CHO cells for expression, and the supernatant was collected and purified to obtain the anti-PD-L1/CD47 bispecific antibody. The sequences of the antibody were verified to be correct by sequencing and used in the following various examples.

The amino acid sequences of the antibody BsAb-71-N297A are shown in Table 1, and the amino acid sequences of the antibody BsAb-71 are shown in Table 2.

Table 1. The amino acid sequences of the antibody BsAb-71-N297A

| Name | SEQ ID NO | Amino acid sequences |
|---|---|---|
| VHa CDR1 | 1 | GFTFSDSWIH |
| VHa CDR2 | 2 | GWISPYGGSTYYADSYRS |
| VHa CDR3 | 3 | RHWPGGFDY |
| VLa CDR1/VLb CDR1 | 4 | LASQTIGTWLA |
| VLa CDR2/VLb CDR2 | 5 | AASTLQS |
| VLa CDR3/VLb CDR3 | 6 | QQYYSTPRT |
| VHb CDR1 | 7 | DNYYWS |
| VHb CDR2 | 8 | YIYYSGNTNYNPSLKS |
| VHb CDR3 | 9 | GGRFLERY |
| VHa | 10 | EVQLVESGGGLVQPGGSLRLSCAASGFTFSDSWIHWVRQAPGKGLEWVGWISPYGGSTYYADSYRSRFTISADTSKNTAYLQMNSLRAEDTAVYYCARRHWPGGFDYWGQGTLVTVSS |
| VHb | 11 | QVQLQESGPGLVKPSETLSLTCTVSGGSLDNYYWSWIRQPPGKGLEWIGYIYYSGNTNYNPSLKSRVTISVDTSKNQFSLKLSSVTAADTAVYYCARGGRFLERYWGQGTLVTVSS |
| VLa/VLb | 12 | EIVLTQSPSSLSASVGDRVTITCLASQTIGTWLAWYQQKPGKSPQLLIYAASTLQSGVPSRFSGSGSGTDFTLTISSLQPEDVATYYCOOYYSTPRTFGOGTKVEIK |
| CHa | 13 | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYASTYRVVSVLTVLHQDWLNGKEYKCKVSNKALPAPIEKTISKAKGQPREPQVYTLPPCRDELTKNQVSLWCLVKGFYPSDIAVEWESNGQPENNYKTTPPVLDSDGSFFLYSKLTVDKSRWQQGNVFSCSVMHEALHNHYTQKSLSLSPGK |
| CLa/CLb | 14 | RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

(continued)

| Name | SEQ ID NO | Amino acid sequences |
|------|-----------|----------------------|
| CHb | 15 | ASTKGPSVFPLAPSSKSTSGGTAALGCLVKDYFPEPVTV SWNSGALTSGVHTFPAVLQSSGLYSLSSVVTVPSSSLGT QTYICNVNHKPSNTKVDKKVEPKSCDKTHTCPPCPAPE LLGGPSVFLFPPKPKDTLMISRTPEVTCVVVDVSHEDPE VKFNWYVDGVEVHNAKTKPREEQYASTYRVVSVLTVL HQDWLNGKEYKCKVSNKALPAPIEKTISKAKGQPREPQ VCTLPPSRDELTKNQVSLSCAVKGFYPSDIAVEWESNGQ PENNYKTTPPVLDSDGSFFLVSKLTVDKSRWQQGNVFS CSVMHEALHNHYTQKSLSLSPGK |
| heavy chain a | 16 | EVQLVESGGGLVQPGGSLRLSCAASGFTFSDSWIHWVR QAPGKGLEWVGWISPYGGSTYYADSYRSRFTISADTSK NTAYLQMNSLRAEDTAVYYCARRHWPGGFDYWGQGT LVTVSSASTKGPSVFPLAPSSKSTSGGTAALGCLVKDYF PEPVTVSWNSGALTSGVHTFPAVLQSSGLYSLSSVVTVP SSSLGTQTYICNVNHKPSNTKVDKKVEPKSCDKTHTCPP CPAPELLGGPSVFLFPPKPKDTLMISRTPEVTCVVVDVS HEDPEVKFNWYVDGVEVHNAKTKPREEQYASTYRVVS VLTVLHQDWLNGKEYKCKVSNKALPAPIEKTISKAKGQ PREPQVYTLPPCRDELTKNQVSLWCLVKGFYPSDIAVEW ESNGQPENNYKTTPPVLDSDGSFFLYSKLTVDKSRWQQ GNVFSCSVMHEALHNHYTQKSLSLSPGK |
| heavy chain b | 17 | QVQLQESGPGLVKPSETLSLTCTVSGGSLDNYYWSWIR QPPGKGLEWIGYIYYSGNTNYNPSLKSRVTISVDTSKNQ FSLKLSSVTAADTAVYYCARGGRFLERYWGQGTLVTVS SASTKGPSVFPLAPSSKSTSGGTAALGCLVKDYFPEPVT VSWNSGALTSGVHTFPAVLQSSGLYSLSSVVTVPSSSLG TQTYICNVNHKPSNTKVDKKVEPKSCDKTHTCPPCPAP ELLGGPSVFLFPPKPKDTLMISRTPEVTCVVVDVSHEDP EVKFNWYVDGVEVHNAKTKPREEQYASTYRVVSVLTV LHQDWLNGKEYKCKVSNKALPAPIEKTISKAKGQPREP QVCTLPPSRDELTKNQVSLSCAVKGFYPSDIAVEWESNG QPENNYKTTPPVLDSDGSFFLVSKLTVDKSRWQQGNVF SCSVMHEALHNHYTQKSLSLSPGK |

(continued)

| Name | SEQ ID NO | Amino acid sequences |
|------|-----------|----------------------|
| light chain a/light chain b (Common light chain) | 18 | EIVLTQSPSSLSASVGDRVTITCLASQTIGTWLAWYQQK PGKSPQLLIYAASTLQSGVPSRFSGSGSGTDFTLTISSLQP EDVATYYCQQYYSTPRTFGQGTKVEIKRTVAAPSVFIFPP SDEQLKSGTASVVCLLNNFYPREAKVQWKVDNALQSG NSQESVTEQDSKDSTYSLSSTLTLSKADYEKHKVYACE VTHQGLSSPVTKSFNRGEC |

Table 2. The amino acid sequences of the antibody BsAb-71

| Name | SEQ ID NO | Amino acid sequences |
|------|-----------|----------------------|
| VHa CDR1 | 1 | GFTFSDSWIH |
| VHa CDR2 | 2 | GWISPYGGSTYYADSYRS |
| VHa CDR3 | 3 | RHWPGGFDY |
| VLa CDR1/VLb CDR1 | 4 | LASQTIGTWLA |
| VLa CDR2/VLb CDR2 | 5 | AASTLQS |
| VLa CDR3/VLb CDR3 | 6 | QQYYSTPRT |
| VHb CDR1 | 7 | DNYYWS |
| VHb CDR2 | 8 | YIYYSGNTNYNPSLKS |
| VHb CDR3 | 9 | GGRFLERY |
| VHa | 10 | EVQLVESGGGLVQPGGSLRLSCAASGFTFSDSWIHWVR QAPGKGLEWVGWISPYGGSTYYADSYRSRFTISADTSK NTAYLQMNSLRAEDTAVYYCARRHWPGGFDYWGQGTL VTVSS |
| VHb | 11 | QVQLQESGPGLVKPSETLSLTCTVSGGSLDNYYWSWIRQ PPGKGLEWIGYIYYSGNTNYNPSLKSRVTISVDTSKNQFS LKLSSVTAADTAVYYCARGGRFLERYWGQGTLVTVSS |
| VLa/VLb | 12 | EIVLTQSPSSLSASVGDRVTITCLASQTIGTWLAWYQQKP GKSPQLLIYAASTLQSGVPSRFSGSGSGTDFTLTISSLQPE DVATYYCQQYYSTPRTFGQGTKVEIK |

(continued)

| Name | SEQ ID NO | Amino acid sequences |
|---|---|---|
| CHa | 19 | ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPELLG GPSVFLFPPKPKDTLMISRTPEVTCVVVDVSHEDPEVKF NWYVDGVEVHNAKTKPREEQYNSTYRVVSVLTVLHQD WLNGKEYKCKVSNKALPAPIEKTISKAKGQPREPQVYT LPPCRDELTKNQVSLWCLVKGFYPSDIAVEWESNGQPEN NYKTTPPVLDSDGSFFLYSKLTVDKSRWQQGNVFSCSV MHEALHNHYTQKSLSLSPGK |
| CLa/CLb | 14 | RTVAAPSVFIFPPSDEQLKSGTASVVCLLNNFYPREAKVQ WKVDNALQSGNSQESVTEQDSKDSTYSLSSTLTLSKAD YEKHKVYACEVTHQGLSSPVTKSFNRGEC |
| CHb | 20 | ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPELLG GPSVFLFPPKPKDTLMISRTPEVTCVVVDVSHEDPEVKF NWYVDGVEVHNAKTKPREEQYNSTYRVVSVLTVLHQD WLNGKEYKCKVSNKALPAPIEKTISKAKGQPREPQVCTL PPSRDELTKNQVSLSCAVKGFYPSDIAVEWESNGQPENN YKTTPPVLDSDGSFFLVSKLTVDKSRWQQGNVFSCSVM HEALHNHYTQKSLSLSPGK |
| heavy chain a | 21 | EVQLVESGGGLVQPGGSLRLSCAASGFTFSDSWIHWVR QAPGKGLEWVGWISPYGGSTYYADSYRSRFTISADTSK NTAYLQMNSLRAEDTAVYYCARRHWPGGFDYWGQGTL VTVSSASTKGPSVFPLAPSSKSTSGGTAALGCLVKDYFPE PVTVSWNSGALTSGVHTFPAVLQSSGLYSLSSVVTVPSSS LGTQTYICNVNHKPSNTKVDKKVEPKSCDKTHTCPPCP APELLGGPSVFLFPPKPKDTLMISRTPEVTCVVVDVSHE DPEVKFNWYVDGVEVHNAKTKPREEQYNSTYRVVSVL TVLHQDWLNGKEYKCKVSNKALPAPIEKTISKAKGQPR EPQVYTLPPCRDELTKNQVSLWCLVKGFYPSDIAVEWES NGQPENNYKTTPPVLDSDGSFFLYSKLTVDKSRWQQGN VFSCSVMHEALHNHYTQKSLSLSPGK |

(continued)

| Name | SEQ ID NO | Amino acid sequences |
|---|---|---|
| heavy chain b | 22 | QVQLQESGPGLVKPSETLSLTCTVSGGSLDNYYWSWIRQ PPGKGLEWIGYIYYSGNTNYNPSLKSRVTISVDTSKNQFS LKLSSVTAADTAVYYCARGGRFLERYWGQGTLVTVSSA STKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPELLG GPSVFLFPPKPKDTLMISRTPEVTCVVVDVSHEDPEVKF NWYVDGVEVHNAKTKPREEQYNSTYRVVSVLTVLHQD WLNGKEYKCKVSNKALPAPIEKTISKAKGQPREPQVCTL PPSRDELTKNQVSLSCAVKGFYPSDIAVEWESNGQPENN YKTTPPVLDSDGSFFLVSKLTVDKSRWQQGNVFSCSVM HEALHNHYTQKSLSLSPGK |
| light chain a/light chain b (Common light chain) | 18 | EIVLTQSPSSLSASVGDRVTITCLASQTIGTWLAWYQQKP GKSPQLLIYAASTLQSGVPSRFSGSGSGTDFTLTISSLQPE DVATYYCQQYYSTPRTFGQGTKVEIKRTVAAPSVFIFPPS DEQLKSGTASVVCLLNNFYPREAKVQWKVDNALQSGN SQESVTEQDSKDSTYSLSSTLTLSKADYEKHKVYACEVT HQGLSSPVTKSFNRGEC |

Example 2. Agglutination Assay on Red Blood Cells

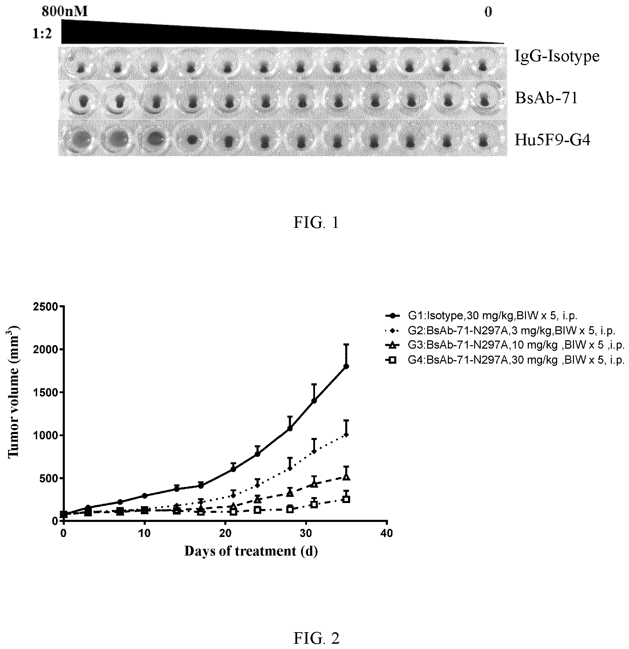

The specific assay method was as follows: fresh human blood was collected, washed with normal saline three times, and then prepared as a 2% human red blood cell suspension in normal saline; the 2% human red blood cell suspension was mixed with a test antibody (the highest concentration being 800 nM, two-fold serial dilution to obtain 11 post-dilution concentrations in total, the vehicle being normal saline) in equal volume, and the mixture was incubated at 37 °C for 4 h; then the agglutination of red blood cells caused by the antibody was evaluated by tilting the 96-well U-shaped plate 45° and observing the flow direction of the red blood cell cluster, and if massed of red blood cells flowed in a straight line, it indicated that the red blood cells did not agglutinate. The results for the agglutination reaction of red blood cells are shown in FIG. 1. As can be seen from FIG. 1, the positive control antibody Hu5F9-G4 at a high concentration level caused agglutination of red blood cells, and the exemplary bispecific antibody BsAb-71 of the present invention did not cause agglutination of red blood cells, and was significantly less active in promoting agglutination of red blood cells than the positive control antibody Hu5F9-G4. Among these antibodies, the positive control antibody Hu5F9-G4 (Magrolimab) is a human CD47 antibody transiently expressed in HEK293F cells, the sequence of which is identical to that of the antibody "Hu5F9" in U.S. Patent No. US2015/0183874A1; the negative control antibody IgG-Isotype was purchased from Sino Biological Inc. (Cat. No.: HG1K).

Example 3. *In Vivo* Pharmacodynamic Test

3.1 Evaluation of the anti-tumor effect of the antibody BsAb-71-N297A in a tumor-bearing model of humanized mouse C57BL/6-hSIRPα subcutaneously inoculated with mouse colon cancer cells MC38-hCD47(Tg) (Jiangsu GemPharmatech Co., Ltd.).
1) Tumor cell inoculation: MC38-hCD47(Tg) cells in logarithmic phase were collected, the medium was removed, and the cells were washed twice with PBS and then inoculated subcutaneously into the right forelimb of humanized mice C57BL/6-hSIRPα (female; 6-9 weeks old; Jiangsu GemPharmatech Co., Ltd.) in an amount of $1 \times 10^6$ cells/100 μL/mouse.

2) Grouping and administration: When the mean tumor volume reached 77.69 mm$^3$, the mice were randomly grouped, with 10 in each group. The day of grouping was defined as D0, and the intraperitoneal administration (i.p.) was performed on D0, D3, D7, D10, D14, D17, D21, D24, D28, and D31. The administration dosage and administration mode are shown in Table 3.

Table 3. Administration regimen

| Group | Treatment group | Dose (mg/kg) | Route | Number of actual administrations |
|---|---|---|---|---|
| G1 | Isotype (Sino Biological, Cat No.: HG4K) | 30 | i.p. | BIW $\times$ 5, for 10 administrations in total |
| G2 | BsAb-71-N297A | 3 | i.p. | BIW $\times$ 5, for 10 administrations in total |
| G3 | BsAb-71-N297A | 10 | i.p. | BIW $\times$ 5, for 10 administrations in total |
| G4 | BsAb-71-N297A | 30 | i.p. | BIW $\times$ 5, for 10 administrations in total |
| Note: i.p.: intraperitoneal administration; BIW $\times$ 5: twice a week, for 10 administrations in total; vehicle: normal saline. | | | | |

3) Experimental observation and data acquisition:

After cell inoculation, the effect of tumors on the normal behavior of the animals was routinely monitored weekly. Specifically, routine monitoring included activity, food and water intake, weight gain or loss, eyes, hair and other abnormal conditions of the experimental animals.

After the administration was started, the mice were observed for tumor size and determined for body weight on days of D0, D3, D7, D10, D14, D17, D21, D24, D28, D31, and D35. The tumor volume was calculated as follows: tumor volume (mm$^3$) = 0.5 $\times$ (long diameter of tumor $\times$ short diameter of tumor$^2$). The *in vivo* experimental endpoint was on D35 after administration. The tumor growth inhibition of relative tumor volume (TGItv) was calculated.

TGItv (tumor growth inhibition of relative tumor volume) calculation formula:

$$\text{TGItv} = (1 - (\text{mean RTV}_{\text{treatment group}})/(\text{mean RTV}_{\text{vehicle control group}})) \times 100\%; \text{mean RTV}_{\text{treatment group}}:$$

mean RTV of treatment group, mean RTV$_{\text{vehicle control group}}$: mean RTV of vehicle control group; wherein RTV$_n$ = V$_{nt}$/V$_{n0}$; V$_{nt}$: tumor volume of mice numbered n on day t, V$_{n0}$: tumor volume of mice numbered n on day 0, and RTV$_n$: relative tumor volume of mice numbered n on day t.

4) Statistical analysis: The experimental results for the tumor volume, mouse body weight, tumor weight, and the like of each group of animals were expressed as mean $\pm$ standard error (Mean $\pm$ SEM). Different treatment groups were compared with the control group for any significant difference by the independent sample T test. Data were analyzed using SPSS. $P < 0.05$ means that there was a significant difference.

As shown in FIG. 2 and Table 4, the antibodies BsAb-71-N297A (3 mg/kg), BsAb-71-N297A (10 mg/kg), and BsAb-71-N297A (30 mg/kg) all showed significant tumor inhibitory effects. At a dose of 30 mg/kg, the tumor growth inhibition of relative tumor volume (TGItv) at the test endpoint (D35) was 86.22%.

Table 4. Tumor volume and tumor growth inhibition on D35 after administration

| Group | Tumor volume (mm$^3$) | TGItv | P value |
|---|---|---|---|
| G1 (control) | 1803.59$\pm$255.87 | -- | -- |
| G2 | 1006.41$\pm$165.89 | 43.64% | 0.018 |
| G3 | 517.11$\pm$117.94 | 75.96% | 0.001 |
| G4 | 254.1$\pm$99.66 | 86.22% | < 0.001 |

3.2 Evaluation of the anti-tumor effect of the antibody BsAb-71 in a tumor-bearing model of humanized mouse C57BL/6-hSIRPα subcutaneously inoculated with mouse colon cancer cells MC38-hCD47(Tg) (Jiangsu GemPharmatech Co., Ltd.).

1) Tumor cell inoculation: MC38-hCD47(Tg) cells in logarithmic phase were collected, the medium was removed, and the cells were washed twice with PBS and then inoculated subcutaneously into the right forelimb of humanized mice C57BL/6-hSIRPα (female; 5-8 weeks old; Jiangsu GemPharmatech Co., Ltd.) in an amount of 1 $\times$ 10$^6$ cells/100 μL/ mouse.

2) Grouping and administration: When the mean tumor volume reached 77.71 mm$^3$, the mice were randomly grouped, with 10 in each group. The day of grouping was defined as D0, and the intraperitoneal administration (i.p.) was performed on D0, D3, D7, D10, D14, D17, D21, D24, D28, and D31. The administration dosage and administration mode are shown in Table 5.

Table 5. Administration regimen

| Group | Treatment group | Dose (mg/kg) | Route | Number of actual administrations |
|---|---|---|---|---|
| G1 | Isotype IgG1 (Sino Biological, Cat No.: HG1K) | 15 | i.p. | BIW × 5, for 10 administrations in total |
| G2 | BsAb-71 | 5 | i.p. | BIW × 5, for 10 administrations in total |
| G3 | BsAb-71 | 10 | i.p. | BIW × 5, for 10 administrations in total |
| G4 | BsAb-71 | 15 | i.p. | BIW × 5, for 10 administrations in total |
| Note: i.p.: intraperitoneal administration; BIW × 5: twice a week, for 10 administrations in total; vehicle: normal saline. | | | | |

3) Experimental observation and data acquisition:

After cell inoculation, the effect of tumors on the normal behavior of the animals was routinely monitored weekly. Specifically, routine monitoring included activity, food and water intake, weight gain or loss, eyes, hair and other abnormal conditions of the experimental animals.

After the administration was started, the mice were observed for tumor size and determined for body weight on days of D0, D3, D6, D9, D13, D16, D20, D23, D27, D30, and D34. The tumor volume was calculated as follows: tumor volume (mm$^3$) = 0.5 × (long diameter of tumor × short diameter of tumor$^2$). The *in vivo* experimental endpoint was on D34 after administration. The tumor growth inhibition of relative tumor volume (TGItv) was calculated.

TGItv (tumor growth inhibition of relative tumor volume) calculation formula:

$$\text{TGItv} = (1 - (\text{mean RTV}_{\text{treatment group}})/(\text{mean RTV}_{\text{vehicle control group}})) \times 100\%;\ \text{mean RTV}_{\text{treatment group}}:$$

mean RTV of treatment group, mean RTV$_{\text{vehicle control group}}$: mean RTV of vehicle control group; wherein RTV$_n$ = V$_{nt}$/V$_{n0}$, V$_{nt}$: tumor volume of mice numbered n on day t, V$_{n0}$: tumor volume of mice numbered n on day 0, and RTV$_n$: relative tumor volume of mice numbered n on day t.

4) Statistical analysis: The experimental results for the tumor volume, mouse body weight, tumor weight, and the like of each group of animals were expressed as mean ± standard error (Mean ± SEM). Different treatment groups were compared with the control group for any significant difference by the independent sample T test. Data were analyzed using SPSS. P < 0.05 means that there was a significant difference.

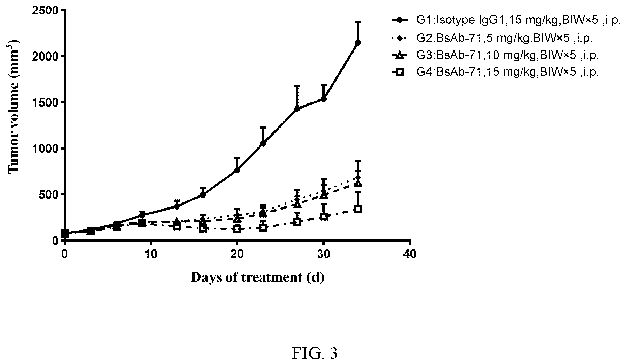

As shown in FIG. 3 and Table 6, the antibodies BsAb-71 (5 mg/kg), BsAb-71 (10 mg/kg), and BsAb-71 (15 mg/kg) all showed significant tumor inhibitory effects. At a dose of 15 mg/kg, the tumor growth inhibition of relative tumor volume (TGItv) at the test endpoint (D34) was 84.50%.

Table 6. Tumor volume and tumor growth inhibition on D34 after administration

| Group | Tumor volume (mm$^3$) | TGItv | P value |
|---|---|---|---|
| G1 (control) | 2149.90±301.01 | -- | -- |
| G2 | 689.28±236.25 | 66.97% | 0.0001 |
| G3 | 622.66±192.05 | 70.71% | < 0.0001 |
| G4 | 341.84±259.09 | 84.50% | < 0.0001 |

Example 4. Pharmacokinetic and Toxicological Tests

a) Pharmacokinetics

In this study, 18 cynomolgus monkeys (half female and half male) were used and randomly divided into 3 groups, with 3 females and 3 males in each group. Without fasting, the three groups of animals were given the antibody BsAb-71-N297A at 2.5 mg/kg, 20 mg/kg, and 80 mg/kg, respectively, by a single intravenous infusion (60 min), for 1 administration in total, with a vehicle of 0.9% sodium chloride injection. Serum samples were collected from each group of animals until 504 h (Day 22) after administration. According to the plasma concentration data, primary pharmacokinetic parameters were calculated by noncompartmental analysis of Phoenix WinNonlin® 7.0 software.

As shown in Table 7, at a dose range of 2.5-80 mg/kg, after male and female cynomolgus monkeys were given the antibody BsAb-71-N297A by a single intravenous infusion without fasting, the systemic exposure of the antibody BsAb-71-N297A in serum was not significantly different between the sexes, and the systemic exposure of the antibody BsAb-71-N297A in serum was positively correlated with the administration dose.

Table 7. Primary pharmacokinetic parameters (Mean ± SD) of the antibody BsAb-71-N297A in serum after the cynomolgus monkeys were given the antibody BsAb-71-N297A by a single intravenous infusion

| Administration dose | Sex of animals | Number of animals | $T_{1/2}$ | $T_{max}$ | $C_{max}$ | $AUC_{(0-t)}$ | $AUC_{(0-\infty)}$ | Vd | Cl | $MRT_{(0-t)}$ | $MRT_{(0-\infty)}$ |
|---|---|---|---|---|---|---|---|---|---|---|---|
| mg/kg | | n | day | h | ng/mL | day*ng/mL | day*ng/mL | mL/kg | mL/day/kg | day | day |
| 2.5 | Male | 3 | 1.37 ± 0.21 | 1.083 ± 0.000 | 32730.94 ± 7536.25 | 38299.87 ± 12211.54 | 39543.82 ± 13523.47 | 130.57 ± 31.34 | 67.65 ± 19.46 | 1.47 ± 0.23 | 1.70 ± 0.40 |
| | Female | 3 | 1.32 ± 0.22 | 1.17 ± 0.29 | 36596.30 ± 5554.19 | 43591.10 ± 11306.74 | 44728.72 ± 11737.65 | 110.54 ± 29.33 | 58.24 ± 13.43 | 1.46 ± 0.16 | 1.67 ± 0.26 |
| 20 | Male | 3 | 1.48 ± 0.19 | 1.055 ± 0.048 | 542652.51 ± 47786.32 | 778701.46 ± 186979.54 | 780190.75 ± 187459.86 | 55.82 ± 8.60 | 26.62 ± 6.18 | 2.07 ± 0.19 | 2.10 ± 0.19 |
| | Female | 3 | 1.28 ± 0.08 | 1.00 ± 0.00 | 500030.49 ± 136768.65 | 686896.26 ± 250622.93 | 696864.79 ± 242344.86 | 57.30 ± 20.09 | 30.89 ± 9.57 | 1.62 ± 0.27 | 1.77 ± 0.21 |
| 80 | Male | 3 | 2.41 ± 1.81 | 2.722 ± 2.839 | 2351366.09 ± 972432.76 | 6154129.30 ± 3454559.44 | 6290876.72 ± 3649922.27 | 44.40 ± 26.23 | 15.37 ± 6.94 | 3.43 ± 1.25 | 3.75 ± 1.66 |
| | Female | 3 | 1.69 ± 0.33 | 1.083 ± 0.000 | 1682302.34 ± 328044.22 | 3458807.69 ± 1101208.82 | 3467932.71 ± 1108355.03 | 58.55 ± 10.71 | 25.13 ± 9.69 | 2.72 ± 0.27 | 2.75 ± 0.29 |

b) Toxicity evaluation

1) Single dose toxicity test

To investigate the toxic response of the antibody BsAb-71-N297A in cynomolgus monkeys after a single intravenous infusion. In this test, a total of 4 groups were designed, with 1 female and 1 male in each group. Grouping was as follows: an excipient group (0 mg/kg), and antibody BsAb-71-N297A low-dose (30 mg/kg), medium-dose (100 mg/kg), and high-dose (300 mg/kg) groups. The administration was performed by intravenous infusion for 1 h once, with a vehicle of 0.9% sodium chloride injection, followed by an observation period of 14 days.

After the cynomolgus monkeys were given the antibody BsAb-71-N297A at 30 mg/kg, 100 mg/kg, and 300 mg/kg by a single intravenous infusion, they were observed for 2 weeks. No toxic response related to administration of the antibody BsAb-71-N297A was seen in terms of indexes such as symptoms, body weight, food consumption, body temperature, electrocardiogram, blood pressure, hematology, blood coagulation, biochemistry, urine, and gross anatomy, and the maximum tolerated dose was more than 300 mg/kg.

2) Repeated dose toxicity test

To investigate the toxicity response and toxicokinetic characteristics of the antibody BsAb-71-N297A in cynomolgus monkeys after repeated intravenous infusions. In this test, a total of 4 groups were designed, with 5 females and 5 males in each group. Grouping was as follows: an excipient group (0 mg/kg), and antibody BsAb-71-N297A low-dose (5 mg/kg), medium-dose (30 mg/kg), and high-dose (100 mg/kg) groups. The administration was performed by intravenous infusion for 1 h once a week (Day 1, Day 8, Day 15, Day 22, and Day 29), for a total of 5 administrations, with a vehicle of 0.9% sodium chloride injection. The day of the first administration of the animals was recorded as the first day (Day 1) of the experiment.

As shown in Table 8, after single and repeated intravenous administrations of the antibody BsAb-71-N297A at 5 mg/kg, 30 mg/kg, and 100 mg/kg to cynomolgus monkeys, the exposure of the antibody BsAb-71-N297A in serum increased with dose, with a ratio of increase higher than the ratio of increase in dose, and there was no difference in the exposure between sexes; after four repeated administrations, the exposure of the antibody BsAb-71-N297A in the 5 mg/kg and 30 mg/kg groups decreased significantly, and there was no significant change in the exposure in the 100 mg/kg group.

Under the conditions of this test, after the cynomolgus monkeys were repeatedly given the excipient (0 mg/kg), and the antibody BsAb-71-N297A at 5 mg/kg, 30 mg/kg, and 100 mg/kg by intravenous infusion once a week for 4 consecutive weeks, followed by 4-week recovery after discontinuation, no toxic response related to the test sample was observed in the dose groups of the test sample, and the no observed adverse effect level (NOAEL) was 100 mg/kg.

Table 8. Primary toxicokinetic parameters after single and four repeated administrations of BsAb-71-N297A

| Period | Sex | Dose (mg/kg) | Mean $t_{1/2}$ (h) | Mean $T_{max}$ (h) | Mean $C_{max}$ (ng/mL) | Mean $AUC_{(0-t)}$ (h·ng/mL) |
|---|---|---|---|---|---|---|
| Day 1 (the first administration) | Male | 5 | 41.932 | 1.083 | 89560 | 2657959 |
| | | 30 | 58.705 | 1.083 | 837886 | 38499915 |
| | | 100 | 63.003 | 1.083 | 2826790 | 163801841 |
| | Female | 5 | 54.556 | 1.083 | 84175 | 2765594 |
| | | 30 | 54.881 | 1.083 | 684387 | 30914651 |
| | | 100 | 76.965 | 1.666 | 2490053 | 134376910 |
| Day 22 (the fourth administration) | Male | 5 | 3.519 | 1.083 | 33641 | 165128 |
| | | 30 | 15.932 | 2.466 | 783337 | 23248923 |
| | | 100 | 11.526 | 1.666 | 3124515 | 112686075 |
| | Female | 5 | 3.332 | 1.083 | 62684 | 382436 |
| | | 30 | 6.450 | 1.083 | 562898 | 5776507 |
| | | 100 | 41.392 | 0.966 | 2895196 | 152076893 |

The immunogenicity results showed that: on Day 14 and Day 28 after the repeated administrations, an anti-drug antibody could be detected in the antibody BsAb-71-N297A low-dose, medium-dose, and high-dose groups; the number of animals positive for the anti-drug antibody on Day 28 was significantly higher than that on Day 14, the anti-drug antibody positive rate of the three dose groups on Day 28 and Day 56 ranged from 75% (3/4) to 100% (10/10), and the three dose groups were arranged in terms of the number of animals positive for the anti-drug antibody in the following order: the antibody BsAb-71-N297A low-dose group > medium-dose group > high-dose group; the anti-drug antibody could still be detected by the end of the recovery period.

c) Hemolysis test

New Zealand rabbit red blood cells were used for the hemolysis test. The antibody BsAb-71-N297A injection (50.5 mg/mL) subjected to a 10-fold dilution did not cause hemolysis or agglutination of New Zealand rabbit red blood cells *in vitro.*

Example 5. Clinical Study of Antibody BsAb-71-N297A

This study is a multi-center, open-label phase Ia/Ib clinical trial for evaluating the safety, tolerability, pharmacokinetic characteristics, and preliminary clinical efficacy of antibody BsAb-71-N297A injection in patients with advanced malignant tumors. This study was conducted to explore the maximum tolerated dose (MTD) or maximum administered dose (MAD) and provide recommended doses and a reasonable administration regimen for phase II or subsequent clinical trials.

The study was divided into 2 stages:

First stage: dose escalation phase Ia study. Rapid titration and the "3 + 3" dose escalation rule were used to explore the safety, tolerability, and pharmacokinetic characteristics of the antibody BsAb-71-N297A.
In the dose escalation study, 7 dose groups were arranged, namely, 0.1 mg/kg group, 0.3 mg/kg group, 1 mg/kg group, 3 mg/kg group, 10 mg/kg group, 20 mg/kg group, and 40 mg/kg group. Among these dose groups, the 0.1 mg/kg group and the 0.3 mg/kg group were subjected to dose escalation based on an accelerated titration method; the 1 mg/kg group, 3 mg/kg group, 10 mg/kg group, 20 mg/kg group, and 40 mg/kg group were subjected to dose escalation following the standard "3 + 3" rule.

The dose escalation rule was as follows:

The first 2 dose groups (0.1 mg/kg group and 0.3 mg/kg group) were treated with a dose escalation protocol based on accelerated titration, i.e., cohort size = 1. One subject in the 0.1 mg/kg group was first enrolled, and if no grade ≥ 2 toxic response related to the study drug was observed, the dose escalation could be directly advanced to the 0.3 mg/kg group. By analogy with this rule ("1 + 1"), accelerated dose escalation was conducted in the 1 mg/kg group. During the accelerated dose escalation, if any grade ≥ 2 toxic response related to the study drug was observed, the dose escalation based on accelerated titration was discontinued, 2 additional subjects in the dose group were enrolled, and the study was performed following the standard "3 + 3" dose escalation (i.e. cohort size = 3) rule until the MTD or MAD was reached. Starting with the 1 mg/kg group, the dose escalation rule was adjusted to the standard "3 + 3" rule.
Second stage: phase Ib study with dose extension. This study was performed to further explore the safety and clinical efficacy of the antibody BsAb-71-N297A.
The study drug was administered once every 2 weeks by intravenous infusion for ≥ 60 min. If the patient had infusion-related reactions and was able to continue treatment, a prophylactic administration of drugs such as diphenhydramine or acetaminophen could be considered based on previous experience and clinical practice. Treatment with the study drug should be continued until the onset of: disease progression, or withdrawal due to intolerable toxicity, or receipt of a new anti-tumor therapy due to lack of efficacy benefit, or withdrawal voluntarily due to withdrawal of informed consent and for other reasons, or up to 1 year of treatment, whichever came first. The first and second administration cycles were the DLT evaluation period, i.e., from the first administration to 28 days after administration.
The tolerance evaluation indexes involved: dose-limiting toxicity (DLT) events and their incidence; the safety evaluation indexes involved: vital signs, physical examination, laboratory examination (routine blood test, blood biochemistry, thyroid function, coagulation, urinalysis, stool routine, and pregnancy test), ECOG score, electrocardiogram, adverse events (including immune-related adverse events), and the like.
The immunogenicity evaluation indexes involved: the sample positive rate and individual positive rate for the anti-drug antibody (ADA), the titer of the ADA-positive sample, and whether the ADA-positive sample will continue to be tested for a neutralizing antibody (NAb).

Subjects in all dose groups were required for blood sample collection at specified time points during treatment (first 6 treatment cycles), and the plasma concentrations ($C_{trough}$) were monitored for 60 min before administration. At each time point, 2 mL of blood samples were scheduled to be collected for the determination of the drug concentration level in serum and the study of pharmacokinetic (PK) characteristics. The following PK parameters were calculated from the actual administration dose and the actual sampling time by noncompartmental analysis: single administration: $C_{max}$, $T_{max}$, $T_{1/2}$, CL, Vd, Ke, MRT, $AUC_{(0-\tau)}$, and $AUC_{(0-\infty)}$; multiple administrations: $C_{max, ss}$, $C_{avg, ss}$, $C_{min, ss}$, $AUC_{(0-\tau)ss}$, $AUC_{(0-\infty)ss}$, $T_{max, ss}$, $T_{1/2, ss}$, CL, Vss, Ke, MRT, accumulation index ($R_{ac}$), and fluctuation index (DF).

Clinical efficacy evaluation: objective response rate (ORR), duration of response (DOR), disease control rate (DCR), progression-free survival (PFS), and overall survival (OS) as defined by RECIST 1.1 (lymphomas were evaluated according to Lugano2014 evaluation criteria).

Objective response rate (ORR): proportion of subjects in complete response (CR) and partial response (PR); duration of response (DOR): the duration of ORR is reflected by the time from the first assessment of the tumor as objective response (OR) to the first assessment of the tumor as PD (progressive disease) or death of any cause prior to PD, reflecting the duration of ORR; progression-free survival (PFS): the time from the first administration to the occurrence of objective progression of the tumor or all-cause death (whichever occurred first). "MTD" is the explored highest dose level at which $\leq 1/6$ subjects in a certain dose group were observed to experience DLT during the DLT evaluation period.

Adverse events (AE) were evaluated based on CTCAE v5.0. Dose-limiting toxicity (DLT) is defined as AE that occurs during the DLT observation period and is considered to be at least possibly related to the study drug, as follows:

■ Grade 5 toxicity;
■ Hematological toxicity:

· Grade 4 hematological toxicity (note: grade 3-4 lymphopenia was not included in DLT);
· Grade 4 thrombocytopenia of any duration;
· Grade 3 thrombocytopenia with a bleeding tendency or requiring platelet infusion;
· Grade 4 neutropenia of any duration;
· Grade 3 neutropenia with infection lasting $\geq 7$ days or grade 3 neutropenia with fever;

■ Non-hematologic toxicity:

·Grade 3 or 4 non-hematologic toxicity (note: (1) except for grade 3 nausea, vomiting, and rash; (2) except for grade 3 diarrhea that can be controlled within 3 days after the clinical intervention; (3) except for fatigue/weakness that lasts less than 7 days);
·Grade 3 or 4 non-hematological toxicity by clinical laboratory examination that meets any one of the following conditions: (1) requiring clinical intervention; (2) leading to hospitalization; (3) lasting $\geq 7$ days; (4) all that results in the discontinuation of treatment in the subject in cycle 1; (5) any treatment-related toxicity that results in a delay of more than 2 weeks in the administration of cycle 2;
·Grade $\geq 2$ brain lesions;
·Ocular toxicity requiring systemic treatment (note: any patient with changes in visual acuity or ocular toxicity of grade 2 or higher will be evaluated by an ophthalmologist).

In the determination of DLT, some AEs that are clearly and indisputably due to disease progression or causes other than DLT should not be included in DLT.

**Claims**

1. A method for treating an autoimmune disease, an inflammatory disease, an infectious disease, or a tumor, comprising administering to a patient in need thereof an effective amount of an anti-PD-L1/CD47 bispecific antibody or an antigen-binding fragment; wherein the anti-PD-L1/CD47 bispecific antibody or the antigen-binding fragment is administered once every two days to once every six weeks, at a dose of 0.1 mg/kg to 100 mg/kg or at a dose of 6 mg to 3000 mg;

the anti-PD-L1/CD47 bispecific antibody or the antigen-binding fragment comprises a variable region a specifically binding to PD-L1 and a variable region b specifically binding to CD47; wherein the variable region a comprises at least one or more of VHa CDR1 set forth in SEQ ID NO: 1, VHa CDR2 set forth in SEQ ID NO: 2, VHa CDR3 set forth in SEQ ID NO: 3, VLa CDR1 set forth in SEQ ID NO: 4, VLa CDR2 set forth in SEQ ID NO: 5, and VLa CDR3 set forth in SEQ ID NO: 6; the variable region b comprises at least one or more of VHb CDR1 set forth in SEQ ID NO: 7, VHb CDR2 set forth in SEQ ID NO: 8, VHb CDR3 set forth in SEQ ID NO: 9, VLb CDR1 set forth in SEQ ID NO: 4, VLb CDR2 set forth in SEQ ID NO: 5, and VLb CDR3 set forth in SEQ ID NO: 6.

2. The method according to claim 1, wherein the anti-PD-L1/CD47 bispecific antibody or the antigen-binding fragment binds to PD-L1 with greater affinity than to CD47.

3. The method according to claim 1 or 2, wherein the variable region a comprises VHa CDR1 set forth in SEQ ID NO: 1, VHa CDR2 set forth in SEQ ID NO: 2, VHa CDR3 set forth in SEQ ID NO: 3, VLa CDR1 set forth in SEQ ID NO: 4, VLa CDR2 set forth in SEQ ID NO: 5, and VLa CDR3 set forth in SEQ ID NO: 6; the variable region b comprises VHb CDR1 set forth in SEQ ID NO: 7, VHb CDR2 set forth in SEQ ID NO: 8, VHb CDR3 set forth in SEQ ID NO: 9, VLb CDR1 set forth in SEQ ID NO: 4, VLb CDR2 set forth in SEQ ID NO: 5, and VLb CDR3 set forth in SEQ ID NO: 6.

4. The method according to any one of claims 1-3, wherein the variable region a comprises a heavy chain variable region VHa and a light chain variable region VLa; wherein the VHa comprises an amino acid sequence set forth in SEQ ID NO: 10, or an amino acid sequence having at least 80% or 90% identity to the amino acid sequence set forth in SEQ ID NO: 10, or an amino acid sequence having one or more conservative amino acid substitutions as compared with the amino acid sequence set forth in SEQ ID NO: 10; and/or the VLa comprises an amino acid sequence set forth in SEQ ID NO: 12, or an amino acid sequence having at least 80% or 90% identity to the amino acid sequence set forth in SEQ ID NO: 12, or an amino acid sequence having one or more conservative amino acid substitutions as compared with the amino acid sequence set forth in SEQ ID NO: 12.

5. The method according to any one of claims 1-4, wherein the variable region b comprises a heavy chain variable region VHb and a light chain variable region VLb; wherein the VHb comprises an amino acid sequence set forth in SEQ ID NO: 11, or an amino acid sequence having at least 80% or 90% identity to the amino acid sequence set forth in SEQ ID NO: 11, or an amino acid sequence having one or more conservative amino acid substitutions as compared with the amino acid sequence set forth in SEQ ID NO: 11; and/or the VLb comprises an amino acid sequence set forth in SEQ ID NO: 12, or an amino acid sequence having at least 80% or 90% identity to the amino acid sequence set forth in SEQ ID NO: 12, or an amino acid sequence having one or more conservative amino acid substitutions as compared with the amino acid sequence set forth in SEQ ID NO: 12.

6. The method according to claim 5, wherein the anti-PD-L1/CD47 bispecific antibody further comprises a heavy chain constant region CHa and a heavy chain constant region CHb, wherein the heavy chain constant region CHa is linked to the heavy chain variable region VHa to form a heavy chain a, and the heavy chain constant region CHb is linked to the heavy chain variable region VHb to form a heavy chain b; or the heavy chain constant region CHa and/or the heavy chain constant region CHb comprises an amino acid mutation of N297A, wherein an amino acid position is numbered according to the Eu numbering scheme.

7. The method according to claim 6, wherein the heavy chain constant region CHa comprises an amino acid sequence set forth in SEQ ID NO: 13 or 19, or an amino acid sequence having at least 80% or 90% identity to the amino acid sequence set forth in SEQ ID NO: 13 or 19, or an amino acid sequence having one or more conservative amino acid substitutions as compared with the amino acid sequence set forth in SEQ ID NO: 13 or 19; and/or the heavy chain constant region CHb comprises an amino acid sequence set forth in SEQ ID NO: 15 or 20, or an amino acid sequence having at least 80% or 90% identity to the amino acid sequence set forth in SEQ ID NO: 15 or 20, or an amino acid sequence having one or more conservative amino acid substitutions as compared with the amino acid sequence set forth in SEQ ID NO: 15 or 20.

8. The method according to any one of claims 1-7, wherein the anti-PD-L1/CD47 bispecific antibody comprises a heavy chain a, a heavy chain b, a light chain a, and a light chain b; wherein

the heavy chain a comprises an amino acid sequence set forth in SEQ ID NO: 16 or 21, or an amino acid sequence having at least 80% or 90% identity to the amino acid sequence set forth in SEQ ID NO: 16 or 21, or an amino acid sequence having one or more conservative amino acid substitutions as compared with the amino acid sequence set forth in SEQ ID NO: 16 or 21; and/or
the heavy chain b comprises an amino acid sequence set forth in SEQ ID NO: 17 or 22, or an amino acid sequence having at least 80% or 90% identity to the amino acid sequence set forth in SEQ ID NO: 17 or 22, or an amino acid sequence having one or more conservative amino acid substitutions as compared with the amino acid sequence set forth in SEQ ID NO: 17 or 22; and/or

the light chain a and the light chain b comprise identical amino acid sequences; or the light chain a and light chain b each comprise an amino acid sequence set forth in SEQ ID NO: 18, or an amino acid sequence having at least 80% or 90% identity to the amino acid sequence set forth in SEQ ID NO: 18, or an amino acid sequence having one or more conservative amino acid substitutions as compared with the amino acid sequence set forth in SEQ ID NO: 18.

9.  The method according to any one of claims 1-8, wherein the tumor comprises a benign tumor or cancer; or the cancer comprises a solid tumor, a hematologic cancer, or a metastatic lesion; or the hematologic cancer comprises leukemia, lymphoma, or myeloma; or the hematologic cancer comprises acute lymphocytic leukemia, acute myeloid leukemia, chronic myelogenous leukemia, chronic lymphocytic leukemia, myeloproliferative diseases/tumors, myelodysplastic syndrome, diffuse large B-cell lymphoma, Hodgkin's lymphoma, non-Hodgkin's lymphoma, Burkitt's lymphoma, follicular lymphoma, multiple myeloma, giant cell myeloma, heavy chain myeloma, or light chain or Bence-Jones myeloma; or the solid tumor comprises breast cancer, ovarian cancer, lung cancer, prostate cancer, melanoma, colorectal cancer, head and neck cancer, bladder cancer, esophageal cancer, liver cancer, renal cancer, gastric cancer, cervical cancer, pancreatic cancer, thyroid cancer, neuroglioma, salivary gland cancer, leiomyosarcoma, thymus cancer, or epithelial cancer.

10. The method according to any one of claims 1-9, wherein one treatment cycle is for about two weeks.

11. The method according to any one of claims 1-9, wherein the anti-PD-L1/CD47 bispecific antibody or the antigen-binding fragment is administered once about every two weeks.

12. Use of an anti-PD-L1/CD47 bispecific antibody or an antigen-binding fragment in the treatment of an autoimmune disease, an inflammatory disease, an infectious disease, or a tumor; wherein the anti-PD-L1/CD47 bispecific antibody or the antigen-binding fragment is administered in an amount of 0.1 mg/kg to 100 mg/kg once every two days to once every six weeks;

the anti-PD-L1/CD47 bispecific antibody or the antigen-binding fragment comprises a variable region a specifically binding to PD-L1 and a variable region b specifically binding to CD47; wherein the variable region a comprises at least one or more of VHa CDR1 set forth in SEQ ID NO: 1, VHa CDR2 set forth in SEQ ID NO: 2, VHa CDR3 set forth in SEQ ID NO: 3, VLa CDR1 set forth in SEQ ID NO: 4, VLa CDR2 set forth in SEQ ID NO: 5, and VLa CDR3 set forth in SEQ ID NO: 6; the variable region b comprises at least one or more of VHb CDR1 set forth in SEQ ID NO: 7, VHb CDR2 set forth in SEQ ID NO: 8, VHb CDR3 set forth in SEQ ID NO: 9, VLb CDR1 set forth in SEQ ID NO: 4, VLb CDR2 set forth in SEQ ID NO: 5, and VLb CDR3 set forth in SEQ ID NO: 6.

13. The use according to claim 12, wherein the anti-PD-L1/CD47 bispecific antibody or the antigen-binding fragment binds to PD-L1 with greater affinity than to CD47.

14. The use according to claim 12 or 13, wherein the variable region a comprises VHa CDR1 set forth in SEQ ID NO: 1, VHa CDR2 set forth in SEQ ID NO: 2, VHa CDR3 set forth in SEQ ID NO: 3, VLa CDR1 set forth in SEQ ID NO: 4, VLa CDR2 set forth in SEQ ID NO: 5, and VLa CDR3 set forth in SEQ ID NO: 6; the variable region b comprises VHb CDR1 set forth in SEQ ID NO: 7, VHb CDR2 set forth in SEQ ID NO: 8, VHb CDR3 set forth in SEQ ID NO: 9, VLb CDR1 set forth in SEQ ID NO: 4, VLb CDR2 set forth in SEQ ID NO: 5, and VLb CDR3 set forth in SEQ ID NO: 6.

15. The use according to any one of claims 12-14, wherein the variable region a comprises a heavy chain variable region VHa and a light chain variable region VLa; wherein

the VHa comprises an amino acid sequence set forth in SEQ ID NO: 10, or an amino acid sequence having at least 80% or 90% identity to the amino acid sequence set forth in SEQ ID NO: 10, or an amino acid sequence having one or more conservative amino acid substitutions as compared with the amino acid sequence set forth in SEQ ID NO: 10; and/or

the VLa comprises an amino acid sequence set forth in SEQ ID NO: 12, or an amino acid sequence having at least 80% or 90% identity to the amino acid sequence set forth in SEQ ID NO: 12, or an amino acid sequence having one or more conservative amino acid substitutions as compared with the amino acid sequence set forth in SEQ ID NO: 12.

16. The use according to any one of claims 12-15, wherein the variable region b comprises a heavy chain variable region VHb and a light chain variable region VLb; wherein

the VHb comprises an amino acid sequence set forth in SEQ ID NO: 11, or an amino acid sequence having at least 80% or 90% identity to the amino acid sequence set forth in SEQ ID NO: 11, or an amino acid sequence having one or more conservative amino acid substitutions as compared with the amino acid sequence set forth in SEQ ID NO: 11; and/or

the VLb comprises an amino acid sequence set forth in SEQ ID NO: 12, or an amino acid sequence having at least 80% or 90% identity to the amino acid sequence set forth in SEQ ID NO: 12, or an amino acid sequence having one or more conservative amino acid substitutions as compared with the amino acid sequence set forth in SEQ ID NO: 12.

17. The use according to any one of claims 12-16, wherein the anti-PD-L1/CD47 bispecific antibody further comprises a heavy chain constant region CHa and a heavy chain constant region CHb, wherein the heavy chain constant region CHa is linked to the heavy chain variable region VHa to form a heavy chain a, and the heavy chain constant region CHb is linked to the heavy chain variable region VHb to form a heavy chain b; or the heavy chain constant region CHa and/or the heavy chain constant region CHb comprises an amino acid mutation of N297A, wherein an amino acid position is numbered according to the Eu numbering scheme.

18. The use according to any one of claims 12-17, wherein the heavy chain constant region CHa comprises an amino acid sequence set forth in SEQ ID NO: 13 or 19, or an amino acid sequence having at least 80% or 90% identity to the amino acid sequence set forth in SEQ ID NO: 13 or 19, or an amino acid sequence having one or more conservative amino acid substitutions as compared with the amino acid sequence set forth in SEQ ID NO: 13 or 19; and/or the heavy chain constant region CHb comprises an amino acid sequence set forth in SEQ ID NO: 15 or 20, or an amino acid sequence having at least 80% or 90% identity to the amino acid sequence set forth in SEQ ID NO: 15 or 20, or an amino acid sequence having one or more conservative amino acid substitutions as compared with the amino acid sequence set forth in SEQ ID NO: 15 or 20.

19. The use according to any one of claims 12-18, wherein the anti-PD-L1/CD47 bispecific antibody comprises a heavy chain a, a heavy chain b, a light chain a, and a light chain b; wherein

the heavy chain a comprises an amino acid sequence set forth in SEQ ID NO: 16 or 21, or an amino acid sequence having at least 80% or 90% identity to the amino acid sequence set forth in SEQ ID NO: 16 or 21, or an amino acid sequence having one or more conservative amino acid substitutions as compared with the amino acid sequence set forth in SEQ ID NO: 16 or 21; and/or

the heavy chain b comprises an amino acid sequence set forth in SEQ ID NO: 17 or 22, or an amino acid sequence having at least 80% or 90% identity to the amino acid sequence set forth in SEQ ID NO: 17 or 22, or an amino acid sequence having one or more conservative amino acid substitutions as compared with the amino acid sequence set forth in SEQ ID NO: 17 or 22; and/or

the light chain a and the light chain b comprise identical amino acid sequences; or the light chain a and light chain b each comprise an amino acid sequence set forth in SEQ ID NO: 18, or an amino acid sequence having at least 80% or 90% identity to the amino acid sequence set forth in SEQ ID NO: 18, or an amino acid sequence having one or more conservative amino acid substitutions as compared with the amino acid sequence set forth in SEQ ID NO: 18.

20. The use according to any one of claims 12-19, wherein the tumor comprises a benign tumor or cancer; or the cancer comprises a solid tumor, a hematologic cancer, or a metastatic lesion; or the hematologic cancer comprises leukemia, lymphoma, or myeloma; or the hematologic cancer comprises acute lymphocytic leukemia, acute myeloid leukemia, chronic myelogenous leukemia, chronic lymphocytic leukemia, myeloproliferative diseases/tumors, myelodysplastic syndrome, diffuse large B-cell lymphoma, Hodgkin's lymphoma, non-Hodgkin's lymphoma, Burkitt's lymphoma, follicular lymphoma, multiple myeloma, giant cell myeloma, heavy chain myeloma, or light chain or Bence-Jones myeloma; or the solid tumor comprises breast cancer, ovarian cancer, lung cancer, prostate cancer, melanoma, colorectal cancer, head and neck cancer, bladder cancer, esophageal cancer, liver cancer, renal cancer, gastric cancer, cervical cancer, pancreatic cancer, thyroid cancer, neuroglioma, salivary gland cancer, leiomyosarcoma, thymus cancer, or epithelial cancer.

21. The use according to any one of claims 12-20, wherein one treatment cycle is for about two weeks.

22. The use according to any one of claims 12-20, wherein the anti-PD-L1/CD47 bispecific antibody or the antigen-binding fragment is administered once about every two weeks.

23. Use of an anti-PD-L1/CD47 bispecific antibody or an antigen-binding fragment in the preparation of a medicament for treating an autoimmune disease, an inflammatory disease, an infectious disease, or a tumor; wherein the anti-PD-L1/CD47 bispecific antibody or the antigen-binding fragment is administered in an amount of 0.1 mg/kg to 100 mg/kg once every two days to once every six weeks;

the anti-PD-L1/CD47 bispecific antibody or the antigen-binding fragment comprises a variable region a specifically binding to PD-L1 and a variable region b specifically binding to CD47; wherein the variable region a comprises at least one or more of VHa CDR1 set forth in SEQ ID NO: 1, VHa CDR2 set forth in SEQ ID NO: 2, VHa CDR3 set forth in SEQ ID NO: 3, VLa CDR1 set forth in SEQ ID NO: 4, VLa CDR2 set forth in SEQ ID NO: 5, and VLa CDR3 set forth in SEQ ID NO: 6; the variable region b comprises at least one or more of VHb CDR1 set forth in SEQ ID NO: 7, VHb CDR2 set forth in SEQ ID NO: 8, VHb CDR3 set forth in SEQ ID NO: 9, VLb CDR1 set forth in SEQ ID NO: 4, VLb CDR2 set forth in SEQ ID NO: 5, and VLb CDR3 set forth in SEQ ID NO: 6.

24. The use according to claim 23, wherein the anti-PD-L1/CD47 bispecific antibody or the antigen-binding fragment binds to PD-L1 with greater affinity than to CD47.

25. The use according to claim 23 or 24, wherein the variable region a comprises VHa CDR1 set forth in SEQ ID NO: 1, VHa CDR2 set forth in SEQ ID NO: 2, VHa CDR3 set forth in SEQ ID NO: 3, VLa CDR1 set forth in SEQ ID NO: 4, VLa CDR2 set forth in SEQ ID NO: 5, and VLa CDR3 set forth in SEQ ID NO: 6; the variable region b comprises VHb CDR1 set forth in SEQ ID NO: 7, VHb CDR2 set forth in SEQ ID NO: 8, VHb CDR3 set forth in SEQ ID NO: 9, VLb CDR1 set forth in SEQ ID NO: 4, VLb CDR2 set forth in SEQ ID NO: 5, and VLb CDR3 set forth in SEQ ID NO: 6.

26. The use according to any one of claims 23-25, wherein the variable region a comprises a heavy chain variable region VHa and a light chain variable region VLa; wherein

the VHa comprises an amino acid sequence set forth in SEQ ID NO: 10, or an amino acid sequence having at least 80% or 90% identity to the amino acid sequence set forth in SEQ ID NO: 10, or an amino acid sequence having one or more conservative amino acid substitutions as compared with the amino acid sequence set forth in SEQ ID NO: 10; and/or
the VLa comprises an amino acid sequence set forth in SEQ ID NO: 12, or an amino acid sequence having at least 80% or 90% identity to the amino acid sequence set forth in SEQ ID NO: 12, or an amino acid sequence having one or more conservative amino acid substitutions as compared with the amino acid sequence set forth in SEQ ID NO: 12.

27. The use according to any one of claims 23-26, wherein the variable region b comprises a heavy chain variable region VHb and a light chain variable region VLb; wherein

the VHb comprises an amino acid sequence set forth in SEQ ID NO: 11, or an amino acid sequence having at least 80% or 90% identity to the amino acid sequence set forth in SEQ ID NO: 11, or an amino acid sequence having one or more conservative amino acid substitutions as compared with the amino acid sequence set forth in SEQ ID NO: 11; and/or
the VLb comprises an amino acid sequence set forth in SEQ ID NO: 12, or an amino acid sequence having at least 80% or 90% identity to the amino acid sequence set forth in SEQ ID NO: 12, or an amino acid sequence having one or more conservative amino acid substitutions as compared with the amino acid sequence set forth in SEQ ID NO: 12.

28. The use according to any one of claims 23-27, wherein the anti-PD-L1/CD47 bispecific antibody further comprises a heavy chain constant region CHa and a heavy chain constant region CHb, wherein the heavy chain constant region CHa is linked to the heavy chain variable region VHa to form a heavy chain a, and the heavy chain constant region CHb is linked to the heavy chain variable region VHb to form a heavy chain b; or the heavy chain constant region CHa and/or the heavy chain constant region CHb comprises an amino acid mutation of N297A, wherein an amino acid position is numbered according to the Eu numbering scheme.

29. The use according to any one of claims 23-28, wherein the heavy chain constant region CHa comprises an amino acid sequence set forth in SEQ ID NO: 13 or 19, or an amino acid sequence having at least 80% or 90% identity to the amino acid sequence set forth in SEQ ID NO: 13 or 19, or an amino acid sequence having one or more conservative amino acid substitutions as compared with the amino acid sequence set forth in SEQ ID NO: 13 or 19; and/or
the heavy chain constant region CHb comprises an amino acid sequence set forth in SEQ ID NO: 15 or 20, or an amino acid sequence having at least 80% or 90% identity to the amino acid sequence set forth in SEQ ID NO: 15 or 20, or an amino acid sequence having one or more conservative amino acid substitutions as compared with the amino acid sequence set forth in SEQ ID NO: 15 or 20.

30. The use according to any one of claims 23-29, wherein the anti-PD-L1/CD47 bispecific antibody comprises a heavy chain a, a heavy chain b, a light chain a, and a light chain b; wherein

the heavy chain a comprises an amino acid sequence set forth in SEQ ID NO: 16 or 21, or an amino acid sequence having at least 80% or 90% identity to the amino acid sequence set forth in SEQ ID NO: 16 or 21, or an amino acid sequence having one or more conservative amino acid substitutions as compared with the amino acid sequence set forth in SEQ ID NO: 16 or 21; and/or
the heavy chain b comprises an amino acid sequence set forth in SEQ ID NO: 17 or 22, or an amino acid sequence having at least 80% or 90% identity to the amino acid sequence set forth in SEQ ID NO: 17 or 22, or an amino acid sequence having one or more conservative amino acid substitutions as compared with the amino acid sequence set forth in SEQ ID NO: 17 or 22; and/or
the light chain a and the light chain b comprise identical amino acid sequences; or the light chain a and light chain b each comprise an amino acid sequence set forth in SEQ ID NO: 18, or an amino acid sequence having at least 80% or 90% identity to the amino acid sequence set forth in SEQ ID NO: 18, or an amino acid sequence having one or more conservative amino acid substitutions as compared with the amino acid sequence set forth in SEQ ID NO: 18.

31. The use according to any one of claims 23-30, wherein the tumor comprises a benign tumor or cancer; or the cancer comprises a solid tumor, a hematologic cancer, or a metastatic lesion; or the hematologic cancer comprises leukemia, lymphoma, or myeloma; or the hematologic cancer comprises acute lymphocytic leukemia, acute myeloid leukemia, chronic myelogenous leukemia, chronic lymphocytic leukemia, myeloproliferative diseases/tumors, myelodysplastic syndrome, diffuse large B-cell lymphoma, Hodgkin's lymphoma, non-Hodgkin's lymphoma, Burkitt's lymphoma, follicular lymphoma, multiple myeloma, giant cell myeloma, heavy chain myeloma, or light chain or Bence-Jones myeloma; or the solid tumor comprises breast cancer, ovarian cancer, lung cancer, prostate cancer, melanoma, colorectal cancer, head and neck cancer, bladder cancer, esophageal cancer, liver cancer, renal cancer, gastric cancer, cervical cancer, pancreatic cancer, thyroid cancer, neuroglioma, salivary gland cancer, leiomyosarcoma, thymus cancer, or epithelial cancer.

32. The use according to any one of claims 23-31, wherein one treatment cycle is for about two weeks.

33. The use according to any one of claims 23-31, wherein the anti-PD-L1/CD47 bispecific antibody or the antigen-binding fragment is administered once about every two weeks.

34. A kit comprising an anti-PD-L1/CD47 bispecific antibody or an antigen-binding fragment and instructions for directing administration of the anti-PD-L1/CD47 bispecific antibody or the antigen-binding fragment at 0.1 mg/kg to 100 mg/kg to a patient in need thereof once every two days to once every six weeks; wherein the anti-PD-L1/CD47 bispecific antibody or the antigen-binding fragment comprises a variable region a specifically binding to PD-L1 and a variable region b specifically binding to CD47; wherein the variable region a comprises at least one or more of VHa CDR1 set forth in SEQ ID NO: 1, VHa CDR2 set forth in SEQ ID NO: 2, VHa CDR3 set forth in SEQ ID NO: 3, VLa CDR1 set forth in SEQ ID NO: 4, VLa CDR2 set forth in SEQ ID NO: 5, and VLa CDR3 set forth in SEQ ID NO: 6; the variable region b comprises at least one or more of VHb CDR1 set forth in SEQ ID NO: 7, VHb CDR2 set forth in SEQ ID NO: 8, VHb CDR3 set forth in SEQ ID NO: 9, VLb CDR1 set forth in SEQ ID NO: 4, VLb CDR2 set forth in SEQ ID NO: 5, and VLb CDR3 set forth in SEQ ID NO: 6.

35. The kit according to claim 34, wherein the anti-PD-L1/CD47 bispecific antibody or the antigen-binding fragment binds to PD-L1 with greater affinity than to CD47.

36. The kit according to claim 34 or 35, wherein the variable region a comprises VHa CDR1 set forth in SEQ ID NO: 1, VHa CDR2 set forth in SEQ ID NO: 2, VHa CDR3 set forth in SEQ ID NO: 3, VLa CDR1 set forth in SEQ ID NO: 4, VLa CDR2 set forth in SEQ ID NO: 5, and VLa CDR3 set forth in SEQ ID NO: 6; the variable region b comprises VHb CDR1 set forth in SEQ ID NO: 7, VHb CDR2 set forth in SEQ ID NO: 8, VHb CDR3 set forth in SEQ ID NO: 9, VLb CDR1 set forth in SEQ ID NO: 4, VLb CDR2 set forth in SEQ ID NO: 5, and VLb CDR3 set forth in SEQ ID NO: 6.

37. The kit according to any one of claims 34-36, wherein the variable region a comprises a heavy chain variable region VHa and a light chain variable region VLa; wherein

the VHa comprises an amino acid sequence set forth in SEQ ID NO: 10, or an amino acid sequence having at least 80% or 90% identity to the amino acid sequence set forth in SEQ ID NO: 10, or an amino acid sequence having one or more conservative amino acid substitutions as compared with the amino acid sequence set forth in SEQ ID NO: 10; and/or

the VLa comprises an amino acid sequence set forth in SEQ ID NO: 12, or an amino acid sequence having at least 80% or 90% identity to the amino acid sequence set forth in SEQ ID NO: 12, or an amino acid sequence having one or more conservative amino acid substitutions as compared with the amino acid sequence set forth in SEQ ID NO: 12.

38. The kit according to any one of claims 34-37, wherein the variable region b comprises a heavy chain variable region VHb and a light chain variable region VLb; wherein

the VHb comprises an amino acid sequence set forth in SEQ ID NO: 11, or an amino acid sequence having at least 80% or 90% identity to the amino acid sequence set forth in SEQ ID NO: 11, or an amino acid sequence having one or more conservative amino acid substitutions as compared with the amino acid sequence set forth in SEQ ID NO: 11; and/or

the VLb comprises an amino acid sequence set forth in SEQ ID NO: 12, or an amino acid sequence having at least 80% or 90% identity to the amino acid sequence set forth in SEQ ID NO: 12, or an amino acid sequence having one or more conservative amino acid substitutions as compared with the amino acid sequence set forth in SEQ ID NO: 12.

39. The kit according to any one of claims 34-38, wherein the anti-PD-L1/CD47 bispecific antibody further comprises a heavy chain constant region CHa and a heavy chain constant region CHb, wherein the heavy chain constant region CHa is linked to the heavy chain variable region VHa to form a heavy chain a, and the heavy chain constant region CHb is linked to the heavy chain variable region VHb to form a heavy chain b; or the heavy chain constant region CHa and/or the heavy chain constant region CHb comprises an amino acid mutation of N297A, wherein an amino acid position is numbered according to the Eu numbering scheme.

40. The kit according to any one of claims 34-39, wherein the heavy chain constant region CHa comprises an amino acid sequence set forth in SEQ ID NO: 13 or 19, or an amino acid sequence having at least 80% or 90% identity to the amino acid sequence set forth in SEQ ID NO: 13 or 19, or an amino acid sequence having one or more conservative amino acid substitutions as compared with the amino acid sequence set forth in SEQ ID NO: 13 or 19; and/or

the heavy chain constant region CHb comprises an amino acid sequence set forth in SEQ ID NO: 15 or 20, or an amino acid sequence having at least 80% or 90% identity to the amino acid sequence set forth in SEQ ID NO: 15 or 20, or an amino acid sequence having one or more conservative amino acid substitutions as compared with the amino acid sequence set forth in SEQ ID NO: 15 or 20.

41. The kit according to any one of claims 34-40, wherein the anti-PD-L1/CD47 bispecific antibody comprises a heavy chain a, a heavy chain b, a light chain a, and a light chain b; wherein

the heavy chain a comprises an amino acid sequence set forth in SEQ ID NO: 16 or 21, or an amino acid sequence having at least 80% or 90% identity to the amino acid sequence set forth in SEQ ID NO: 16 or 21, or an amino acid sequence having one or more conservative amino acid substitutions as compared with the amino acid sequence set forth in SEQ ID NO: 16 or 21; and/or

the heavy chain b comprises an amino acid sequence set forth in SEQ ID NO: 17 or 22, or an amino acid sequence having at least 80% or 90% identity to the amino acid sequence set forth in SEQ ID NO: 17 or 22, or an amino acid sequence having one or more conservative amino acid substitutions as compared with the amino acid sequence set forth in SEQ ID NO: 17 or 22; and/or

the light chain a and the light chain b comprise identical amino acid sequences; or the light chain a and light chain b each comprise an amino acid sequence set forth in SEQ ID NO: 18, or an amino acid sequence having at least 80% or 90% identity to the amino acid sequence set forth in SEQ ID NO: 18, or an amino acid sequence having one or more conservative amino acid substitutions as compared with the amino acid sequence set forth in SEQ ID NO: 18.

FIG. 1

FIG. 2

FIG. 3

## INTERNATIONAL SEARCH REPORT

| International application No. |
|---|
| **PCT/CN2022/110627** |

**A. CLASSIFICATION OF SUBJECT MATTER**

C07K 16/46(2006.01)i; C12N 15/13(2006.01)i; A61K 39/395(2006.01)i; A61P 29/00(2006.01)n; A61P 35/00(2006.01)n; A61P 35/02(2006.01)n

According to International Patent Classification (IPC) or to both national classification and IPC

**B. FIELDS SEARCHED**

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

CNABS, CNTXT, SIPOABS, DWPI, WOTXT, USTXT, EPTXT, CNKI, 万方数据库, WANFANG DATABASE: 双特异性抗体, 多特异性抗体, bispecific, multispecific, antibody, 共同轻链, PD-L1, PDL1, CD47, IAP, VH, VL, CH, CL, 癌症, 肿瘤, 自身免疫, 炎性疾病, cancer, tumor, SEQ ID NOs: 1-22, 百奥泰生物制药股份有限公司

**C. DOCUMENTS CONSIDERED TO BE RELEVANT**

| Category* | Citation of document, with indication, where appropriate, of the relevant passages | Relevant to claim No. |
|---|---|---|
| PX | CN 114437227 A (BIO-THERA SOLUTIONS, LTD.) 06 May 2022 (2022-05-06) description, paragraphs 83, 96, 107, 128-143, 285, 296, 302, 333, and 406-410 | 1-41 |
| PY | CN 114057877 A (BIO-THERA SOLUTIONS, LTD.) 18 February 2022 (2022-02-18) description, embodiments 1-7 | 1-41 |
| PY | CN 114057876 A (BIO-THERA SOLUTIONS, LTD.) 18 February 2022 (2022-02-18) description, embodiments 1-5 | 1-41 |
| A | WO 2021124073 A1 (PFIZER INC.) 24 June 2021 (2021-06-24) entire document | 1-41 |
| A | CN 108347906 A (F. HOFFMANN-LA ROCHE AG.) 31 July 2018 (2018-07-31) entire document | 1-41 |
| A | WO 2020259605 A1 (INNOVENT BIOLOGICS (SUZHOU) CO., LTD.) 30 December 2020 (2020-12-30) entire document | 1-41 |

☑ Further documents are listed in the continuation of Box C.　　☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **11 October 2022** | **28 October 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| | International application No. |
| --- | --- |
| | **PCT/CN2022/110627** |

### C. DOCUMENTS CONSIDERED TO BE RELEVANT

| Category* | Citation of document, with indication, where appropriate, of the relevant passages | Relevant to claim No. |
| --- | --- | --- |
| A | WO 2019129054 A1 (INNOVENT BIOLOGICS (SUZHOU) CO., LTD.) 04 July 2019 (2019-07-04) entire document | 1-41 |
| A | CN 111801352 A (BIOCAD JOINT STOCK CO.) 20 October 2020 (2020-10-20) entire document | 1-41 |
| A | CN 107459578 A (TAIZHOU MABTECH PHARMACEUTICS CO., LTD.) 12 December 2017 (2017-12-12) entire document | 1-41 |
| A | EP 3722322 A1 (BEIJING HANMI PHARMACEUTICAL CO., LTD.) 14 October 2020 (2020-10-14) entire document | 1-41 |
| A | CN 112745392 A (SHANGHAI NOVAMAB BIOPHARMACEUTICALS CO., LTD.) 04 May 2021 (2021-05-04) entire document | 1-41 |
| A | 杨长良 等 (YANG, Changliang et al.). "新型免疫检查点—恶性肿瘤免疫治疗研究进展 (New Immune Checkpoint: Advances in Immunotherapy for Malignant Tumors)" 肿瘤防治研究 (Cancer Research on Prevention and Treatment), Vol. 45, No. 12, 31 December 2018 (2018-12-31), pp. 1027-1035 | 1-41 |
| A | LIU, Xiaojuan et al. "Dual Targeting of Innate and Adaptive Checkpoints on Tumor Cells Limits Immune Evasion" Cell Reports, Vol. 24, 21 August 2018 (2018-08-21), pp. 2101-2111 | 1-41 |

Form PCT/ISA/210 (second sheet) (January 2015)

**INTERNATIONAL SEARCH REPORT**

| International application No. |
| --- |
| **PCT/CN2022/110627** |

| Box No. I | Nucleotide and/or amino acid sequence(s) (Continuation of item 1.c of the first sheet) |
| --- | --- |

1.  With regard to any nucleotide and/or amino acid sequence disclosed in the international application, the international search was carried out on the basis of a sequence listing:

    a.  ☑  forming part of the international application as filed:

          ☑   in the form of an Annex C/ST.25 text file.

          ☐   on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

          ☐   in the form of an Annex C/ST.25 text file (Rule 13*ter*.1(a)).

          ☐   on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

    [1]   The actually submitted sequence table is an XML file in Standard ST.26.

Form PCT/ISA/210 (continuation of first sheet) (January 2015)

**INTERNATIONAL SEARCH REPORT**

International application No.

**PCT/CN2022/110627**

**Box No. II**     **Observations where certain claims were found unsearchable (Continuation of item 2 of first sheet)**

This international search report has not been established in respect of certain claims under Article 17(2)(a) for the following reasons:

1.  ☑  Claims Nos.: **1-22**
       because they relate to subject matter not required to be searched by this Authority, namely:

   [1]  Claims 1-11 relate to a method for treating autoimmune diseases, inflammatory diseases, infectious
        diseases, or tumors. Claims 12-22 relate to a use of an anti-PD-L1/CD47 bispecific antibody or
        an antigen-binding fragment in the treatment of the autoimmune diseases, inflammatory diseases,
        infectious diseases, or tumors. Therefore, claims 1-22 relate to the subject matter as defined in PCT
        Rule 39.1 that does not warrant a search conducted by the international searching authority: (4) a
        method for the treatment of a human or animal body by surgery or therapy, and a diagnostic method
        implemented on a human or animal body. An international search was made on the basis of a use of an
        effective amount of the anti-PD-L1/CD47 bispecific antibody or the antigen-binding fragment in the
        preparation of a drug for treating the autoimmune diseases, inflammatory diseases, infectious diseases,
        or tumors.

2.  ☐  Claims Nos.:
       because they relate to parts of the international application that do not comply with the prescribed requirements to such an
       extent that no meaningful international search can be carried out, specifically:

3.  ☐  Claims Nos.:
       because they are dependent claims and are not drafted in accordance with the second and third sentences of Rule 6.4(a).

Form PCT/ISA/210 (continuation of first sheet) (January 2015)

<div style="text-align:center">

**INTERNATIONAL SEARCH REPORT**
Information on patent family members

</div>

| International application No. |
| --- |
| **PCT/CN2022/110627** |

| Patent document cited in search report | | | Publication date (day/month/year) | Patent family member(s) | | | Publication date (day/month/year) |
| --- | --- | --- | --- | --- | --- | --- | --- |
| CN | 114437227 | A | 06 May 2022 | WO | 2022095970 | A1 | 12 May 2022 |
| CN | 114057877 | A | 18 February 2022 | TW | 202214698 | A | 16 April 2022 |
| | | | | WO | 2022028608 | A1 | 10 February 2022 |
| CN | 114057876 | A | 18 February 2022 | TW | 202208426 | A | 01 March 2022 |
| | | | | WO | 2022022662 | A1 | 03 February 2022 |
| WO | 2021124073 | A1 | 24 June 2021 | TW | 202130659 | A | 16 August 2021 |
| | | | | KR | 20220114049 | A | 17 August 2022 |
| | | | | US | 2021179716 | A1 | 17 June 2021 |
| | | | | CA | 3164623 | A1 | 24 June 2021 |
| | | | | AU | 2020410410 | A1 | 09 June 2022 |
| | | | | CO | 2022008407 | A2 | 08 July 2022 |
| | | | | IL | 293926 | A | 01 August 2022 |
| CN | 108347906 | A | 31 July 2018 | EP | 3367786 | A1 | 05 September 2018 |
| | | | | JP | 2018537968 | A | 27 December 2018 |
| | | | | WO | 2017072208 | A1 | 04 May 2017 |
| | | | | CN | 113862300 | A | 31 December 2021 |
| | | | | SG | 10202006332P | A | 28 August 2020 |
| | | | | HK | 1258306 | A1 | 08 November 2019 |
| | | | | CN | 113897371 | A | 07 January 2022 |
| | | | | SG | 11201802698X | A | 30 May 2018 |
| WO | 2020259605 | A1 | 30 December 2020 | KR | 20220036998 | A | 23 March 2022 |
| | | | | BR | 112021026414 | A2 | 12 April 2022 |
| | | | | AU | 2020304112 | A1 | 27 January 2022 |
| | | | | CA | 3144244 | A1 | 30 December 2020 |
| | | | | US | 2022251210 | A1 | 11 August 2022 |
| | | | | CN | 114040777 | A | 11 February 2022 |
| | | | | TW | 202104270 | A | 01 February 2021 |
| | | | | EP | 3991745 | A1 | 04 May 2022 |
| | | | | JP | 2022540781 | A | 20 September 2022 |
| WO | 2019129054 | A1 | 04 July 2019 | | None | | |
| CN | 111801352 | A | 20 October 2020 | JO | P20200078 | A1 | 03 April 2019 |
| | | | | JP | 2020535839 | A | 10 December 2020 |
| | | | | EP | 3693390 | A1 | 12 August 2020 |
| | | | | PE | 20210460 | A1 | 08 March 2021 |
| | | | | CL | 2020000919 | A1 | 16 October 2020 |
| | | | | US | 2020270345 | A1 | 27 August 2020 |
| | | | | AR | 113342 | A1 | 22 April 2020 |
| | | | | EA | 201791961 | A1 | 30 April 2019 |
| | | | | RU | 2020115122 | A3 | 29 October 2021 |
| | | | | TW | 201922781 | A | 16 June 2019 |
| | | | | CO | 2020004199 | A2 | 24 April 2020 |
| | | | | MA | 49599 | A1 | 29 January 2021 |
| | | | | CA | 3078413 | A1 | 11 April 2019 |
| | | | | KR | 20200058542 | A | 27 May 2020 |
| | | | | EC | SP20024555 | A | 30 June 2020 |
| | | | | PH | 12020550214 | A1 | 15 February 2021 |
| | | | | ZA | 202002047 | B | 31 August 2022 |
| | | | | BR | 112020006706 | A2 | 06 October 2020 |
| | | | | WO | 2019068302 | A1 | 11 April 2019 |

Form PCT/ISA/210 (patent family annex) (January 2015)

**INTERNATIONAL SEARCH REPORT**
Information on patent family members

International application No.

**PCT/CN2022/110627**

| Patent document cited in search report | | | Publication date (day/month/year) | Patent family member(s) | | | Publication date (day/month/year) |
|---|---|---|---|---|---|---|---|
| | | | | AU | 2018345458 | A1 | 14 May 2020 |
| CN | 107459578 | A | 12 December 2017 | | None | | |
| EP | 3722322 | A1 | 14 October 2020 | US | 2021230277 | A1 | 29 July 2021 |
| | | | | WO | 2019109876 | A1 | 13 June 2019 |
| | | | | AU | 2018378529 | A1 | 18 June 2020 |
| | | | | EA | 202091364 | A1 | 13 October 2020 |
| | | | | SG | 11202005216X | A | 29 July 2020 |
| | | | | KR | 20200093639 | A | 05 August 2020 |
| | | | | CN | 111615521 | A | 01 September 2020 |
| | | | | BR | 112020011295 | A2 | 24 November 2020 |
| | | | | ZA | 202003382 | B | 30 June 2021 |
| | | | | JP | 2021505195 | A | 18 February 2021 |
| | | | | IL | 275135 | A | 30 July 2020 |
| | | | | CA | 3084626 | A1 | 13 June 2019 |
| CN | 112745392 | A | 04 May 2021 | | None | | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8216805 B2
- US 5892019 A
- US 4816397 A
- US 5168062 A
- US 4510245 A
- US 4968615 A
- US 4399216 A
- US 4634665 A
- US 5179017 A
- US 20150183874 A1

**Non-patent literature cited in the description**

- **JOHN B. B. RIDGWAY et al.** Knobs-into-holes' engineering of antibody CH3 domains for heavy chain heterodimerization. *Protein Engineering,* 1996, vol. 9 (7), 617-21
- **SHANE ATWELL et al.** Stable heterodimers form remodeling the domain interface of a homodimer using a phage display library. *J. mol. Biol.,* 1997, vol. 270, 26-35
- **PAUL CARTER.** Bispecific human IgG by design. *Journal of Immunological Methods,* 2001, vol. 248, 7-15
- **J. LUND et al.** Oligosaccharide-protein interactions in IgG can modulate recognition by Fc gamma receptors. *FASEB. J.,* 1995, vol. 9, 115-119
- The Scientist. Scientist, Inc, 17 April 2000, vol. 14, 25-28
- **WU ; WU.** *J. Biol. Chem.,* 1987, vol. 262, 4429-4432
- **KABAT et al.** Sequences of Proteins of Immunological Interes. Public Health Service, National Institutes of Health, 1991
- Immunology-A Synthesis. Sinauer Associates, 1991
- Proteins: Structures and Molecular Principles. W. H. Freeman and Company, 1984
- Introduction to Protein Structure. Garland Publishing, 1991
- **THORNTON et al.** *Nature,* 1991, vol. 354, 105
- Current Protocols in Molecular Biology. 2007
- **KABAT, E. et al.** Sequences of Proteins of Immunological Interest. U.S. Department of Health and Human Services, 1983
- **CHOTHIA ; LESK.** *J. Mol. Biol.,* 1987, vol. 196, 901-917
- **KABAT et al.** Sequences of Proteins of Immunological Interest. U.S. Dept. of Health and Human Services, 1983
- **CHOTHIA et al.** *J. Mol. Biol.,* 1987, vol. 196, 901-917
- **KABAT et al.** Sequence of Proteins of Immunological Interest. U.S. Dept. of Health and Human Services, 1983
- **ROUX et al.** *J. Immunol.,* 1998, vol. 161, 4083
- McGraw-Hill Dictionary of Chemical Terms. McGraw-Hill, 1985
- Molecular Cloning: A Laboratory Manual. Cold Spring Harbor, 1989
- Current Protocols in Molecular Biology. Greene Publishing Associates, 1989
- **TIHOMIR S. DODEV et al.** A tool kit for rapid cloning and expression of recombinant antibodies. *Scientific Reports,* 2014, vol. 4
- **STEFAN SCHLATTER et al.** On the Optimal Ratio of Heavy to Light Chain Genes for Efficient Recombinant Antibody Production by CHO Cells. *Biotechnol Progress,* 2005, vol. 21, 122-133
- **HADI BAYAT et al.** Evaluation of different vector design strategies for the expression of recombinant monoclonal antibody in CHO cells. *Preparative Biochemistry & Biotechnology,* 2018, vol. 48 (8), 822-829
- **GOEDDEL.** Gene Expression Technology: Methods in Enzymology. Academic Press, 1990, 185
- Solid Phase Peptide Synthesis. The Pierce Chemical Co, 1984
- **CHU et al.** Roche Molecular Biologicals. *Biochemia,* 2001, (2
- **MURRAY et al.** *Current Opinion in Chemical Biology,* 2013, vol. 17, 420-426